# EUROPEAN PATENT APPLICATION

(11) **EP 4 796 113 A2**
(43) Date of publication of application: **26.08.2026**
(21) Application number: 26178638.8
(22) Date of filing: 09.04.2021
(51) Int. Cl.: A61K 31/439

(54) **CRYSTALLINE FORMS OF (9R,13S)-13-{4-[5-CHLORO-2-(4-CHLORO-1H-1,2,3-TRIAZOL-1-YL)PHENYL]-6-OXO-1,6-DIHYDROPYRIMIDIN-1-YL}-3-(DIFLUOROMETHYL)-9-METHYL-3,4,7,15-TETRAAZATRICYCLO[12.3.1.02,6]OCTADECA-1(18),2(6),4,14,16-PENTAEN-8-ONE**

(30) Priority: 10.04.2020 US 202063008161 P
(62) Document number(s) of the earlier application(s) in accordance with Art. 76 EPC: 21722085.4 / 4 132 930
(71) Applicant: Bristol-Myers Squibb Company, Princeton, NJ 08543 (US); Janssen Pharmaceutica NV, 2340 Beerse (BE)
(72) Inventor: DILGER, Andrew K., Princeton, 08543 (US); GALELLA, Michael A., Princeton, 08543 (US); SMITH, Daniel, Princeton, 08543 (US); ZIEMBA, Theresa M., Princeton, 08543 (US); LÜDEKER, David, 2340 Beerse (BE); XIOURAS, Christos, 2340 Beerse (BE); AHUJA, Dipali, 2340 Beerse (BE); CLEEREN, Dirk Angelina J., 2340 Beerse (BE)
(74) Representative: Mewburn Ellis LLP

(57) **Abstract**

Disclosed are crystalline forms of Compound (I). Compound (I) is useful as an anti-thromboembolic agent in the treatment of cardiovascular diseases.

## Description

### FIELD OF INDUSTRIAL APPLICABILITY

The present invention generally relates to crystalline forms of (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo[12.3.1.0^{2,6}]octadeca-1(18),2(6),4,14,16-pentaen-8-one. The present invention also generally relates to pharmaceutical compositions comprising the crystalline forms, as well as methods of using the crystalline forms in the treatment of thromboembolic disorders, and methods for obtaining such crystalline forms.

### BACKGROUND OF THE INVENTION

Factor XIa is a plasma serine protease involved in the regulation of blood coagulation, which is initiated *in vivo* by the binding of tissue factor (TF) to factor VII (FVII) to generate factor VIIa (FVIIa). The resulting TF:FVIIa complex activates factor IX (FIX) and factor X (FX) that leads to the production of factor Xa (FXa). The generated FXa catalyzes the transformation of prothrombin into small amounts of thrombin before this pathway is shut down by tissue factor pathway inhibitor (TFPI). The process of coagulation is then further propagated via the feedback activation of Factors V, VIII and XI by catalytic amounts of thrombin. (Gailani, D. et al., Arterioscler. Thromb. Vasc. Biol., 27:2507-2513 (2007).) The resulting burst of thrombin converts fibrinogen to fibrin that polymerizes to form the structural framework of a blood clot, and activates platelets, which are a key cellular component of coagulation (Hoffman, M., Blood Reviews, 17:S1-S5 (2003)). Therefore, factor XIa plays a key role in propagating this amplification loop and is thus an attractive target for anti-thrombotic therapy.

The compound, (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo[12.3.1.0^{2,6}]octadeca-1(18),2(6),4,14,16-pentaen-8-one, has the structure: 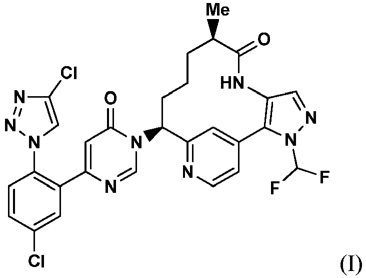 and is referred to herein as "Compound (I)". Compound (I), a process to prepare Compound (I), and methods of treatment employing Compound (I) are disclosed in U.S. Patent Application Publication No. 2016/0096839 (hereinafter referred to as the '839 publication), which is incorporated herein by reference in its entirety. In Example 88, the '839 publication discloses the synthesis of Compound (I). By the embodiments exemplified below, crystalline forms of Compound (I) can be obtained.

Compound (I) is useful for treating thromboembolic disorders. However, before Compound (I) is used to treat diseases in patients, it must be formulated into a pharmaceutical composition that can be administered to the patient; for example, into a dosage form suitable for oral, mucosal, parenteral, or transdermal administration. Formulations for oral administration are preferred since they are more convenient and easier to administer than other formulations. Also, the oral route of administration avoids the pain and discomfort of parenteral administration. Accordingly, formulations for oral administration are preferred by patients, and typically result in better patient compliance with dosing schedules.

Typically, in preparing a pharmaceutical composition, a form of the active ingredient is sought that has a balance of desired properties, such as, for example, dissolution rate, solubility, bioavailability, and/or storage stability. For example, a form of the active ingredient is sought having sufficient stability, solubility, and bioavailability to prevent the sufficiently soluble and bioavailable form from converting during the manufacture, preparation, and/or storage of the pharmaceutical composition to another form having an undesirable solubility and/or bioavailability profile. In addition, a form of the active ingredient may also be sought that permits the active ingredient to be isolated and/or purified during, for example, a preparative process.

Active pharmaceutical ingredients can exist in different physical forms (e.g., liquid or solid in different crystalline, amorphous, hydrate, or solvate forms), which can vary the processability, stability, solubility, bioavailability, pharmacokinetics (e.g. absorption, distribution, metabolism, excretion, or the like), and the pharmaceutical compositions comprising it. It is unpredictable whether a compound will exist in a particular polymorph form, how many such forms might exist for a particular compound (or salt form), what process might produce these forms, and what properties the particular polymorph might demonstrate. It is important in pharmaceutical development to generate and identify advantageous physical forms (e.g., free base or salt in solid, liquid, crystalline, hydrate, solvate, or amorphous forms) of active pharmaceutical ingredients. Therefore, there remains a need for particular polymorph forms of Compound (I).

The present invention provides at least one form of Compound (I) that surprisingly affords a balance of properties sought in a pharmaceutical composition. The present invention is also directed to other important aspects.

### SUMMARY OF THE INVENTION

The present invention provides crystalline forms of Compound (I). Embodiments of these crystalline forms include those characterized herein as Form A, Form B, Form C, Form D, Form E, Form F, Form G, Form H, Form I, and Form J. The names used herein to characterize a specific form, e.g. "Form A" etc., should not be considered limiting with respect to any other substance possessing similar or identical physical and chemical characteristics, but rather it should be understood that these designations are mere identifiers that should be interpreted according to the characterization information also presented herein.

### BRIEF DESCRIPTION OF THE DRAWINGS

FIG. 1A shows the observed powder x-ray diffraction pattern (PXRD) (CuKα λ=1.541874 Å at room temperature) of crystalline Form A of Compound (I). FIG. 1B shows an infrared (IR) spectrum of crystalline Form A of Compound (I). FIG. 1C shows dynamic vapor sorption (DVS) isotherms of crystalline Form A of Compound (I): y-axis: change in mass (%), x-axis: target relative humidity (RH)(%). FIG. 1D shows dynamic vapor sorption (DVS) isotherms of crystalline Form A of Compound (I): y-axis: change in mass (%), x-axis: time (min).
FIG. 2 shows the solid state nuclear magnetic resonance spectra (ssNMR) of crystalline Form A of Compound (I).
FIG. 3 shows a differential scanning calorimetry (DSC) thermogram of crystalline Form A of Compound (I).
FIG. 4 shows a thermogravimetric analysis (TGA) thermogram of the Form A of Compound (I).
FIG. 5A shows the observed powder x-ray diffraction pattern (CuKα λ=1.541874 Å at room temperature) of crystalline Form B of Compound (I). FIG. 5B shows an infrared (IR) spectrum of crystalline Form B of Compound (I). FIG. 5C shows a differential scanning calorimetry (DSC) thermogram of crystalline Form B of Compound (I).
FIG. 6A shows the observed powder x-ray diffraction pattern (CuKα λ=1.541874 Å at room temperature) of crystalline Form C of Compound (I). FIG. 6B shows an infrared (IR) spectrum of crystalline Form C of Compound (I). FIG. 6C shows a differential scanning calorimetry (DSC) thermogram of crystalline Form C of Compound (I).
FIG. 7A shows the observed powder x-ray diffraction pattern (CuKα λ=1.541874 Å at 25 °C) of crystalline Form D of Compound (I). FIG. 7B shows an infrared (IR) spectrum of crystalline Form D of Compound (I). FIG. 7C shows a differential scanning calorimetry (DSC) thermogram of crystalline Form D of Compound (I).
FIG. 8A shows the observed powder x-ray diffraction pattern (CuKα λ=1.541874 Å at 25 °C) of crystalline Form E of Compound (I). FIG. 8B shows an infrared (IR) spectrum of crystalline Form E of Compound (I).
FIG. 9A shows the observed powder x-ray diffraction pattern (CuKα λ=1.541874 Å at 25 °C) of crystalline Form F of Compound (I). FIG. 9B shows an infrared (IR) spectrum of crystalline Form F of Compound (I). FIG. 9C shows a differential scanning calorimetry (DSC) thermogram of crystalline Form F of Compound (I).
FIG. 10A shows the observed powder x-ray diffraction pattern (CuKα λ=1.541874 Å at 25 °C) of crystalline Form G of Compound (I). FIG. 10B shows an infrared (IR) spectrum of crystalline Form G of Compound (I).
FIG. 11A shows the observed powder x-ray diffraction pattern (CuKα λ=1.541874 Å at 25 °C) of crystalline Form H of Compound (I). FIG. 11B shows an infrared (IR) spectrum of crystalline Form H of Compound (I).
FIG. 12 shows the observed powder x-ray diffraction pattern (CuKα λ=1.541874 Å at 25 °C) of crystalline Form I of Compound (I).
FIG. 13A shows the observed powder x-ray diffraction pattern (CuKα λ=1.541874 Å at 25 °C) of crystalline Form J of Compound (I). FIG. 13B shows an infrared (IR) spectrum of crystalline Form J of Compound (I). FIG. 13C shows a differential scanning calorimetry (DSC) thermogram of crystalline Form J of Compound (I). FIG. 13D shows a thermogravimetric analysis (TGA) thermogram of crystalline Form J of Compound (I). FIG. 13E shows dynamic vapor sorption (DVS) isotherms of crystalline Form J of Compound (I).

### DETAILED DESCRIPTION OF THE INVENTION

The features and advantages of the invention may be more readily understood by those of ordinary skill in the art upon reading the following detailed description. It is to be appreciated that certain features of the invention that are, for clarity reasons, described above and below in the context of separate embodiments, may also be combined to form a single embodiment. Conversely, various features of the invention that are, for brevity reasons, described in the context of a single embodiment, may also be combined so as to form sub-combinations thereof.

All numbers expressing quantities of ingredients, weight percentages, temperatures, and so forth that are preceded by the word "about" are to be understood as only approximations so that slight variations above and below the stated number may be used to achieve substantially the same results as the stated number. Accordingly, unless indicated to the contrary, numerical parameters preceded by the word "about" are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

All measurements are subject to experimental error and are within the spirit of the invention.

As used herein, "hydrate" refers to a crystalline form of a molecule that further comprises water incorporated into the crystalline structure. The water molecules in the hydrate may be present in a regular arrangement and/or a non-ordered arrangement. The hydrate may comprise either a stoichiometric or nonstoichiometric amount of the water molecules, for example, monohydrate, hemihydrate, or a mixture thereof.

As used herein, "room temperature" is 25 °C, unless otherwise indicated.

As used herein "solvate" refers to a crystalline form of a molecule, atom, and/or ions that further comprises molecules of a solvent or solvents incorporated into the crystalline lattice structure. The solvent molecules in the solvate may be present in a regular arrangement and/or a non-ordered arrangement. The solvate may comprise either a stoichiometric or nonstoichiometric amount of the solvent molecules.

As used herein, "substantially pure," when used in reference to a crystalline form, means a compound having a purity greater than 90 weight %, including greater than 90, 91 , 92, 93, 94, 95, 96, 97, 98, and 99 weight %, and also including equal to about 100 weight % of Compound (I), based on the weight of the compound. The remaining material comprises other form(s) of the compound, and/or reaction impurities and/or processing impurities arising from its preparation. For example, a crystalline form of Compound (I) may be deemed substantially pure in that it has a purity greater than 90 weight %, as measured by means that are at this time known and generally accepted in the art, where the remaining less than 10 weight % of material comprises amorphous and/or other form(s) of Compound (I) and/or reaction impurities and/or processing impurities.

As used herein, a "free base neat crystalline form" of Compound (I) is a parent form (that is, not a salt form) of Compound (I) that is substantially pure and is not solvated or hydrated.

As used herein, a PXRD pattern "comprising" a number of peaks selected from a specified group of peaks, is intended to include PXRD patterns having additional peaks that are not included in the specified group of peaks. For example, a PXRD pattern comprising four or more, preferably five or more, 2θ values selected from: A, B, C, D, E, F, G, H, I, and J, is intended to include a PXRD pattern having: (a) four or more, preferably five or more, 2θ values selected from: A, B, C, D, E, F, G, H, I, and J; and (b) zero or more peaks that are not one of peaks A, B, C, D, E, F, G, H, I, and J.

The presence of reaction impurities and/or processing impurities may be determined by analytical techniques known in the art, such as, for example, chromatography, nuclear magnetic resonance spectroscopy, mass spectrometry, and/or infrared spectroscopy.

The definitions set forth herein take precedence over definitions set forth in any patent, patent application, and/or patent application publication incorporated herein by reference.

Abbreviations as used herein, are defined as follows: "1 x" for once, "2 x" for twice, "3 x" for thrice, "°C" for degrees Celsius, "eq" for equivalent or equivalents, "g" for gram or grams, "mg" for milligram or milligrams, "kg" for kilogram or kilograms, "L" for liter or liters, "mL" for milliliter or milliliters, "µL" for microliter or microliters, "N" for normal, "M" for molar, "mmol" for millimole or millimoles, "min" for minute or minutes, "h" for hour or hours, "rt" for room temperature (e.g., about 20, 21, 22, 23, 24, 25 °C.), "RT" for retention time, "RBF" for round bottom flask, "atm" for atmosphere, "psi" for pounds per square inch, "conc." for concentrate, "wt." for weight, "sat" or "sat'd" for saturated, "SFC" for supercritical fluid chromatography "MW" for molecular weight, "mp" for melting point, "ee" for enantiomeric excess, "MS" or "Mass Spec" for mass spectrometry, "ESI" for electrospray ionization mass spectroscopy, "HR" for high resolution, "HRMS" for high resolution mass spectrometry, "LCMS" for liquid chromatography mass spectrometry, "HPLC" for high pressure liquid chromatography, "RP HPLC" for reverse phase HPLC, "TLC" or "tlc" for thin layer chromatography, "NMR" for nuclear magnetic resonance spectroscopy, "nOe" for nuclear Overhauser effect spectroscopy, "¹H" for proton, "δ" for delta, "s" for singlet, "d" for doublet, "t" for triplet, "q" for quartet, "m" for multiplet, "br" for broad, "Hz" for hertz, and "α", "β", "R", "S", "E", and "Z" are stereochemical designations familiar to one skilled in the art.
- Me: methyl
- Et: ethyl
- Pr: propyl
- *i*-Pr: isopropyl
- Bu: butyl
- *i*-Bu: isobutyl
- *t*-Bu: *tert*-butyl
- Ph: phenyl
- Bn: benzyl
- Boc or BOC: *tert*-butyloxycarbonyl
- Boc₂O: di-*tert*-butyl dicarbonate
- AcOH or HOAc: acetic acid
- AlCl₃: aluminum chloride
- aqueous: aq
- DCM or CH₂Cl₂: dichloromethane
- CH₃CN or ACN: acetonitrile
- CDCl₃: deutero-chloroform
- CHCl₃: chloroform
- mCPBA or m-CPBA: *meta*-chloroperbenzoic acid
- Cs₂CO₃: cesium carbonate
- Cu(OAc)₂: copper (II) acetate
- CuI: copper(I) iodide
- CuSO₄: copper(II) sulfate
- Cy₂NMe: N-cyclohexyl-N-methylcyclohexanamine
- DBU: 1,8-diazabicyclo[5.4.0]undec-7-ene
- DCE: 1,2-dichloroethane
- DEA: diethylamine
- DMAc: dimethylacetamide
- DME: 1,2-dimethoxyethane
- DMF: dimethyl formamide
- DMSO: dimethyl sulfoxide
- EDC: *N*-(3-dimethylaminopropyl)-*N*'-ethylcarbodiimide
- EDCI: *N*-(3-dimethylaminopropyl)-*N*'-ethylcarbodiimide hydrochloride
- EDTA: ethylenediaminetetraacetic acid
- Et₃N or TEA: triethylamine
- EtOAc: ethyl acetate
- Et₂O: diethyl ether
- EtOH: ethanol
- GMF: glass microfiber filter
- Grubbs II: (1,3-bis(2,4,6-trimethylphenyl)-2-imidazolidinylidene)dichloro (phenylmethylene)(triycyclohexylphosphine)ruthenium
- HCl: hydrochloric acid
- HATU: O-(7-azabenzotriazol-1-yl)-N,N,N',N'-tetramethyluronium hexafluorophosphate
- HEPES: 4-(2-hydroxyethyl)piperazine-1-ethanesulfonic acid
- HOBt or HOBT: 1-hydroxybenzotriazole
- H₂SO₄: sulfuric acid
- LG: leaving group
- MeOH: methanol
- MgSO₄: magnesium sulfate
- MsOH or MSA: methylsulfonic acid
- NaCl: sodium chloride
- NaH: sodium hydride
- NaHCO₃: sodium bicarbonate
- Na₂CO₃: sodium carbonate
- NaOH: sodium hydroxide
- Na₂SO₃: sodium sulfite
- Na₂SO₄: sodium sulfate
- NCS: N-chlorosuccinimide
- NH₃: ammonia
- NH₄Cl: ammonium chloride
- NH₄OH: ammonium hydroxide
- NH₄COOH: ammonium formate
- Pd₂(dba)₃: tris(dibenzylideneacetone)dipalladium(0)
- Pd(OAc)₂: palladium(II) acetate
- Pd/C: palladium on carbon
- Pd(dppf)Cl₂: [1,1'-bis(diphenylphosphino)-ferrocene]dichloropalladium(II)
- PG: protecting group
- POCl₃: phosphorus oxychloride
- TFA: trifluoroacetic acid
- THF: tetrahydrofuran
- TMSCHN₂: trimethylsilyldiazomethane
- T3P^{®}: propane phosphonic acid anhydride
- TRIS: tris (hydroxymethyl) aminomethane
- pTsOH: p-toluenesulfonic acid
- t-BME: tert-butyl methyl ether

### Form A of Compound (I)

In one embodiment, Compound (I) is provided as a crystalline material comprising Form A. The crystalline Form A of Compound (I) is a neat crystalline form.

The crystalline Form A of Compound (I) can be produced by several solvent systems such as acetonitrile, acetonitrile-water mixture, butanol, *tert*-butanol, ethanol, ethanol-water mixture, isopropanol, isopropanol-water mixture, methyl isobutyl ketone, nitromethane, and methyl *tert*-butyl ether. In one embodiment, the crystalline Form A of Compound (I) can be produced through solvent systems such as water, heptane, cyclohexane, methyl tert-butyl ether, acetonitrile, ethanol, isopropyl ether, and mixtures thereof. In one embodiment, the crystalline Form A is produced by a mixture of diethyl ether and dimethyl formamide. The Form A solid can either be obtained by stirring excess Compound (I) in the aforementioned solvent systems or by precipitation from a saturated solution of Compound (I) *via* antisolvent addition (ex. water) and/or cooling.

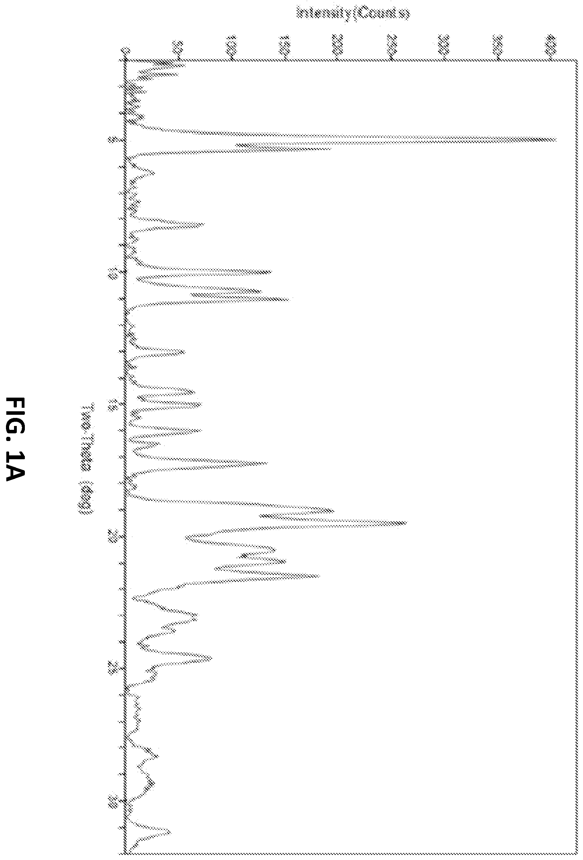

In one embodiment, the crystalline Form A of Compound (I) is characterized by a powder X-ray diffraction pattern comprising the 2θ values (CuKα λ= 1.5418 Å) at room temperature substantially as shown in FIG. 1A.

In one embodiment, the crystalline Form A of Compound (I) is characterized by a PXRD pattern (CuKα λ= 1.541874 Å at room temperature) comprising one, two, three, four, five, six, seven, eight, nine, ten or more 2θ values selected from: 5.0±0.2, 5.3±0.2, 8.2±0.2, 10.0±0.2, 10.7±0.2, 10.9±0.2, 13.0±0.2, 14.6±0.2, 15.1±0.2, 16.1±0.2, 17.2±0.2, 19.0±0.2, 19.5±0.2, 20.5±0.2, 21.5±0.2, 22.9±0.2, and 24.5±0.2, wherein the PXRD pattern of Form A is measured at room temperature.

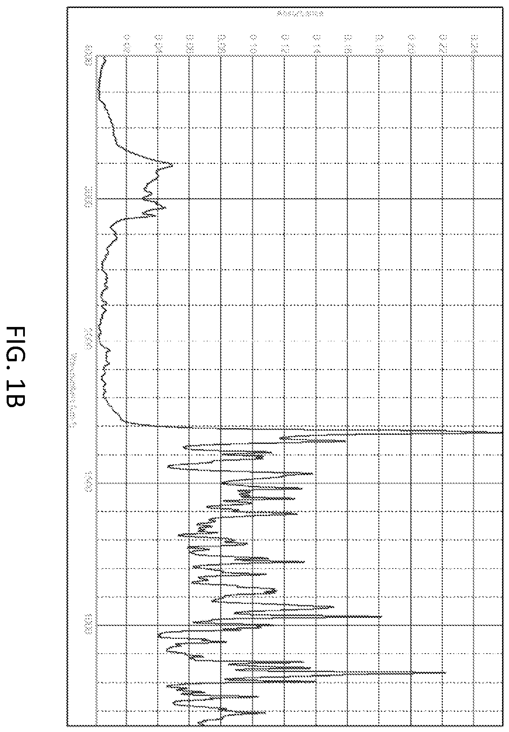

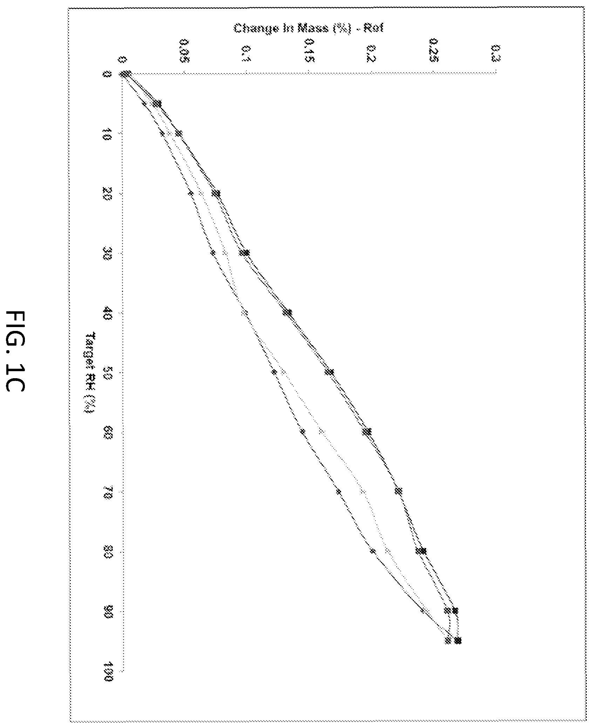

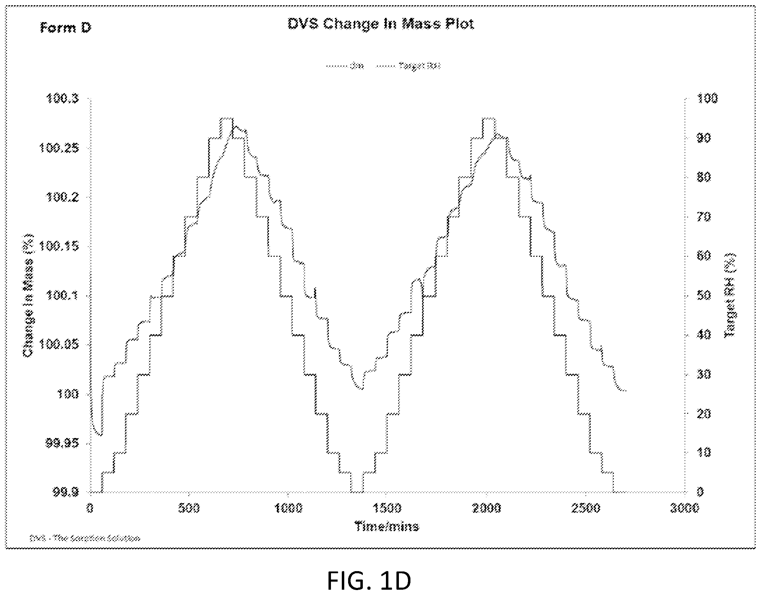

In one embodiment, the crystalline Form A of Compound (I) is characterized by a a) PXRD pattern comprising four or more 2θ values selected from: 5.0±0.2, 5.3±0.2, 8.2±0.2, 10.0±0.2, 10.7±0.2, 11.0±0.2, 13.0±0.2, 14.5±0.2, 15.1±0.2, and 16.1±0.2; and b) an observed powder x-ray diffraction pattern substantially as shown in Figure 1.

In one embodiment, the crystalline Form A of Compound (I) is characterized by a PXRD pattern comprising five or more 2θ values selected from: 5.0±0.2, 5.3±0.2, 8.2±0.2, 10.0±0.2, 10.7±0.2, 11.0±0.2, 13.0±0.2, 14.5±0.2, 15.1±0.2, and 16.1±0.2.

In one embodiment, the crystalline Form A of Compound (I) is characterized by a PXRD pattern comprising four or more 2θ values selected from: 5.0±0.2, 5.3±0.2, 8.2±0.2, 10.0±0.2, 10.7±0.2, 11.0±0.2, 13.0±0.2, 14.5±0.2, 15.1±0.2, and 16.1±0.2.

In one embodiment, the crystalline Form A of Compound (I) is characterized by a PXRD pattern comprising the 2θ values at 5.0±0.2, 10.0±0.2, and 10.7±0.2.

In one embodiment, the crystalline Form A of Compound (I) is characterized by a PXRD pattern (CuKα λ= 1.541874 Å at room temperature) comprising 2θ values: 10.0±0.2, 13.0±0.2, 19.0±0.2, 19.5±0.2, 21.5±0.2, and 24.5±0.2, wherein the PXRD pattern of Form A is measured at room temperature.

In one embodiment, the crystalline Form A of Compound (I) is characterized by a PXRD pattern (CuKα λ= 1.541874 Å at room temperature) comprising 2θ values: 5.0±0.2, 8.2±0.2, 10.0±0.2, 10.7±0.2, 10.9±0.2, 13.0±0.2, 19.0±0.2, 19.5±0.2, 21.5±0.2, and 24.5±0.2, wherein the PXRD pattern of Form A is measured at room temperature.

In one embodiment, the crystalline Form A can be characterized by an infrared (IR) spectrum at room temperature substantially as shown in FIG. 1B.

In one embodiment, the crystalline Form A is characterized by an infrared (IR) spectrum having one, two, three, four, five, six, seven, eight, nine, ten or more peaks selected from: 1677±2, 1647±2, 1607±2, 1593±2, 1586±2, 1532±2, 1481±2, 1445±2, 1429±2, 1391±2, 1325±2, 1298±2, 1286±2, 1234±2, 1223±2, 1178±2, 1120±2, 1063±2, 1029±2, 1000±2, 983±2, 941±2, 850±2, 832±2, 803±2, 749±2, and 692±2 cm⁻¹.

In one embodiment, the crystalline Form A is characterized by an infrared (IR) spectrum having peaks at 1677±2, 1647±2, 1607±2, 1593±2, 1586±2, 1532±2, 1481±2, 1445±2, 1429±2, 1391±2, 1325±2, 1298±2, 1286±2, 1234±2, 1223±2, 1178±2, 1120±2, 1063±2, 1029±2, 1000±2, 983±2, 941±2, 850±2, 832±2, 803±2, 749±2, and 692±2 cm⁻¹.

In one embodiment, the crystalline Form A can be characterized by a dynamic vapor desorption (DVS) isotherm substantially as shown in FIG. 1C. In one embodiment, the crystalline Form A can be characterized by a dynamic vapor desorption (DVS) isotherm substantially as shown in FIG. 1D.

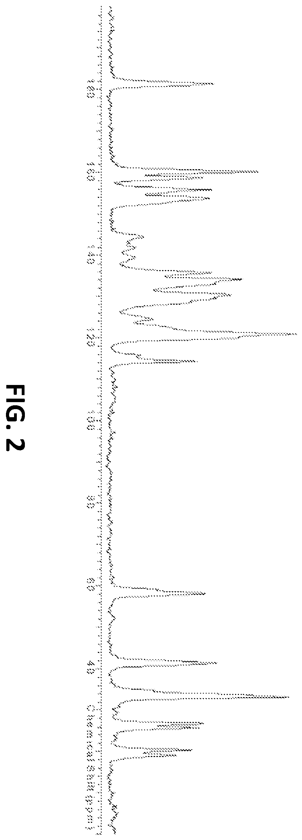

In one embodiment, the crystalline Form A of Compound (I) is characterized by a ¹³C solid state NMR spectrum substantially as shown in FIG. 2, comprising three or more resonances (ppm) values selected from the group consisting of 25.7, 26.8, 58.2, 114.3, 121.0, 158.7, 160.2, and 181.4.

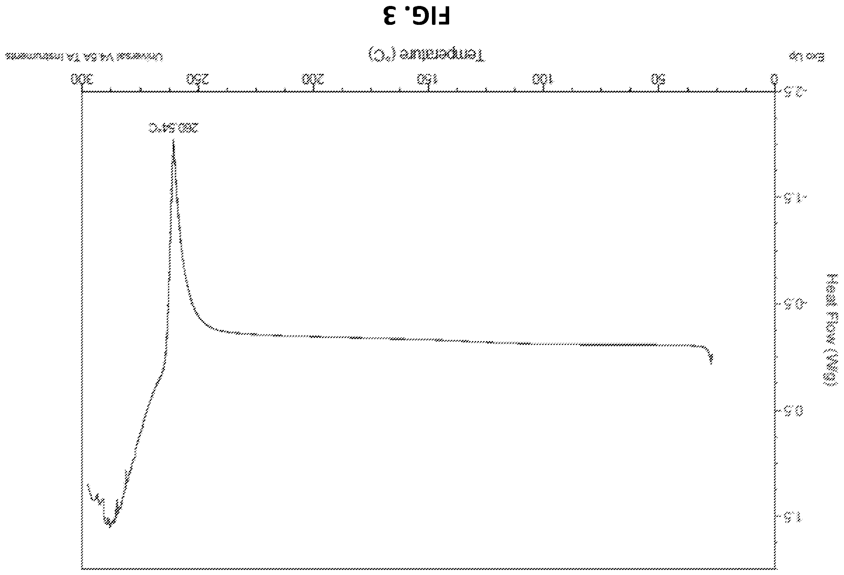

In one embodiment, the crystalline Form A of Compound (I) is characterized by a differential scanning calorimetry (DSC) thermogram substantially as shown in FIG. 3.

In one embodiment, the crystalline Form A can be characterized by a differential scanning calorimetry (DSC) thermogram having an endothermic peak at about 261 °C. In one embodiment, the crystalline Form A can be characterized by a differential scanning calorimetry (DSC) thermogram having an endothermic peak at 260.5 °C.

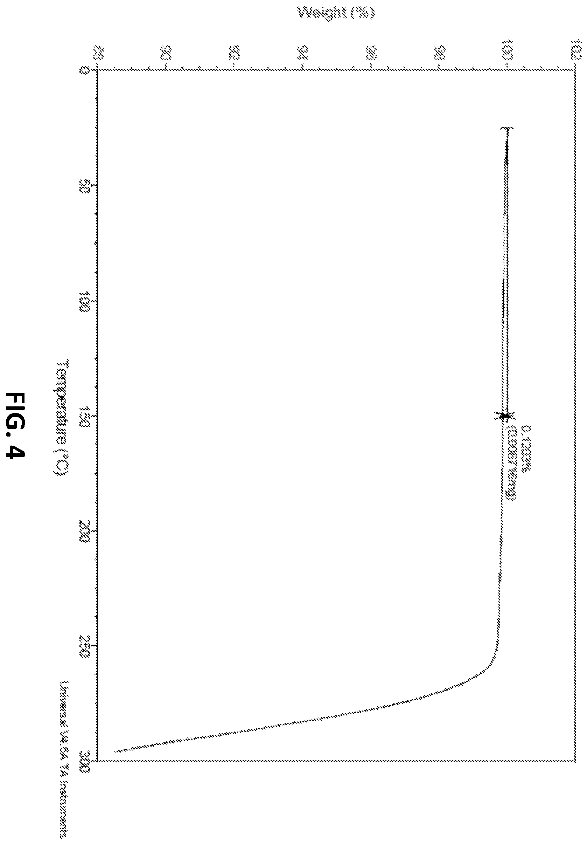

In one embodiment, the crystalline Form A exhibits a thermogravimetric analysis (TGA) thermogram substantially the same as shown in FIG. 4.

In one embodiment, the crystalline form has at least 90 wt% that is in Form A. In one embodiment, the crystalline form consists essentially of Form A.

In another embodiment, the crystalline Form A of Compound (I) is substantially pure.

In another embodiment, the crystalline form of Compound (I) consists essentially of Form A. The crystalline form of this embodiment may comprise at least 90 wt. %, preferably at least 95 wt. %, and more preferably at least 99 wt. %, based on the weight of the crystalline Form A of Compound (I).

In yet another embodiment, a pharmaceutical composition is provided comprising Form A of Compound (I); and at least one pharmaceutically-acceptable carrier and/or diluent.

In still another embodiment, a pharmaceutical composition comprises substantially pure Form A of Compound (I); and at least one pharmaceutically-acceptable carrier and/or diluent.

In still an even further embodiment, a therapeutically effective amount of Form A of Compound (I) is combined with at least one pharmaceutically acceptable carrier and/or diluent to provide at least one pharmaceutical composition.

### Form B of Compound (I)

The crystalline Form B of Compound (I) can be produced through solvent systems such as methanol, methanol-water mixture, ethyl acetate, and acetic acid solution. The Form B solid can be obtained by stirring excess Compound (I) in the aforementioned solvent systems. In one embodiment, the crystalline Form B of Compound (I) is produced through solvent systems such as water, methanol, ethanol, isopropanol, and mixtures thereof. In one embodiment, the crystalline Form B is produced from crystalline Form A in a mixture of water and methanol.

In one embodiment, Compound (I) is provided using the method described above as a crystalline hydrate Form B.

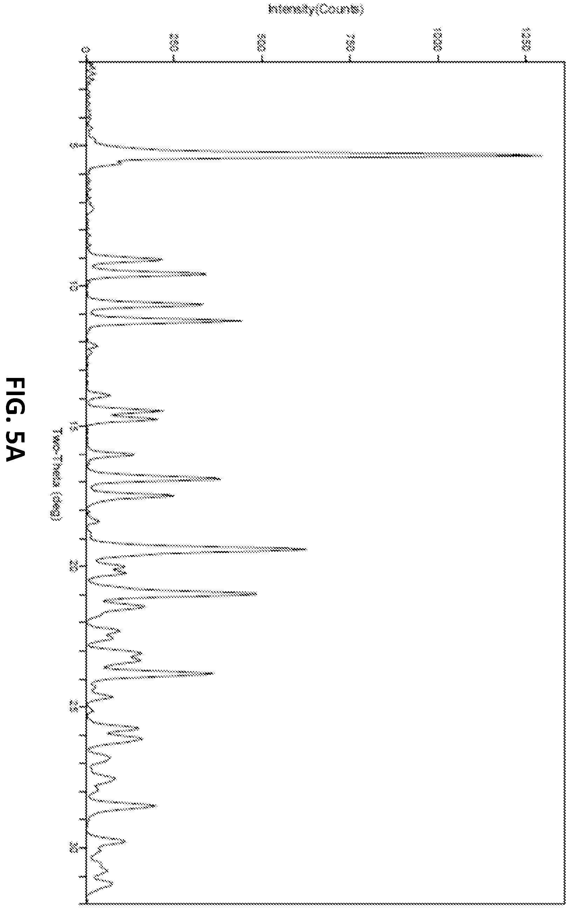

In one embodiment, the crystalline Form B may be characterized by a powder X-ray diffraction pattern comprising the 2θ values (CuKα λ= 1.541874 Å) at room temperature substantially as shown in FIG. 5A.

In one embodiment, the crystalline Form B of Compound (I) is characterized by a PXRD pattern (CuKα λ= 1.541874 Å at room temperature) comprising one, two, three, four, five, six, seven, eight, nine, ten or more 2θ values selected from: 5.3±0.2, 9.0±0.2, 9.5±0.2, 10.6±0.2, 11.2±0.2, 13.9±0.2, 14.4±0.2, 14.7±0.2, 16.0±0.2, 16.8±0.2, 17.4±0.2, 18.3±0.2, 19.3±0.2, 20.2±0.2, 20.9±0.2, 21.3±0.2, 23.0±0.2, 23.8±0.2, 25.8±0.2, and 26.1±0.2, wherein the PXRD pattern of Form B is measured at room temperature.

In one embodiment, the crystalline Form B of Compound (I) is characterized by a PXRD pattern (CuKα λ= 1.541874 Å at room temperature) comprising 2θ values: 5.3±0.2, 10.6±0.2, 11.2±0.2, 16.8±0.2, 19.3±0.2, 20.9±0.2, and 23.8±0.2, wherein the PXRD pattern of Form B is measured at room temperature.

In one embodiment, the crystalline Form B of Compound (I) is characterized by a PXRD pattern (CuKα λ= 1.541874 Å at room temperature) comprising 2θ values: 5.3±0.2, 9.0±0.2, 9.5±0.2, 10.6±0.2, 11.2±0.2, 14.4±0.2, 14.7±0.2, 16.0±0.2, 16.8±0.2, 17.4±0.2, 18.3±0.2, 19.3±0.2, 20.9±0.2, 21.3±0.2, 23.0±0.2, and 23.8±0.2, wherein the PXRD pattern of Form B is measured at room temperature.

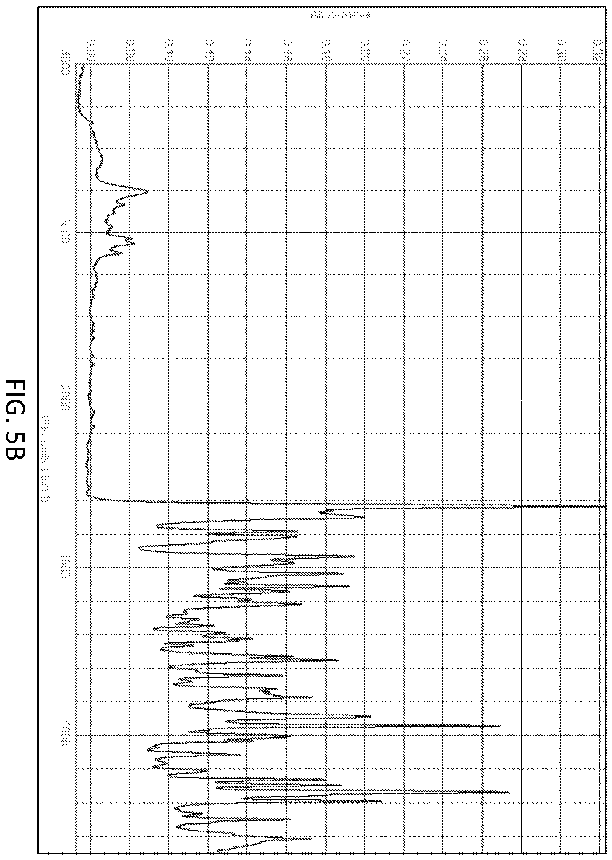

In one embodiment, the crystalline Form B can be characterized by an infrared (IR) spectrum at room temperature substantially as shown in FIG. 5B.

In one embodiment, the crystalline Form B is characterized by an infrared (IR) spectrum having one, two, three, four, five, six, seven, eight, nine, ten or more peaks selected from: 1682±2, 1649±2, 1607±2, 1592±2, 1532±2, 1481±2, 1444±2, 1428±2, 1389±2, 1287±2, 1235±2, 1223±2, 1176±2, 1136±2, 1127±2, 1112±2, 1054±2, 1027±2, 994±2, 983±2, 942±2, 867±2, 850±2, 829±2, 804±2, 749±2, and 690±2 cm⁻¹.

In one embodiment, the crystalline Form B is characterized by an infrared (IR) spectrum having peaks at 1682±2, 1649±2, 1607±2, 1592±2, 1532±2, 1481±2, 1444±2, 1428±2, 1389±2, 1287±2, 1235±2, 1223±2, 1176±2, 1136±2, 1127±2, 1112±2, 1054±2, 102742, 994±2, 983±2, 942±2, 867±2, 850±2, 829±2, 804±2, 749±2, and 690±2 cm⁻¹.

In one embodiment, the crystalline Form B of Compound (I) is characterized by a differential scanning calorimetry (DSC) thermogram having an endothermic peak at about 235 °C. In one embodiment, the crystalline Form B of Compound (I) is characterized by a differential scanning calorimetry (DSC) thermogram having an endothermic peak at 234.6 °C.

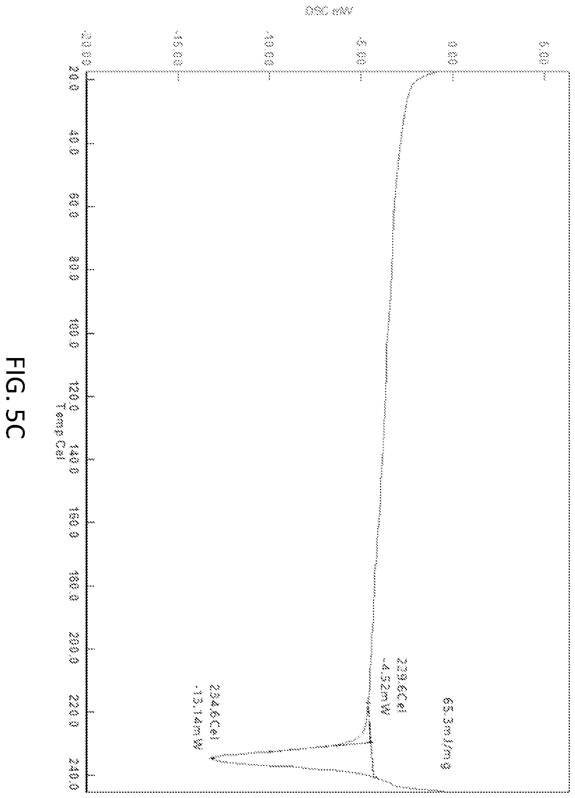

In one embodiment, the crystalline Form B of Compound (I) is characterized by a differential scanning calorimetry (DSC) thermogram substantially as shown in FIG. 5C.

In one embodiment, the crystalline form has at least 90 wt% that is in Form B. In one embodiment, the crystalline form consists essentially of Form B. In one embodiment, the crystalline Form B is substantially pure.

In another embodiment, the crystalline form of Compound (I) consists essentially of Form B. The crystalline form of this embodiment may comprise at least 90 wt. %, preferably at least 95 wt. %, and more preferably at least 99 wt. %, based on the weight of the crystalline Form B of Compound (I).

In yet another embodiment, a pharmaceutical composition is provided comprising Form B of Compound (I); and at least one pharmaceutically-acceptable carrier and/or diluent.

In still another embodiment, a pharmaceutical composition comprises substantially pure Form B of Compound (I); and at least one pharmaceutically-acceptable carrier and/or diluent.

In still an even further embodiment, a therapeutically effective amount of Form B of Compound (I) is combined with at least one pharmaceutically acceptable carrier and/or diluent to provide at least one pharmaceutical composition.

### Form C of Compound (I)

In one embodiment, Compound (I) is provided as a crystalline material comprising Form C. The crystalline Form C of Compound (I) is a free base neat crystalline form.

The crystalline Form C of Compound (I) can be produced through solvent systems such as diethyl ether, water-dimethyl formamide mixture, diisopropyl ether, water, 1-pentanol, acetic acid, and methyl tert-butyl ether solution. For example, the Form C solid can be obtained from a saturated solution of Compound (I) in dimethylformamide (DMF) *via* antisolvent (for example, water) addition and/or cooling.

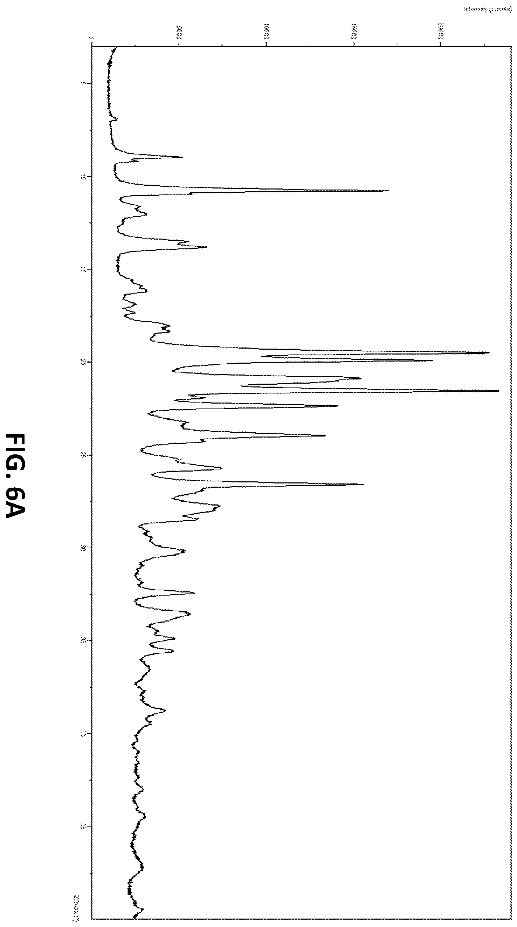

In one embodiment, the crystalline Form C can be characterized by a powder X-ray diffraction pattern comprising the 2θ values (CuKα λ= 1.541874 Å) at room temperature substantially as shown in FIG. 6A.

In one embodiment, the crystalline Form C of Compound (I) is characterized by a PXRD pattern (CuKα λ= 1.541874 Å at room temperature) comprising one, two, three, four, five, six, seven, eight, nine, ten or more 2θ values selected from: 8.9±0.2, 10.8±0.2, 13.5±0.2, 13.8±0.2, 19.5±0.2, 19.9±0.2, 20.8±0.2, 21.6±0.2, 21.9±0.2, 22.3±0.2, 24.0±0.2, 25.8±0.2, and 26.6±0.2, wherein the PXRD pattern of Form C is measured at room temperature.

In one embodiment, the crystalline Form C of Compound (I) is characterized by a PXRD pattern (CuKα λ= 1.541874 Å at room temperature) comprising 2θ values: 10.8±0.2, 19.5±0.2, 19.9±0.2, 20.8±0.2, 21.6±0.2, 22.3±0.2, 24.0±0.2, and 26.6±0.2, wherein the PXRD pattern of Form C is measured at room temperature.

In one embodiment, the crystalline Form C of Compound (I) is characterized by a PXRD pattern (CuKα λ= 1.541874 Å at room temperature) comprising 2θ values: 8.9±0.2, 10.8±0.2, 13.5±0.2, 13.8±0.2, 19.5±0.2, 19.9±0.2, 20.8±0.2, 21.6±0.2, 21.9±0.2, 22.3±0.2, 24.0±0.2, 25.8±0.2, and 26.6±0.2, wherein the PXRD pattern of Form C is measured at room temperature.

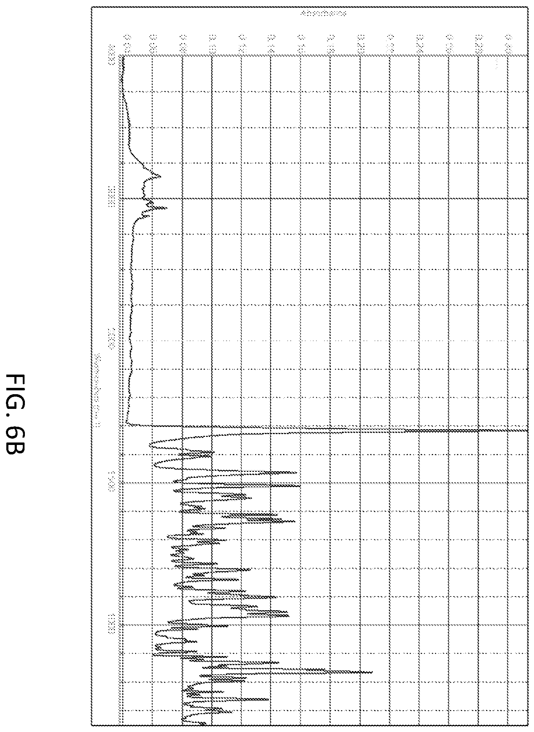

In one embodiment, the crystalline Form C can be characterized by an infrared (IR) spectrum at room temperature substantially as shown in FIG. 6B.

In one embodiment, the crystalline Form C is characterized by an infrared (IR) spectrum having one, two, three, four, five, six, seven, eight, nine, ten or more peaks selected from: 1683±2, 1536±2, 1489±2, 1457±2, 1446±2, 1388±2, 1373±2, 1363±2, 119342, 1119±2, 1098±2, 1065±2, 1045±2, 1032±2, 867±2, 842±2, 832±2, 813±2, 802±2, and 738±2 cm⁻¹.

In one embodiment, the crystalline Form C is characterized by an infrared (IR) spectrum having peaks at 1683±2, 1536±2, 1489±2, 1457±2, 1446±2, 1388±2, 1373±2, 1363±2, 1193±2, 1119±2, 1098±2, 1065±2, 1045±2, 1032±2, 867±2, 842±2, 832±2, 813±2, 802±2, and 738±2 cm⁻¹. In one embodiment, the crystalline Form C is characterized by a differential scanning calorimetry (DSC) thermogram having an onset temperature at about 275 °C.

In one embodiment, the crystalline Form C of Compound (I) is characterized by a differential scanning calorimetry (DSC) thermogram having an endothermic peak at about 277 °C. In one embodiment, the crystalline Form C of Compound (I) is characterized by a differential scanning calorimetry (DSC) thermogram having an endothermic peak at 276.9 °C.

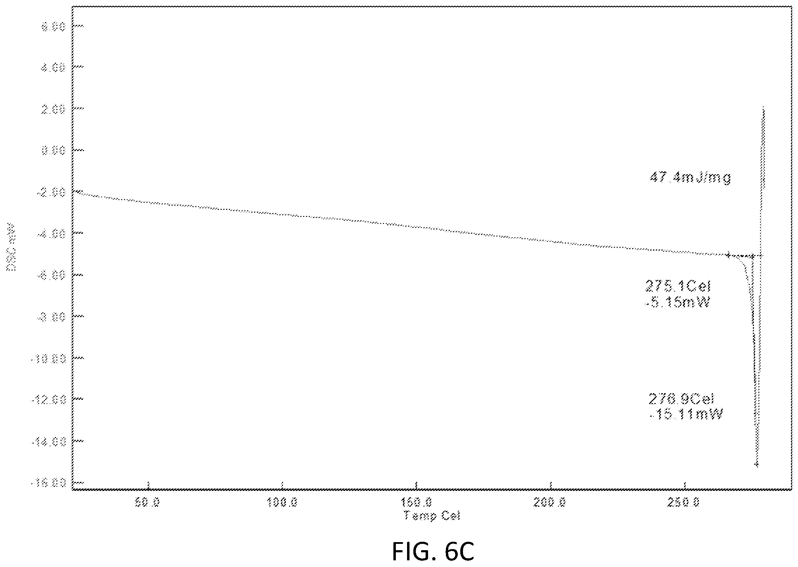

In one embodiment, the crystalline Form C of Compound (I) is characterized by a differential scanning calorimetry (DSC) thermogram substantially as shown in FIG. 6C.

In one embodiment, the crystalline form has at least 90 wt. % that is in Form C. In one embodiment, the crystalline form consists essentially of Form C. In one embodiment, the crystalline Form C is substantially pure.

In another embodiment, the crystalline form of Compound (I) consists essentially of Form C. The crystalline form of this embodiment may comprise at least 90 wt. %, preferably at least 95 wt. %, and more preferably at least 99 wt. %, based on the weight of the crystalline Form C of Compound (I).

In yet another embodiment, a pharmaceutical composition is provided comprising Form C of Compound (I); and at least one pharmaceutically-acceptable carrier and/or diluent.

In still another embodiment, a pharmaceutical composition comprises substantially pure Form C of Compound (I); and at least one pharmaceutically-acceptable carrier and/or diluent.

In still an even further embodiment, a therapeutically effective amount of Form C of Compound (I) is combined with at least one pharmaceutically acceptable carrier and/or diluent to provide at least one pharmaceutical composition.

### Form D of Compound (I)

In one embodiment, Compound (I) is provided as a crystalline material comprising Form D. The crystalline Form D of Compound (I) is a free base neat crystalline form.

The crystalline Form D of Compound (I) can be produced through solvent systems such as 1,4-dioxane. In one embodiment, the crystalline Form D is produced in 1,4-dioxane.

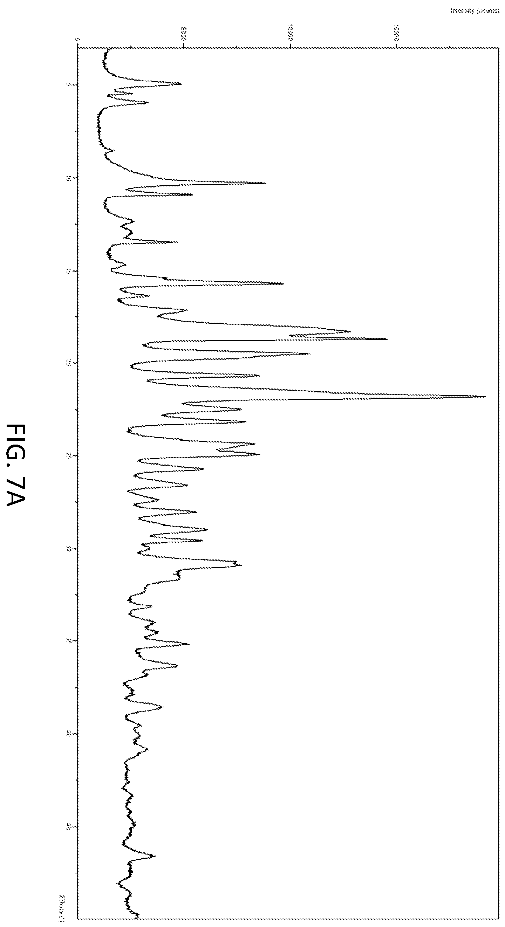

In one embodiment, the crystalline Form D can be characterized by a powder X-ray diffraction pattern comprising the 2θ values (CuKα λ= 1.541874 Å) at room temperature substantially as shown in FIG. 7A.

In one embodiment, the crystalline Form D of Compound (I) is characterized by a PXRD pattern (CuKα λ= 1.541874 Å at room temperature) comprising one, two, three, four, five, six, seven, eight, nine, ten or more 2θ values selected from: 4.9±0.2, 5.5±0.2, 6.0±0.2, 10.3±0.2, 10.9±0.2, 13.5±0.2, 15.7±0.2, 18.3±0.2, 18.7±0.2, 19.5±0.2, 20.7±0.2, 21.8±0.2, 22.5±0.2, 23.2±0.2, 24.3±0.2, 25.0±0.2, 25.7±0.2, 26.6±0.2, 28.0±0.2, and 29.0±0.2, wherein the PXRD pattern of Form D is measured at room temperature.

In one embodiment, the crystalline Form D of Compound (I) is characterized by a PXRD pattern (CuKα λ= 1.541874 Å at room temperature) comprising 2θ values: 10.3±0.2, 15.7±0.2, 18.3±0.2, 18.7±0.2, 19.5±0.2, and 21.8±0.2, wherein the PXRD pattern of Form D is measured at room temperature.

In one embodiment, the crystalline Form D of Compound (I) is characterized by a PXRD pattern (CuKα λ= 1.541874 Å at room temperature) comprising 2θ values: 4.9±0.2, 5.5±0.2, 6.0±0.2, 10.3±0.2, 10.9±0.2, 13.5±0.2, 15.7±0.2, 18.3±0.2, 18.7±0.2, 19.5±0.2, 20.7±0.2, 21.8±0.2, 22.5±0.2, 23.2±0.2, 24.3±0.2, 25.0±0.2, 25.7±0.2, 26.6±0.2, 28.0±0.2, and 29.0±0.2, wherein the PXRD pattern of Form D is measured at room temperature.

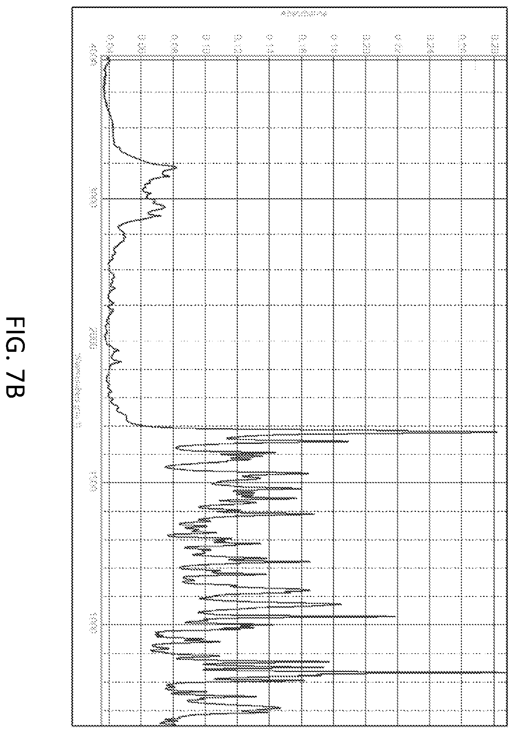

In one embodiment, the crystalline Form D can be characterized by an infrared (IR) spectrum at room temperature substantially as shown in FIG. 7B.

In one embodiment, the crystalline Form D is characterized by an infrared (IR) spectrum having one, two, three, four, five, six, seven, eight, nine, ten or more peaks selected from: 1678±2, 1645±2, 1605±2, 1593±2, 1582±2, 1533±2, 1516±2, 1480±2, 1451±2, 1444±2, 1404±2, 1389±2, 1303±2, 1294±2, 1285±2, 1234±2, 1222±2, 1177±2, 1120±2, 1072±2, 1028±2, 1000±2, 990±2, 983±2, 869±2, 850±2, 832±2, 804±2, 748±2, 708±2, and 692±2 cm⁻¹.

In one embodiment, the crystalline Form D is characterized by an infrared (IR) spectrum having peaks at 1678±2, 1645±2, 1605±2, 1593±2, 1582±2, 1533±2, 1516±2, 1480±2, 1451±2, 1444±2, 1404±2, 1389±2, 1303±2, 1294±2, 1285±2, 1234±2, 1222±2, 1177±2, 1120±2, 107242, 1028±2, 1000±2, 990±2, 983±2, 869±2, 850±2, 832±2, 804±2, 748±2, 708±2, and 692±2 cm⁻¹.

In one embodiment, the crystalline Form D is characterized by a differential scanning calorimetry (DSC) thermogram having an onset temperature at about 247 °C.

In one embodiment, the crystalline Form D of Compound (I) is characterized by a differential scanning calorimetry (DSC) thermogram having an endothermic peak at about 250 °C. In one embodiment, the crystalline Form D of Compound (I) is characterized by a differential scanning calorimetry (DSC) thermogram having an endothermic peak at 250.2 °C.

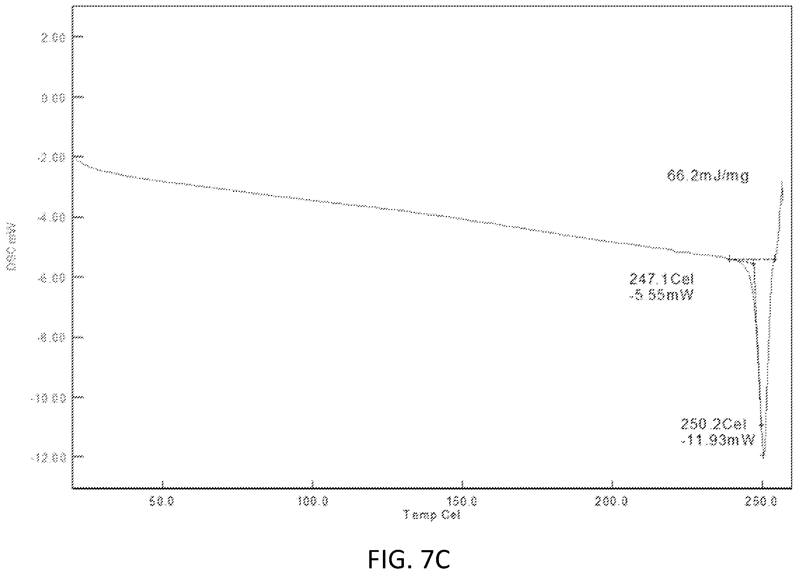

In one embodiment, the crystalline Form D of Compound (I) is characterized by a differential scanning calorimetry (DSC) thermogram substantially as shown in FIG. 7C.

In one embodiment, the crystalline form has at least 90 wt. % that is in Form D. In one embodiment, the crystalline form consists essentially of Form D. In one embodiment, the crystalline Form D is substantially pure.

In another embodiment, the crystalline form of Compound (I) consists essentially of Form D. The crystalline form of this embodiment may comprise at least 90 wt. %, preferably at least 95 wt. %, and more preferably at least 99 wt. %, based on the weight of the crystalline Form D of Compound (I).

In yet another embodiment, a pharmaceutical composition is provided comprising Form D of Compound (I); and at least one pharmaceutically-acceptable carrier and/or diluent.

In still another embodiment, a pharmaceutical composition comprises substantially pure Form D of Compound (I); and at least one pharmaceutically-acceptable carrier and/or diluent.

In still an even further embodiment, a therapeutically effective amount of Form D of Compound (I) is combined with at least one pharmaceutically acceptable carrier and/or diluent to provide at least one pharmaceutical composition.

### Form E of Compound (I)

In one embodiment, Compound (I) is provided as a crystalline material comprising Form E. The crystalline Form E of Compound (I) is a free base neat crystalline form.

The crystalline Form E of Compound (I) can be produced through solvent systems such as acetonitrile, propionitrile, and mixtures thereof. In one embodiment, the crystalline Form E is produced in acetonitrile via slow solvent evaporation.

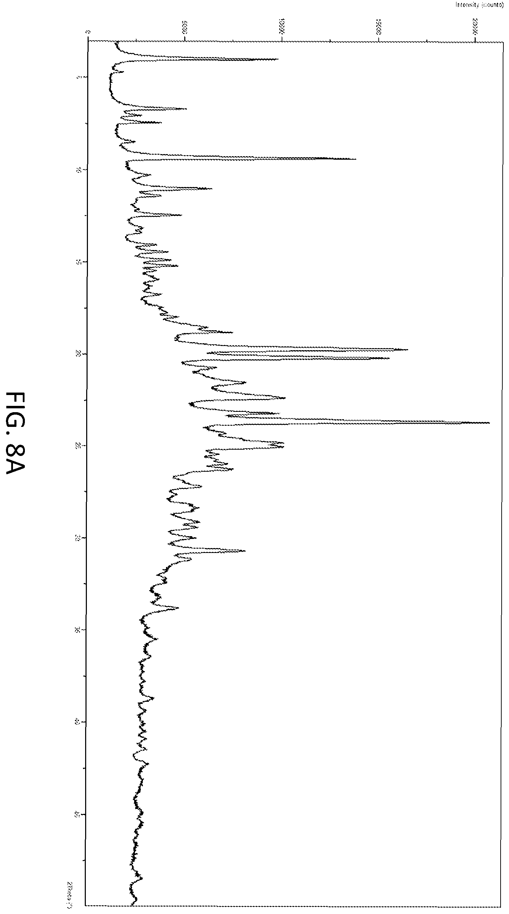

In one embodiment, the crystalline Form E can be characterized by a powder X-ray diffraction pattern comprising the 2θ values (CuKα λ= 1.541874 Å) at room temperature substantially as shown in FIG. 8A.

In one embodiment, the crystalline Form E of Compound (I) is characterized by a PXRD pattern (CuKα λ= 1.541874 Å at room temperature) comprising one, two, three, four, five, six, seven, eight, nine, ten or more 2θ values selected from: 4.0±0.2, 6.7±0.2, 7.0±0.2, 7.4±0.2, 9.4±0.2, 11.0±0.2, 12.5±0.2, 14.4±0.2, 14.9±0.2, 15.2±0.2, 18.8±0.2, 19.7±0.2, 20.2±0.2, 21.5±0.2, 22.4±0.2, 23.2±0.2, and 23.7±0.2, wherein the PXRD pattern of Form E is measured at room temperature.

In one embodiment, the crystalline Form E of Compound (I) is characterized by a PXRD pattern (CuKα λ= 1.541874 Å at room temperature) comprising 2θ values: 4.0±0.2, 9.4±0.2, 19.7±0.2, 20.2±0.2, and 23.7±0.2, wherein the PXRD pattern of Form E is measured at room temperature.

In one embodiment, the crystalline Form E of Compound (I) is characterized by a PXRD pattern (CuKα λ= 1.541874 Å at room temperature) comprising 2θ values: 4.0±0.2, 6.7±0.2, 7.0±0.2, 7.4±0.2, 9.4±0.2, 11.0±0.2, 12.5±0.2, 14.4±0.2, 14.9±0.2, 15.2±0.2, 18.8±0.2, 19.7±0.2, 20.2±0.2, 21.5±0.2, 22.4±0.2, 23.2±0.2, and 23.7±0.2, wherein the PXRD pattern of Form E is measured at room temperature.

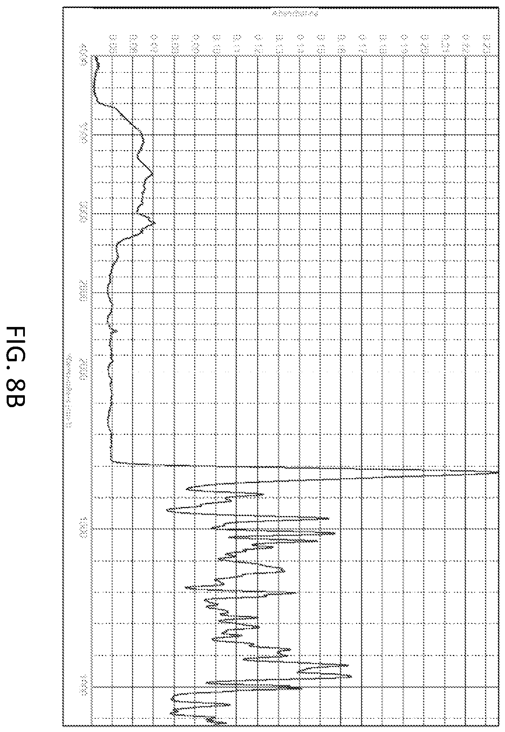

In one embodiment, the crystalline Form E can be characterized by an infrared (IR) spectrum at room temperature substantially as shown in FIG. 8B.

In one embodiment, the crystalline Form E is characterized by an infrared (IR) spectrum having one, two, three, four, five, six, seven, eight, nine, ten or more peaks selected from: 1678±2, 1610±2, 1590±2, 1533±2, 1486±2, 1461±2, 1442±2, 1373±2, 1366±2, 1296±2, 1218±2, 1187±2, 1161±2, 1116±2, 1097±2, 1066±2, 1031±2, 1002±2, 994±2, 941±2, 865±2, 832±2, 803±2, 770±2, 738±2, 694±2, and 651±2 cm⁻¹.

In one embodiment, the crystalline Form E is characterized by an infrared (IR) spectrum having peaks at 1678±2, 1610±2, 1590±2, 1533±2, 1486±2, 1461±2, 1442±2, 1373±2, 1366±2, 1296±2, 1218±2, 1187±2, 1161±2, 1116±2, 1097±2, 1066±2, 1031±2, 1002±2, 994±2, 941±2, 865±2, 832±2, 803±2, 770±2, 738±2, 694±2, and 651±2 cm⁻¹.

In one embodiment, the crystalline form has at least 90 wt. % that is in Form E. In one embodiment, the crystalline form consists essentially of Form E. In one embodiment, the crystalline Form E is substantially pure.

In another embodiment, the crystalline form of Compound (I) consists essentially of Form E. The crystalline form of this embodiment may comprise at least 90 wt. %, preferably at least 95 wt. %, and more preferably at least 99 wt. %, based on the weight of the crystalline Form E of Compound (I).

In yet another embodiment, a pharmaceutical composition is provided comprising Form E of Compound (I); and at least one pharmaceutically-acceptable carrier and/or diluent.

In still another embodiment, a pharmaceutical composition comprises substantially pure Form E of Compound (I); and at least one pharmaceutically-acceptable carrier and/or diluent.

In still an even further embodiment, a therapeutically effective amount of Form E of Compound (I) is combined with at least one pharmaceutically acceptable carrier and/or diluent to provide at least one pharmaceutical composition.

### Form F of Compound (I)

In one embodiment, Compound (I) is provided as a crystalline material comprising Form F. The crystalline Form F of Compound (I) is a free base neat crystalline form.

The crystalline Form F of Compound (I) can be produced through solvent systems such as isopropyl ether, pyridine, methyl tert-butyl ether, and mixtures thereof, and subsequent desolvation of the resulting solvate solids (e.g., by slurrying in heptane or ethanol). The Form F solid can be obtained by stirring excess of Compound (I) in heptane or ethanol. In one embodiment, the crystalline Form F is produced by slurrying solid Compound (I) in heptane.

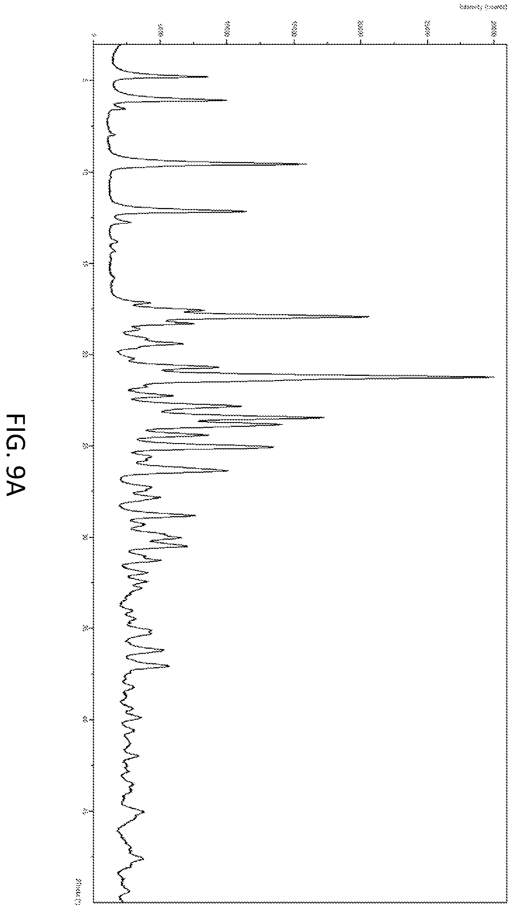

In one embodiment, the crystalline Form F can be characterized by a powder X-ray diffraction pattern comprising the 2θ values (CuKα λ= 1.541874 Å) at room temperature substantially as shown in FIG. 9A.

In one embodiment, the crystalline Form F of Compound (I) is characterized by a PXRD pattern (CuKα λ= 1.541874 Å at room temperature) comprising one, two, three, four, five, six, seven, eight, nine, ten or more 2θ values selected from: 4.8±0.2, 6.1±0.2, 9.6±0.2, 12.2±0.2, 17.2±0.2, 17.6±0.2, 18.0±0.2, 18.3±0.2, 19.4±0.2, 20.7±0.2, 21.2±0.2, 22.3±0.2, 22.8±0.2, 23.4±0.2, 23.8±0.2, 24.4±0.2, 25.1±0.2, 26.4±0.2, and 28.8±0.2, wherein the PXRD pattern of Form F is measured at room temperature.

In one embodiment, the crystalline Form F of Compound (I) is characterized by a PXRD pattern (CuKα λ= 1.541874 Å at room temperature) comprising 2θ values: 9.6±0.2, 18.0±0.2, 21.2±0.2, 23.4±0.2, and 23.8±0.2, wherein the PXRD pattern of Form F is measured at room temperature.

In one embodiment, the crystalline Form F of Compound (I) is characterized by a PXRD pattern (CuKα λ= 1.541874 Å at room temperature) comprising 2θ values: 4.8±0.2, 6.1±0.2, 9.6±0.2, 12.2±0.2, 17.2±0.2, 17.6±0.2, 18.0±0.2, 18.3±0.2, 19.4±0.2, 20.7±0.2, 21.2±0.2, 22.3±0.2, 22.8±0.2, 23.4±0.2, 23.8±0.2, 24.4±0.2, 25.1±0.2, 26.4±0.2, and 28.8±0.2, wherein the PXRD pattern of Form F is measured at room temperature.

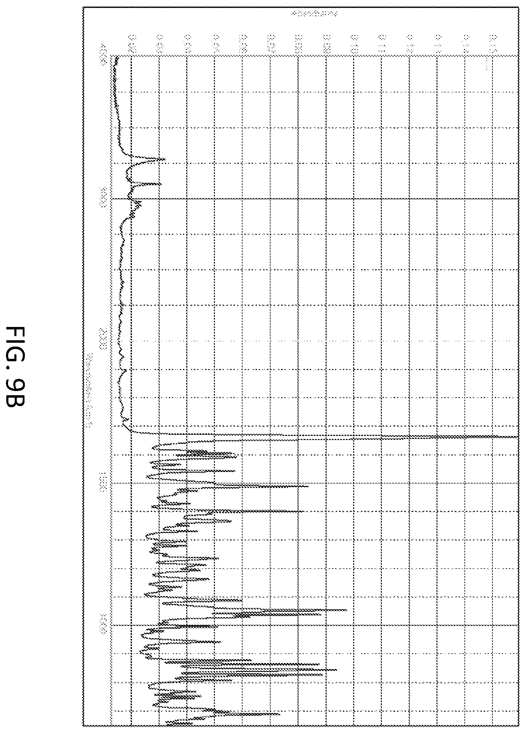

In one embodiment, the crystalline Form F can be characterized by an infrared (IR) spectrum at room temperature substantially as shown in FIG. 9B.

In one embodiment, the crystalline Form F is characterized by an infrared (IR) spectrum having one, two, three, four, five, six, seven, eight, nine, ten or more peaks selected from: 1662±2, 1604±2, 1591±2, 1542±2, 1487±2, 1400±2, 1364±2, 1087±2, 1052±2, 1037±2, 1028±2, 876±2, 863±2, 843±2, 839±2, 825±2, 807±2, 697±2, and 688±2 cm⁻¹.

In one embodiment, the crystalline Form F is characterized by an infrared (IR) spectrum having peaks at 1662±2, 1604±2, 1591±2, 1542±2, 1487±2, 1400±2, 1364±2, 1087±2, 1052±2, 1037±2, 1028±2, 876±2, 863±2, 843±2, 839±2, 825±2, 807±2, 697±2, and 688±2 cm⁻¹.

In one embodiment, the crystalline Form F is characterized by a differential scanning calorimetry (DSC) thermogram having an onset temperature at about 237 °C.

In one embodiment, the crystalline Form F of Compound (I) is characterized by a differential scanning calorimetry (DSC) thermogram having an endothermic peak at about 242 °C. In one embodiment, the crystalline Form F of Compound (I) is characterized by a differential scanning calorimetry (DSC) thermogram having an endothermic peak at 241.7 °C.

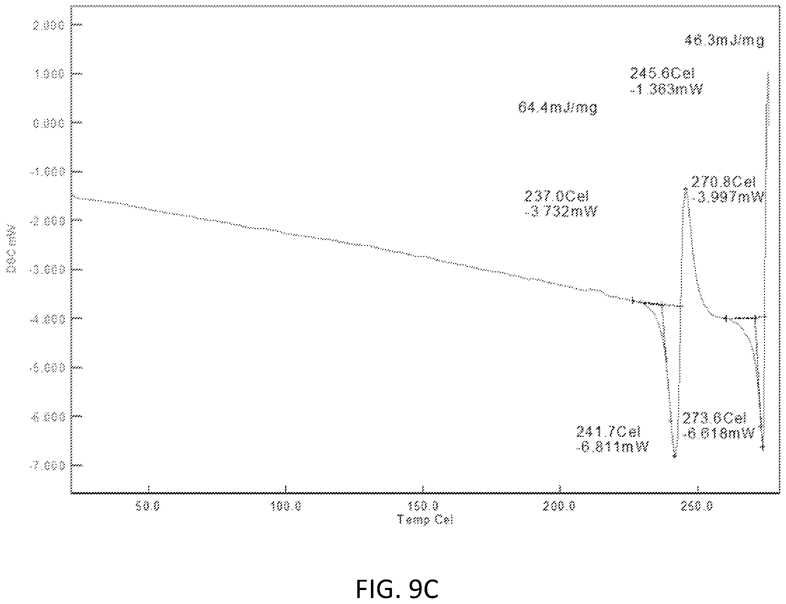

In one embodiment, the crystalline Form F of Compound (I) is characterized by a differential scanning calorimetry (DSC) thermogram substantially as shown in FIG. 9C.

In one embodiment, the crystalline form has at least 90 wt. % that is in Form F. In one embodiment, the crystalline form consists essentially of Form F. In one embodiment, the crystalline Form F is substantially pure.

In another embodiment, the crystalline form of Compound (I) consists essentially of Form F. The crystalline form of this embodiment may comprise at least 90 wt. %, preferably at least 95 wt. %, and more preferably at least 99 wt. %, based on the weight of the crystalline Form F of Compound (I).

In yet another embodiment, a pharmaceutical composition is provided comprising Form F of Compound (I); and at least one pharmaceutically-acceptable carrier and/or diluent.

In still another embodiment, a pharmaceutical composition comprises substantially pure Form F of Compound (I); and at least one pharmaceutically-acceptable carrier and/or diluent.

In still an even further embodiment, a therapeutically effective amount of Form F of Compound (I) is combined with at least one pharmaceutically acceptable carrier and/or diluent to provide at least one pharmaceutical composition.

### Form G of Compound (I)

In one embodiment, Compound (I) is provided as a crystalline material comprising Form G.

The crystalline Form G of Compound (I) can be produced through solvent systems such as acetonitrile, propionitrile, water, and mixtures thereof. In one embodiment, the crystalline Form G is produced by crystallization of the solution of Compound (I) in acetonitrile and water.

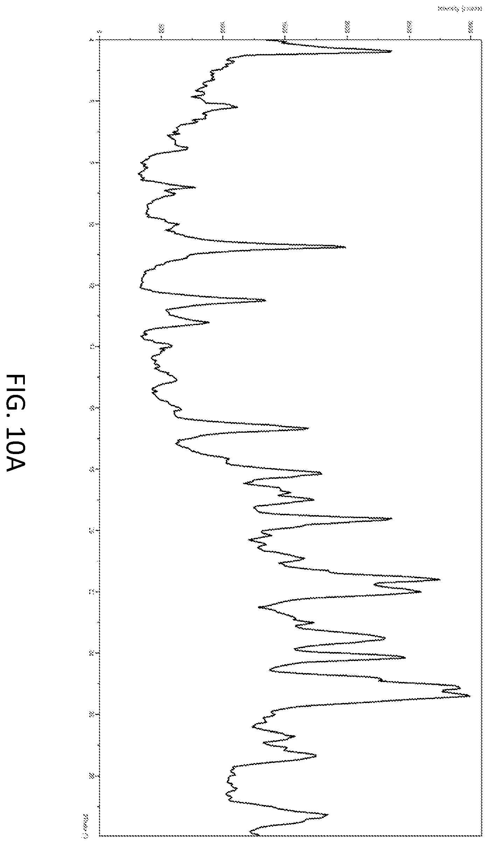

In one embodiment, the crystalline Form G can be characterized by a powder X-ray diffraction pattern comprising the 2θ values (CuKα λ= 1.541874 Å) at room temperature substantially as shown in FIG. 10A.

In one embodiment, the crystalline Form G of Compound (I) is characterized by a PXRD pattern (CuKα λ= 1.541874 Å at room temperature) comprising one, two, three, four, five, six, seven, eight, nine, ten or more 2θ values selected from: 4.4±0.2, 6.2±0.2, 10.7±0.2, 12.5±0.2, 13.2±0.2, 16.7±0.2, 17.6±0.2, 18.1±0.2, 18.7±0.2, 19.0±0.2, 19.6±0.2, 20.9±0.2, 21.6±0.2, 22.0±0.2, 23.0±0.2, 23.5±0.2, 24.1±0.2, 24.8±0.2, 25.1±0.2, and 25.4±0.2, wherein the PXRD pattern of Form G is measured at room temperature.

In one embodiment, the crystalline Form G of Compound (I) is characterized by a PXRD pattern (CuKα λ= 1.541874 Å at room temperature) comprising 2θ values: 4.4±0.2, 10.7±0.2, 16.7±0.2, 18.1±0.2, 19.6±0.2, 21.6±0.2, 22.0±0.2, 23.5±0.2, 24.1±0.2, 24.8±0.2, 25.1±0.2, and 25.4±0.2, wherein the PXRD pattern of Form G is measured at room temperature.

In one embodiment, the crystalline Form G of Compound (I) is characterized by a PXRD pattern (CuKα λ= 1.541874 Å at room temperature) comprising 2θ values: 4.4±0.2, 6.2±0.2, 10.7±0.2, 12.5±0.2, 13.2±0.2, 16.7±0.2, 17.6±0.2, 18.1±0.2, 18.7±0.2, 19.0±0.2, 19.6±0.2, 20.9±0.2, 21.6±0.2, 22.0±0.2, 23.0±0.2, 23.5±0.2, 24.1±0.2, 24.8±0.2, 25.1±0.2, and 25.4±0.2, wherein the PXRD pattern of Form G is measured at room temperature.

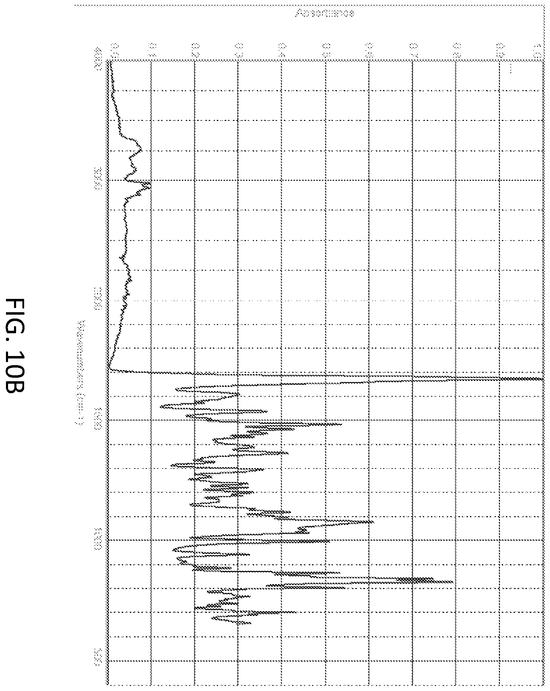

In one embodiment, the crystalline Form G can be characterized by an infrared (IR) spectrum at room temperature substantially as shown in FIG. 10B.

In one embodiment, the crystalline Form G is characterized by an infrared (IR) spectrum having one, two, three, four, five, six, seven, eight, nine, ten or more peaks selected from: 1680±2, 1672±2, 1609±2, 1607±2, 1535±2, 1533±2, 1488±2, 1461±2, 1441±2, 1367±2, 1118±2, 1096±2, 1066±2, 1029±2, 993±2, 866±2, 836±2, 804±2, 738±2, and 694±2 cm⁻¹.

In one embodiment, the crystalline Form G is characterized by an infrared (IR) spectrum having peaks at 1680±2, 1672±2, 1609±2, 1607±2, 1535±2, 1533±2, 1488±2, 1461±2, 1441±2, 1367±2, 1118±2, 1096±2, 1066±2, 1029±2, 993±2, 866±2, 836±2, 804±2, 738±2, and 694±2 cm⁻¹.

In one embodiment, the crystalline form has at least 90 wt% that is in Form G. In one embodiment, the crystalline form consists essentially of Form G. In one embodiment, the crystalline Form G is substantially pure.

In another embodiment, the crystalline form of Compound (I) consists essentially of Form G. The crystalline form of this embodiment may comprise at least 90 wt. %, preferably at least 95 wt. %, and more preferably at least 99 wt. %, based on the weight of the crystalline Form G of Compound (I).

In yet another embodiment, a pharmaceutical composition is provided comprising Form G of Compound (I); and at least one pharmaceutically-acceptable carrier and/or diluent.

In still another embodiment, a pharmaceutical composition comprises substantially pure Form G of Compound (I); and at least one pharmaceutically-acceptable carrier and/or diluent.

In still an even further embodiment, a therapeutically effective amount of Form G of Compound (I) is combined with at least one pharmaceutically acceptable carrier and/or diluent to provide at least one pharmaceutical composition.

### Form H of Compound (I)

In one embodiment, Compound (I) is provided as a crystalline material comprising Form H. The crystalline Form H of Compound (I) is a free base methyl acetate solvate.

The crystalline Form H of Compound (I) can be produced through solvent systems such as methyl acetate, ethyl acetate, ethyl propionate, and mixtures thereof. In one embodiment, the crystalline Form H is produced by crystallization of the solution of Compound (I) in methyl acetate. In one embodiment, the crystalline Form H is produced by slurrying of amorphous Compound (I) at 5 °C in methyl acetate.

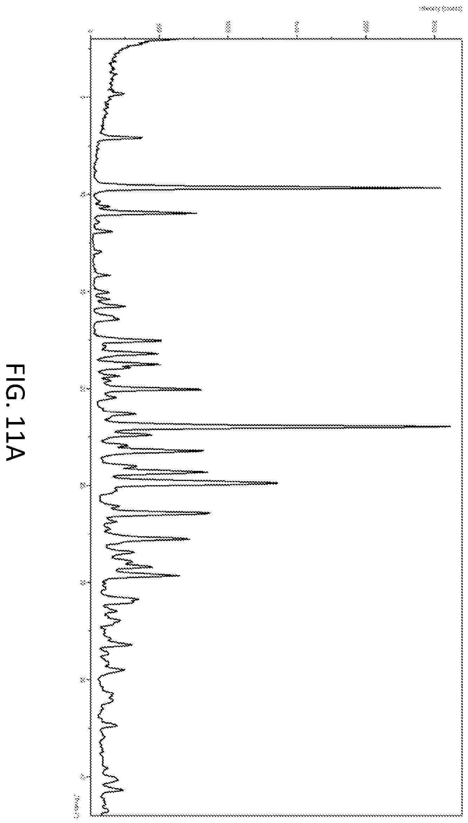

In one embodiment, the crystalline Form H can be characterized by a powder X-ray diffraction pattern comprising the 2θ values (CuKα λ= 1.541874 Å) at room temperature substantially as shown in FIG. 11A.

In one embodiment, the crystalline Form H of Compound (I) is characterized by a PXRD pattern (CuKα λ= 1.541874 Å at room temperature) comprising one, two, three, four, five, six, seven, eight, nine, ten or more 2θ values selected from: 7.1±0.2, 9.6±0.2, 10.9±0.2, 17.5±0.2, 18.2±0.2, 18.7±0.2, 20.0±0.2, 22.0±0.2, 22.4±0.2, 23.2±0.2, 24.3±0.2, 24.9±0.2, 26.4±0.2, 27.7±0.2, and 29.6±0.2, wherein the PXRD pattern of Form H is measured at room temperature.

In one embodiment, the crystalline Form H of Compound (I) is characterized by a PXRD pattern (CuKα λ= 1.541874 Å at room temperature) comprising 2θ values: 9.6±0.2, 10.9±0.2, 20.0±0.2, 22.0±0.2, 23.2±0.2, 24.3±0.2, 24.9±0.2, and 26.4±0.2, wherein the PXRD pattern of Form H is measured at room temperature.

In one embodiment, the crystalline Form H of Compound (I) is characterized by a PXRD pattern (CuKα λ= 1.541874 Å at room temperature) comprising 2θ values: 7.1±0.2, 9.6±0.2, 10.9±0.2, 17.5±0.2, 18.2±0.2, 18.7±0.2, 20.0±0.2, 22.0±0.2, 22.4±0.2, 23.2±0.2, 24.3±0.2, 24.9±0.2, 26.4±0.2, 27.7±0.2, and 29.6±0.2, wherein the PXRD pattern of Form H is measured at room temperature.

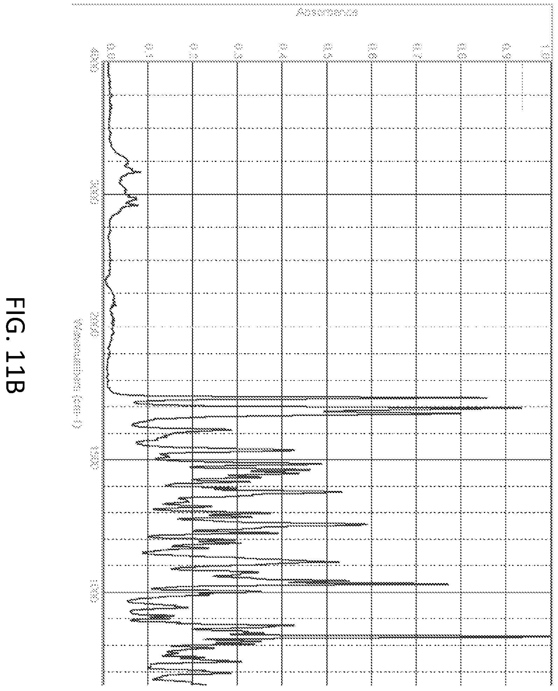

In one embodiment, the crystalline Form H can be characterized by an infrared (IR) spectrum at room temperature substantially as shown in FIG. 11B.

In one embodiment, the crystalline Form H is characterized by an infrared (IR) spectrum having one, two, three, four, five, six, seven, eight, nine, ten or more peaks selected from: 1732±2, 1694±2, 1673±2, 1534±2, 1483±2, 1461±2, 1448±2, 1433±2, 1416±2, 1377±2, 1297±2, 1283±2, 1254±2, 1223±2, 1184±2, 1113±2, 1074±2, 1041±2, 1029±2, 1001±2, 873±2, 850±2, 844±2, 831±2, 814±2, 803±2, and 738±2 cm⁻¹.

In one embodiment, the crystalline Form H is characterized by an infrared (IR) spectrum having peaks at 1732±2, 1694±2, 1673±2, 1534±2, 1483±2, 1461±2, 1448±2, 1433±2, 1416±2, 1377±2, 1297±2, 1283±2, 1254±2, 1223±2, 1184±2, 1113±2, 1074±2, 1041±2, 1029±2, 1001±2, 873±2, 850±2, 844±2, 831±2, 814±2, 803±2, and 738±2 cm⁻¹.

In one embodiment, the crystalline form has at least 90 wt. % that is in Form H. In one embodiment, the crystalline form consists essentially of Form H. In one embodiment, the crystalline Form H is substantially pure.

In another embodiment, the crystalline form of Compound (I) consists essentially of Form H. The crystalline form of this embodiment may comprise at least 90 wt. %, preferably at least 95 wt. %, and more preferably at least 99 wt. %, based on the weight of the crystalline Form H of Compound (I).

In yet another embodiment, a pharmaceutical composition is provided comprising Form H of Compound (I); and at least one pharmaceutically-acceptable carrier and/or diluent.

In still another embodiment, a pharmaceutical composition comprises substantially pure Form H of Compound (I); and at least one pharmaceutically-acceptable carrier and/or diluent.

In still an even further embodiment, a therapeutically effective amount of Form H of Compound (I) is combined with at least one pharmaceutically acceptable carrier and/or diluent to provide at least one pharmaceutical composition.

### Form I of Compound (I)

In one embodiment, Compound (I) is provided as a crystalline material comprising Form I.

The crystalline Form I of Compound (I) can be produced by storing Form H under vacuum at room temperature or by storing Form H at elevated temperature. In one embodiment, the crystalline Form I is produced by heating crystalline Form H.

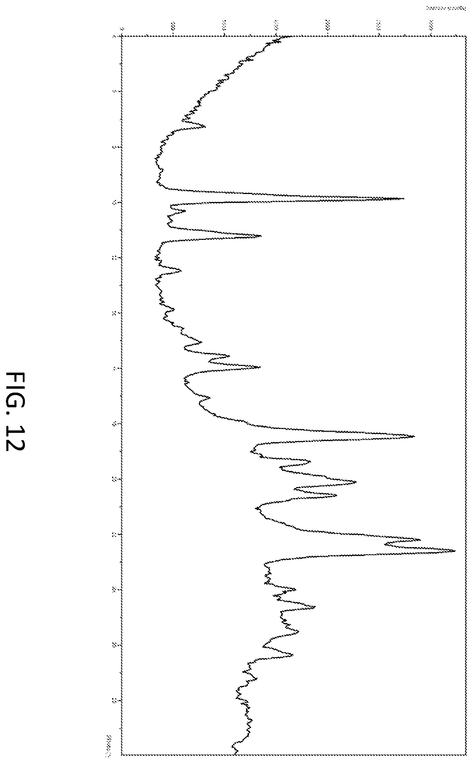

In one embodiment, the crystalline Form I can be characterized by a powder X-ray diffraction pattern comprising the 2θ values (CuKα λ= 1.541874 Å) at room temperature substantially as shown in FIG. 12.

In one embodiment, the crystalline Form I of Compound (I) is characterized by a PXRD pattern (CuKα λ= 1.541874 Å at room temperature) comprising one, two, three, four, five, six, seven, eight, nine, ten or more 2θ values selected from: 7.2±0.2, 9.9±0.2, 11.2±0.2, 15.5±0.2, 16.0±0.2, 18.5±0.2, 19.4±0.2, 20.1±0.2, 20.6±0.2, 22.2±0.2, 22.6±0.2, 24.0±0.2, 24.6±0.2, 25.5±0.2, and 26.4±0.2, wherein the PXRD pattern of Form I is measured at room temperature.

In one embodiment, the crystalline Form I of Compound (I) is characterized by a PXRD pattern (CuKα λ= 1.541874 Å at room temperature) comprising 2θ values: 9.9±0.2, 11.2±0.2, 18.5±0.2, 20.1±0.2, 20.6±0.2, 22.2±0.2, and 22.6±0.2, wherein the PXRD pattern of Form I is measured at room temperature.

In one embodiment, the crystalline Form I of Compound (I) is characterized by a PXRD pattern (CuKα λ= 1.541874 Å at room temperature) comprising 2θ values: 7.2±0.2, 9.9±0.2, 11.2±0.2, 15.5±0.2, 16.0±0.2, 18.5±0.2, 19.4±0.2, 20.1±0.2, 20.6±0.2, 22.2±0.2, 22.6±0.2, 24.0±0.2, 24.6±0.2, 25.5±0.2, and 26.4±0.2, wherein the PXRD pattern of Form I is measured at room temperature.

In one embodiment, the crystalline Form I is characterized by an infrared (IR) spectrum having one, two, three, four, five, six, seven, eight, nine, ten or more peaks selected from: 1682±2, 1534±2, 1485±2, 1461±2, 1380±2, 1118±2, 1106±2, 1066±2, 1041±2, 1032±2, 1002±2, 865±2, 832±2, and 803±2 cm⁻¹.

In one embodiment, the crystalline Form I is characterized by an infrared (IR) spectrum having peaks at 1682±2, 1534±2, 1485±2, 1461±2, 1380±2, 1118±2, 1106±2, 1066±2, 1041±2, 1032±2, 1002±2, 865±2, 832±2, and 803±2 cm⁻¹.

In one embodiment, the crystalline form has at least 90 wt. % that is in Form I. In one embodiment, the crystalline form consists essentially of Form I. In one embodiment, the crystalline Form I is substantially pure.

In another embodiment, the crystalline form of Compound (I) consists essentially of Form I. The crystalline form of this embodiment may comprise at least 90 wt. %, preferably at least 95 wt. %, and more preferably at least 99 wt. %, based on the weight of the crystalline Form I of Compound (I).

In yet another embodiment, a pharmaceutical composition is provided comprising Form H of Compound (I); and at least one pharmaceutically-acceptable carrier and/or diluent.

In still another embodiment, a pharmaceutical composition comprises substantially pure Form H of Compound (I); and at least one pharmaceutically-acceptable carrier and/or diluent.

In still an even further embodiment, a therapeutically effective amount of Form H of Compound (I) is combined with at least one pharmaceutically acceptable carrier and/or diluent to provide at least one pharmaceutical composition.

### Form J of Compound (I)

In one embodiment, Compound (I) is provided as a crystalline material comprising Form J. The crystalline Form J of Compound (I) is a free base neat crystalline form.

The crystalline Form J of Compound (I) can be produced through solvent systems such as ethylene glycol, 1-pentanol, 1-propanol, triethylamine, water, ethanol, acetone, propan-1,2-diol, and mixtures thereof. In one embodiment, the crystalline Form J is produced by temperature cycling of Form G and in an acetonitrile/water mixture at elevated temperature. The Form J solid can be obtained by stirring excess Compound (I), e.g. Form G or H, in the heptane or acetonitrile/water solvent systems.

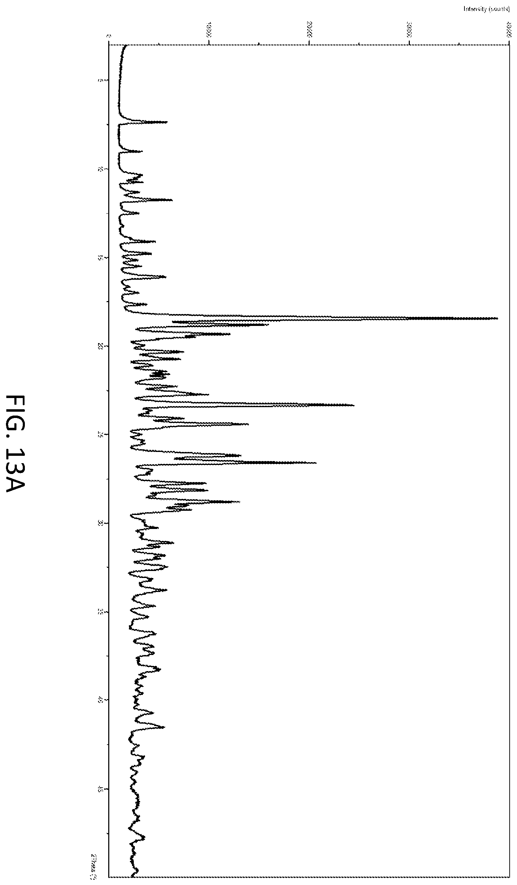

In one embodiment, the crystalline Form J can be characterized by a powder X-ray diffraction pattern comprising the 2θ values (CuKα λ= 1.541874 Å) at room temperature substantially as shown in FIG. 13A.

In one embodiment, the crystalline Form J of Compound (I) is characterized by a PXRD pattern (CuKα λ= 1.541874 Å at room temperature) comprising one, two, three, four, five, six, seven, eight, nine, ten or more 2θ values selected from: 7.4±0.2, 9.0±0.2, 11.3±0.2, 11.7±0.2, 12.5±0.2, 14.8±0.2, 15.1±0.2, 15.5±0.2, 16.1±0.2, 17.6±0.2, 18.4±0.2, 18.8±0.2, 19.3±0.2, 23.3±0.2, 24.4±0.2, 26.2±0.2, 26.6±0.2, 27.7±0.2, 28.2±0.2, and 28.8±0.2, wherein the PXRD pattern of Form J is measured at room temperature.

In one embodiment, the crystalline Form J of Compound (I) is characterized by a PXRD pattern (CuKα λ= 1.541874 Å at room temperature) comprising 2θ values at 18.4±0.2, 18.8±0.2, 19.3±0.2, 23.3±0.2, 24.4±0.2, 26.2±0.2, 26.6±0.2, and 28.8±0.2, wherein the PXRD pattern of Form J is measured at room temperature.

In one embodiment, the crystalline Form J of Compound (I) is characterized by a PXRD pattern (CuKα λ= 1.541874 Å at room temperature) comprising 2θ values at 7.4±0.2, 9.0±0.2, 11.3±0.2, 11.7±0.2, 12.5±0.2, 14.8±0.2, 15.1±0.2, 15.5±0.2, 16.1±0.2, 17.6±0.2, 18.4±0.2, 18.8±0.2, 19.3±0.2, 23.3±0.2, 24.4±0.2, 26.2±0.2, 26.6±0.2, 27.7±0.2, 28.2±0.2, and 28.8±0.2, wherein the PXRD pattern of Form J is measured at room temperature.

In one embodiment, the crystalline Form J is characterized by an infrared (IR) spectrum having one, two, three, four, five, six, seven, eight, nine, ten or more peaks selected from: 1678±2, 1610±2, 1584±2, 1536±2, 1525±2, 1486±2, 1462±2, 1412±2, 1392±2, 1371±2, 1346±2, 1296±2, 1286±2, 1234±2, 1223±2, 1196±2, 1165±2, 1114±2, 1099±2, 1078±2, 1065±2, 1052±2, 1034±2, 999±2, 867±2, 858±2, 841±2, 833±2, 814±2, 800±2, 789±2, 736±2, 703±2, and 694±2 cm⁻¹.

In one embodiment, the crystalline Form J is characterized by an infrared (IR) spectrum having peaks at 1678±2, 1610±2, 1584±2, 1536±2, 1525±2, 1486±2, 1462±2, 1412±2, 1392±2, 1371±2, 134612, 1296±2, 1286±2, 1234±2, 1223±2, 1196±2, 1165±2, 1114±2, 1099±2, 1078±2, 1065±2, 1052±2, 1034±2, 999±2, 867±2, 858±2, 841±2, 833±2, 814±2, 800±2, 789±2, 736±2, 703±2, and 694±2 cm⁻¹.

In one embodiment, the crystalline Form J is characterized by a differential scanning calorimetry (DSC) thermogram having an onset temperature at about 266 °C.

In one embodiment, the crystalline form has at least 90 wt% that is in Form J. In one embodiment, the crystalline form consists essentially of Form J. In one embodiment, the crystalline Form J is substantially pure.

In another embodiment, the crystalline form of Compound (I) consists essentially of Form J. The crystalline form of this embodiment may comprise at least 90 wt. %, preferably at least 95 wt. %, and more preferably at least 99 wt. %, based on the weight of the crystalline Form J of Compound (I).

In yet another embodiment, a pharmaceutical composition is provided comprising Form J of Compound (I); and at least one pharmaceutically-acceptable carrier and/or diluent.

In still another embodiment, a pharmaceutical composition comprises substantially pure Form J of Compound (I); and at least one pharmaceutically-acceptable carrier and/or diluent.

In still an even further embodiment, a therapeutically effective amount of Form J of Compound (I) is combined with at least one pharmaceutically acceptable carrier and/or diluent to provide at least one pharmaceutical composition.

In one embodiment, the crystalline form is selected from a group consisting of Form A, Form B, Form C, Form D, Form E, Form F, Form G, Form H, Form I, and Form J. In one embodiment, the crystalline form is selected from a group consisting of Form B, Form C, Form D, Form E, Form F, Form G, Form H, Form I, and Form J. In one embodiment, the crystalline form is selected from a group consisting of Form A, Form F and Form J. In one embodiment, the crystalline form is selected from a group consisting of Form F and Form J.

Compound (I) may be prepared in accordance with Example 1. Crystalline forms may be prepared by a variety of methods, including for example, crystallization or recrystallization from a suitable solvent, sublimation, growth from a melt, solid state transformation from another phase, crystallization from a supercritical fluid, and jet spraying. Techniques for crystallization or recrystallization of crystalline forms from a solvent mixture include, for example, evaporation of the solvent, decreasing the temperature of the solvent mixture, crystal seeding a supersaturated solvent mixture of the molecule and/or salt, freeze drying the solvent mixture, and addition of antisolvents (countersolvents) to the solvent mixture. High throughput crystallization techniques may be employed to prepare crystalline forms including polymorphs.

Crystals of drugs, methods of preparation, and characterization of drug crystals are discussed in Solid-State Chemistry of Drugs, S.R. Byrn, R.R. Pfeiffer, and J.G. Stowell, 2nd Edition, SSCI, West Lafayette, Indiana (1999).

For crystallization techniques that employ solvent, the choice of solvent or solvents is typically dependent upon one or more factors, such as solubility of the compound, crystallization technique, and vapor pressure of the solvent. Combinations of solvents may be employed, for example, the compound may be solubilized into a first solvent to afford a solution, followed by the addition of an antisolvent to decrease the solubility of the compound in the solution and to afford the formation of crystals. An antisolvent is a solvent in which the compound has low solubility.

In one method to prepare crystals, a compound is suspended and/or stirred in a suitable solvent to afford a slurry, which may be heated to promote dissolution. The term "slurry", as used herein, means a saturated solution of the compound, which may also contain an additional amount of the compound to afford a heterogeneous mixture of the compound and a solvent at a given temperature.

Seed crystals may be added to any crystallization mixture to promote crystallization. Seeding may be employed to control growth of a particular polymorph or to control the particle size distribution of the crystalline product. Accordingly, calculation of the amount of seeds needed depends on the size of the seed available and the desired size of an average product particle as described, for example, in "Programmed Cooling of Batch Crystallizers," J.W. Mullin and J. Nyvlt, Chemical Engineering Science, 1971,26, 369-377. In general, seeds of small size are needed to control effectively the growth of crystals in the batch. Seed of small size may be generated by sieving, milling, or micronizing of large crystals, or by micro-crystallization of solutions. Care should be taken that milling or micronizing of crystals does not result in any change in crystallinity form the desired crystal form (i.e., change to amorphous or to another crystalline form).

A cooled crystallization mixture may be filtered under vacuum, and the isolated solids may be washed with a suitable solvent, such as cold recrystallization solvent, and dried under a nitrogen purge to afford the desired crystalline form. The isolated solids may be analyzed by a suitable spectroscopic or analytical technique, such as solid state nuclear magnetic resonance, differential scanning calorimetry, powder x-ray diffraction, or the like, to assure formation of the preferred crystalline form of the product. The resulting crystalline form is typically produced in an amount of greater than about 70 weight % isolated yield, preferably greater than 90 weight % isolated yield, based on the weight of the compound originally employed in the crystallization procedure. The product may be comilled or passed through a mesh screen to delump the product, if necessary.

Crystalline forms may be prepared directly from the reaction medium of the final process for preparing Compound (I). This may be achieved, for example, by employing in the final process step a solvent or a mixture of solvents from which Compound (I) may be crystallized. Alternatively, crystalline forms may be obtained by distillation or solvent addition techniques. Suitable solvents for this purpose include, for example, the aforementioned nonpolar solvents and polar solvents, including protic polar solvents such as alcohols, and aprotic polar solvents such as ketones.

The presence of more than one crystalline form in a sample may be determined by techniques such as powder x-ray diffraction (PXRD) or solid-state nuclear magnetic resonance spectroscopy. For example, the presence of extra peaks in the comparison of an experimentally measured PXRD pattern with a simulated PXRD pattern may indicate more than one crystalline form in the sample. The simulated PXRD may be calculated from single crystal x-ray data. see Smith, D.K., "A FORTRAN Program for Calculating X-Ray Powder Diffraction Patterns," Lawrence Radiation Laboratory, Livermore, California, UCRL-7196 (April 1963).

The forms of Compound (I) according to the invention may be characterized using various techniques, the operation of which are well known to those of ordinary skill in the art. The forms may be characterized and distinguished using single crystal x-ray diffraction, which is based on unit cell measurements of a single crystal at a fixed analytical temperature. A detailed description of unit cells is provided in Stout & Jensen, X-Ray Structure Determination: A Practical Guide, Macmillan Co., New York (1968), Chapter 3, which is herein incorporated by reference. Alternatively, the unique arrangement of atoms in spatial relation within the crystalline lattice may be characterized according to the observed fractional atomic coordinates. Another means of characterizing the crystalline structure is by powder x-ray diffraction analysis in which the diffraction profile is compared to a simulated profile representing pure powder material, both run at the same analytical temperature, and measurements for the subject form characterized as a series of 2θ values (usually four or more).

Other means of characterizing the form may be used, such as solid-state nuclear magnetic resonance (ssNMR), differential scanning calorimetry, thermography and gross examination of the crystalline morphology. These parameters may also be used in combination to characterize the subject form.

### UTILITY

The crystalline forms of Compound (I) and pharmaceutical compositions thereof may be useful in inhibiting Factor XIa. Accordingly, the present invention provides methods for the treatment and/or prevention of thromboembolic disorders in mammals (*i.e.,* factor XIa-associated disorders).

The crystalline forms of Compound (I) and pharmaceutical compositions thereof, in any of the methods herein, may be used to prevent thrombotic events in diverse patient populations including patients with renal and/or hepatic diseases. The crystalline forms of Compound (I) and pharmaceutical compositions thereof may be useful for treating a patient who is suffering from a disorder, including and may not be limited to acute coronary syndrome, chronic coronary syndrome, deep vein thrombosis, unstable angina, myocardial infarction, atrial fibrillation, ischemic stroke, peripheral occlusive arterial disease, transient ischemic attack, secondary stroke prevention, or pulmonary embolism. The crystalline forms of Compound (I) and pharmaceutical compositions thereof may be useful for use in methods for the treatment and / or prophylaxis of a patient who suffers from unstable angina, an acute coronary syndrome, atrial fibrillation, first myocardial infarction, recurrent myocardial infarction, ischemic sudden death, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from medical implants, devices, or procedures in which blood is exposed to an artificial surface that promotes thrombosis. The crystalline forms of Compound (I) and pharmaceutical compositions thereof may be useful for use in methods for the treatment and / or prophylaxis of a patient who suffers from acute coronary syndrome, deep vein thrombosis, chronic coronary syndrome, or secondary stroke prevention.

The crystalline forms of Compound (I) and pharmaceutical compositions thereof may be useful for prophylaxis of a patient of venous thromboembolism in acutely ill medical patients at risk for thromboembolic complication not at high risk of bleeding, or deep vein thrombosis which may lead to pulmonary embolism in patients undergoing knee or hip replacement surgery. The crystalline forms of Compound (I) and pharmaceutical compositions thereof may be useful in application to reduce the risk of major cardiovascular events (cardiovascular death, myocardial infarction and stroke) in patients with chronic coronary artery disease or peripheral artery disease. The crystalline forms of Compound (I) and pharmaceutical compositions thereof may be useful in application to reduce the risk of recurrence of deep vein thrombosis and/or pulmonary embolism (PE) or stroke in patients with nonvalvular atrial fibrillation.

The crystalline forms of Compound (I) described herein may be formulated into pharmaceutical compositions and/or employed in therapeutic and/or prophylactic methods, wherein the crystalline compound (I) has a crystalline form selected from: Form A, Form B, Form C, Form D, Form E, Form F, Form G, Form H, Form I, Form J, or combinations thereof. These methods include, but are not limited to, the administration of the crystalline compound (I), alone or in combination with one or more other pharmaceutically active agents, including agents that may be useful in the treatment of the disorders mentioned herein, wherein the crystalline compound (I) has a crystalline form selected from: Form A, Form B, Form C, Form D, Form E, Form F, Form G, Form H, Form I, Form J, or combinations thereof.

"Therapeutically effective amount" is intended to include an amount of the crystalline forms of Compound (I) that is effective when administered alone or in combination to inhibit factor XIa. If Compound (I) is used in combination with another medication, the combination of compounds described herein may result in a synergistic combination. Synergy, as described for example by Chou et al., Adv. Enzyme Regul., 22:27-55 (1984), occurs when the effect of the compounds when administered in combination is greater than the additive effect of the compounds when administered alone as a single agent. In general, a synergistic effect is most clearly demonstrated at suboptimal concentrations of the compounds. Synergy can be in terms of lower cytotoxicity, increased antithrombotic effect, or some other beneficial effect of the combination compared with the individual components.

As used herein, "treating" or "treatment" cover the treatment of a disease-state in a mammal, particularly in a human, and include: (a) preventing the disease-state from occurring in a mammal, in particular, when such mammal is predisposed to the disease-state but has not yet been diagnosed as having it; (b) inhibiting the disease-state, *i.e.,* arresting it development; and/or (c) relieving the disease-state, *i.e.,* causing regression of the disease state.

As used herein, "prophylaxis" is the protective treatment of a disease state to reduce and/or minimize the risk and/or reduction in the risk of recurrence of a disease state by administering to a patient a therapeutically effective amount of at least one of the compounds of the present invention or a or a stereoisomer, a tautomer, a pharmaceutically acceptable salt, or a solvate thereof. Patients may be selected for prophylaxis therapy based on factors that are known to increase risk of suffering a clinical disease state compared to the general population. For prophylaxis treatment, conditions of the clinical disease state may or may not be presented yet. "Prophylaxis" treatment can be divided into (a) primary prophylaxis and (b) secondary prophylaxis. Primary prophylaxis is defined as treatment to reduce or minimize the risk of a disease state in a patient that has not yet presented with a clinical disease state, whereas secondary prophylaxis is defined as minimizing or reducing the risk of a recurrence or second occurrence of the same or similar clinical disease state.

As used herein, "prevention" covers the preventive treatment of a subclinical disease-state in a mammal, particularly in a human, aimed at reducing the probability of the occurrence of a clinical disease-state. Patients are selected for preventative therapy based on factors that are known to increase risk of suffering a clinical disease state compared to the general population.

As used herein, "risk reduction" covers therapies that lower the incidence of development of a clinical disease state. As such, primary and secondary prevention therapies are examples of risk reduction.

In general, a thromboembolic disorder is a circulatory disease caused by blood clots (*i.e.,* diseases involving fibrin formation, platelet activation, and/or platelet aggregation). The term "thromboembolic disorders" as used herein includes arterial cardiovascular thromboembolic disorders, venous cardiovascular thromboembolic disorders, and thromboembolic disorders in the chambers of the heart. The term "thromboembolic disorders" as used herein also includes specific disorders selected from, but not limited to, unstable angina or other acute coronary syndromes, atrial fibrillation, first or recurrent myocardial infarction, ischemic sudden death, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from (a) prosthetic valves or other implants, (b) indwelling catheters, (c) stents, (d) cardiopulmonary bypass, (e) hemodialysis, or (f) other procedures in which blood is exposed to an artificial surface that promotes thrombosis. It is noted that thrombosis includes occlusion (*e.g.,* after a bypass) and reocclusion (*e.g.,* during or after percutaneous transluminal coronary angioplasty). The thromboembolic disorders may result from conditions including but not limited to atherosclerosis, surgery or surgical complications, prolonged immobilization, arterial fibrillation, congenital thrombophilia, cancer, diabetes, effects of medications or hormones, and complications of pregnancy. The anticoagulant effect of compounds of the present invention is believed to be due to inhibition of factor XIa or thrombin.

The methods preferably comprise administering to a patient a pharmaceutically effective amount of the novel crystals of the present invention, preferably in combination with one or more pharmaceutically acceptable carriers and/or excipients. The relative proportions of active ingredient and carrier and/or excipient may be determined, for example, by the solubility and chemical nature of the materials, chosen route of administration and standard pharmaceutical practice.

The crystalline forms of Compound (I) may be administered to a patient in such oral dosage forms as tablets, capsules (each of which includes sustained release or timed release formulations), pills, powders, granules, elixirs, tinctures, suspensions, syrups, and emulsions. They may also be administered in intravenous (bolus or infusion), intraperitoneal, subcutaneous, or intramuscular form, all using dosage forms well known to those of ordinary skill in the pharmaceutical arts. They may be administered alone, but generally will be administered with a pharmaceutical carrier selected on the basis of the chosen route of administration and standard pharmaceutical practice.

The dosage regimen for the crystalline forms of Compound (I) will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. A physician or veterinarian can determine and prescribe the effective amount of the drug required to prevent, counter, or arrest the progress of the thromboembolic disorder. Obviously, several unit dosage forms may be administered at about the same time. The dosage of the crystalline form of Compound (I) that will be most suitable for prophylaxis or treatment may vary with the form of administration, the particular crystalline form of the compound chosen and the physiological characteristics of the particular patient under treatment. Broadly, small dosages may be used initially and, if necessary, increased by small increments until the desired effect under the circumstances is reached.

By way of general guidance, in the adult, suitable doses may range from about 0.001 to about 1000 mg/kg body weight, and all combinations and subcombinations of ranges and specific doses therein. Preferred doses may be from about 0.01 to about 100 mg/kg body weight per day by inhalation, preferably 0.1 to 70, more preferably 0.5 to 20 mgkKg body weight per day by oral administration, and from about 0.01 to about 50, preferably 0.01 to 10 mg/kg body weight per day by intravenous administration. In each particular case, the doses may be determined in accordance with the factors distinctive to the subject to be treated, such as age, weight, general state of health and other characteristics which can influence the efficacy of the medicinal product. The crystalline forms of Compound (I) may be administered in a single daily dose, or the total daily dosage may be administered in divided doses of two, three, or four times daily.

For oral administration in solid form such as a tablet or capsule, the crystalline forms of Compound (I) can be combined with a non-toxic, pharmaceutically acceptable inert carrier, such as lactose, starch, sucrose, glucose, methylcellulose, magnesium stearate, dicalcium phosphate, calcium sulfate, mannitol, sorbitol and the like.

Preferably, in addition to the active ingredient, solid dosage forms may contain a number of additional ingredients referred to herein as "excipients". These excipients include among others diluents, binders, lubricants, glidants and disintegrants. Coloring agents may also be incorporated. "Diluents", as used herein, are agents which impart bulk to the formulation to make a tablet a practical size for compression. Examples of diluents are lactose and cellulose. "Binders", as used herein, are agents used to impart cohesive qualities to the powered material to help ensure the tablet will remain intact after compression, as well as improving the free-flowing qualities of the powder. Examples of typical binders are lactose, starch and various sugars. "Lubricants", as used herein, have several functions including preventing the adhesion of the tablets to the compression equipment and improving the flow of the granulation prior to compression or encapsulation. Lubricants are in most cases hydrophobic materials. Excessive use of lubricants is undesired, however, as it may result in a formulation with reduced disintegration and/or delayed dissolution of the drug substance. "Glidants", as used herein, refer to substances which may improve the flow characteristics of the granulation material. Examples of glidants include talc and colloidal silicon dioxide. "Disintegrants", as used herein, are substances or a mixture of substances added to a formulation to facilitate the breakup or disintegration of the solid dosage form after administration. Materials that may serve as disintegrants include starches, clays, celluloses, algins, gums and cross-linked polymers. A group of disintegrants referred to as "super-disintegrants" generally are used at a low level in the solid dosage form, typically 1% to 10% by weight relative to the total weight of the dosage unit. Croscarmellose, crospovidone and sodium starch glycolate represent examples of a cross-linked cellulose, a cross-linked polymer and a cross-linked starch, respectively. Sodium starch glycolate swells seven- to twelve-fold in less than 30 seconds effectively disintegrating the granulations that contain it.

The disintegrant preferably used in the present invention is selected from the group comprising modified starches, croscarmellose sodium, carboxymethylcellulose calcium and crospovidone. A more preferred disintegrant in the present invention is a modified starch such as sodium starch glycolate.

Preferred carriers include capsules or compressed tablets which contain the solid pharmaceutical dosage forms described herein. Preferred capsule or compressed tablet forms generally comprise a therapeutically effective amount of the crystalline forms of Compound (I) and one or more disintegrants in an amount greater than about 10% by weight relative to the total weight of the contents of the capsule or the total weight of the tablet.

Preferred capsule formulations may contain the crystalline forms of Compound (I) in an amount from about 5 to about 1000 mg per capsule. Preferred compressed tablet formulations contain the crystalline forms of Compound (I) in an amount from about 5 mg to about 800 mg per tablet. More preferred formulations contain about 50 to about 200 mg per capsule or compressed tablet. Preferably, the capsule or compressed tablet pharmaceutical dosage form comprises a therapeutically effective amount of Forms A and B of Compound (I); a surfactant; a disintegrant; a binder; a lubricant; and optionally additional pharmaceutically acceptable excipients such as diluents, glidants and the like; wherein the disintegrant is selected from modified starches; croscarmellose sodium, carboxymethylcellulose calcium and crospovidone.

For oral administration in liquid form, the crystalline forms of Compound (I) can be combined with any oral, non-toxic pharmaceutically acceptable inert carrier such as ethanol, glycerol, water and the like. The liquid composition may contain a sweetening agent which to make the compositions more palatable. The sweetening agent can be selected from a sugar such as sucrose, mannitol, sorbitol, xylitol, lactose, etc. or a sugar substitute such as cyclamate, saccharin, aspartame, etc. If sugar substitutes are selected as the sweetening agent the amount employed in the compositions of the invention will be substantially less than if sugars are employed. Taking this into account, the amount of sweetening agent may range from about 0.1 to about 50% by weight, and all combinations and subcombinations of ranges and specific amounts therein. Preferred amounts range from about 0.5 to about 30% by weight.

The more preferred sweetening agents are the sugars and particularly sucrose. The particle size of the powdered sucrose used has been found to have a significant influence in the physical appearance of the finished composition and its ultimate acceptance for taste. The preferred particle size of the sucrose component when used is in the range of from 200 to less than 325 mesh US Standard Screen, and all combinations and subcombinations of ranges and specific particle sizes therein.

Sterile injectable solutions may be prepared by incorporating the crystalline forms of Compound (I) in the required amounts, in the appropriate solvent, with various of the other ingredients enumerated herein, as required, followed by filtered sterilization. Generally, dispersions may be prepared by incorporating the sterilized active ingredient into a sterile vehicle which contains the dispersion medium and any other required ingredients. In the case of sterile powders for the preparation of sterile injectable solutions, the preferred methods of preparation may include vacuum drying and the freeze drying technique which may yield a powder of the active ingredient, plus any additional desired ingredient from the previously sterile-filtered solution thereof.

All forms of the present invention may be used for the preparation of liquid formulations in which Compound (I) may be, for example, dissolved or suspended. When dissolved, Compound (I) loses its crystalline structure, and is therefore considered to be a solution of Compound (I). In addition, the crystalline forms of Compound (I) may be incorporated into solid formulations.

The liquid compositions may also contain other components routinely utilized in formulating pharmaceutical compositions. One example of such components is lecithin. Its use in compositions of the invention as an emulsifying agent in the range of from 0.05 to 1% by weight, and all combinations and subcombinations of ranges and specific amounts therein. More preferably, emulsifying agents may be employed in an amount of from about 0.1 to about 0.5% by weight. Other examples of components that may be used are antimicrobial preservatives, such as benzoic acid or parabens; suspending agents, such as colloidal silicon dioxide; antioxidants; topical oral anesthetics; flavoring agents; and colorants.

The selection of such optional components and their level of use in the compositions of the invention is within the level of skill in the art and will be even better appreciated from the working examples provided hereinafter.

The crystalline forms of Compound (I) may also be coupled with soluble polymers as targetable drug carriers. Such polymers can include polyvinylpyrrolidine pyran copolymer, polyhydroxypropylmethacrylamide-phenol, polyhydroxyethyl-aspartamidephenol or polyethylene oxide-polylysine substituted with palmitolyl residues. Furthermore, the crystalline Compound (I) may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and crosslinked or amphipathic block copolymers of hydrogels.

Gelatin capsules of the crystalline forms of Compound (I) may contain the crystalline Compound (I) and the liquid or solid compositions described herein. Gelatin capsules may also contain powdered carriers such as lactose, starch, cellulose derivatives, magnesium stearate, stearic acid and the like. Similar diluents can be used to make compressed tablets. Both tablets and capsules can be manufactured as sustained release products to provide for continuous release of medication over a period of hours. Tablets can be sugar coated or film coated to mask any unpleasant taste and to protect the tablet from the atmosphere or enteric coated for selective disintegration in the gastrointestinal track.

In general, water, a suitable oil, saline, aqueous dextrose (glucose), and related sugar solutions and glycols, such as propylene glycol or polyethylene glycols are suitable carriers for parenteral solutions. Solutions for parenteral solutions are prepared by dissolving the crystalline Compound (I) in the carrier and, if necessary, adding buffering substances. Anti-oxidizing agents such as sodium bisulfite, sodium sulfite, or ascorbic acid either alone or combined, are suitable stabilizing agents. Citric acid and its salts and sodium EDTA may also be employed. Parenteral solutions may also contain preservatives, such as benzalkonium chloride, methyl- or propyl-paraben and chlorobutanol.

Suitable pharmaceutical carriers are described in Remington's Pharmaceutical Sciences, Mack Publishing Co., the disclosures of which are hereby incorporated herein by reference, in their entireties. Useful pharmaceutical dosage-forms for administration of the compounds of this invention can be illustrated as follows:
The preferred crystalline form of Compound (I) may serve as component (a) of this invention and can independently be in any dosage form, such as those described above, and can also be administered in various combinations, as described above. In the following description component (b) is to be understood to represent one or more agents as described herein suitable for combination therapy.

Accordingly, components (a) and (b) of the present invention may be formulated together, in a single dosage unit (that is, combined together in one capsule, tablet, powder, or liquid, etc.) as a combination product. When component (a) and (b) are not formulated together in a single dosage unit, the component (a) may be administered at the same time as component (b) or in any order; for example component (a) of this invention may be administered first, followed by administration of component (b), or they may be administered in the reverse order. If component (b) contains more than one agent, these agents may be administered together or in any order. When not administered at the same time, preferably the administration of component (a) and (b) occurs less than about one hour apart. Preferably, the route of administration of component (a) and (b) is oral. Although it may be preferable that component (a) and component (b) both be administered by the same route (that is, for example, both orally) or dosage form, if desired, they may each be administered by different routes (that is, for example, one component of the combination product may be administered orally, and another component may be administered intravenously) or dosage forms.

Thus, the crystalline forms of Compound (I) may be used alone or in combination with other diagnostic, anticoagulant, antiplatelet, fibrinolytic, antithrombotic, and/or profibrinolytic agents. For example, adjunctive administration of Factor XIa inhibitors with standard heparin, low molecular weight heparin, direct thrombin inhibitors (i.e., hirudin), aspirin, fibrinogen receptor antagonists, streptokinase, urokinase and/or tissue plasminogen activator may result in improved antithrombotic or thrombolytic efficacy or efficiency. The crystals described herein may be administered to treat thrombotic complications in a variety of animals, such as primates, including humans, sheep, horses, cattle, pigs, dogs, rats and mice. Inhibition of Factor XIa may be useful not only in the anticoagulant therapy of individuals having thrombotic conditions, but also when inhibition of blood coagulation may be required, such as to prevent coagulation of stored whole blood and to prevent coagulation in other biological samples for testing or storage. Thus, any Factor XIa inhibitor, including the crystalline forms of Compound (I) as described herein, can be added to or contacted with any medium containing or suspected of containing Factor XIa and in which it may be desired to inhibit blood coagulation.

The crystalline forms of Compound (I) may be used in combination with any antihypertensive agent or cholesterol or lipid regulating agent, or concurrently in the treatment of restenosis, atherosclerosis or high blood pressure. Some examples of agents that may be useful in combination with a novel form of Compound (I) according to the present invention in the treatment of high blood pressure include, for example, compounds of the following classes: beta-blockers, ACE inhibitors, calcium channel antagonists and alpha-receptor antagonists. Some examples of agents that may be useful in combination with a compound according to the invention in the treatment of elevated cholesterol levels or disregulated lipid levels include compounds known to be HMGCoA reductase inhibitors, or compounds of the fibrate class.

In some embodiments, for any of the pharmaceutical composition described herein, the crystalline compound (I) has a crystalline form selected from: Form A, Form B, Form C, Form D, Form E, Form F, Form G, Form H, Form I, Form J, or combinations thereof. In some embodiments, for any of the pharmaceutical composition described herein, the crystalline compound (I) has a crystalline form selected from: Form A, Form F, Form J, or combinations thereof. In some embodiments, for any of the pharmaceutical composition described herein, the crystalline compound (I) has a crystalline form selected from: Form F, Form J, or combinations thereof. In some embodiments, for any of the pharmaceutical composition described herein, the crystalline compound (I) comprises crystalline Form A. In some embodiments, for any of the pharmaceutical composition described herein, the crystalline compound (I) comprises crystalline Form F. In some embodiments, for any of the pharmaceutical composition described herein, the crystalline compound (I) comprises crystalline Form J.

In some embodiments, for any of the method described herein, the crystalline compound (I) has a crystalline form selected from: Form A, Form B, Form C, Form D, Form E, Form F, Form G, Form H, Form I, Form J, or combinations thereof. In some embodiments, for any of the method described herein, the crystalline compound (I) has a crystalline form selected from: Form A, Form F, Form J, or combinations thereof. In some embodiments, for any of the method described herein, the crystalline compound (I) has a crystalline form selected from: Form F, Form J, or combinations thereof. In some embodiments, for any of the method described herein, the crystalline compound (I) comprises crystalline Form A. In some embodiments, for any of the method described herein, the crystalline compound (I) comprises crystalline Form F. In some embodiments, for any of the method described herein, the crystalline compound (I) comprises crystalline Form J.

Pharmaceutical kits which may be useful for the treatment of various disorders, and which comprise a therapeutically effective amount of a pharmaceutical composition comprising a novel form of Compound (I) in one or more sterile containers, are also within the ambit of the present invention. The kits may further comprise conventional pharmaceutical kit components which will be readily apparent to those skilled in the art, once armed with the present disclosure. Sterilization of the container may be carried out using conventional sterilization methodology well known to those skilled in the art.

The present invention is further described in the following examples. All of the examples are actual examples. These examples are not to be construed as limiting the scope of the appended claims.

### EXAMPLES

The following examples are provided to further aid in understanding the embodiments disclosed in the application, and presuppose an understanding of conventional methods well known to those persons having ordinary skill in the art to which the examples pertain. The particular materials and conditions described hereunder are intended to exemplify particular aspects of embodiments disclosed herein and should not be construed to limit the reasonable scope thereof.

The crystalline forms of (9*R*,13*S*)-13-{4-[5-chloro-2-(4-chloro-1*H*-1,2,3-triazol-1-yl)phenyl]-6-oxo-1,6-dihydropyrimidin-1-yl} -3-(difluoromethyl)-9-methyl-3,4,7,15-tetraazatricyclo[12.3.1.0^{2,6}]octadeca-1(18),2(6),4,14,16-pentaen-8-one were characterized by various analytical techniques, including powder X-ray diffraction (PXRD), differential scanning calorimetry (DSC), dynamic vapor sorption (DVS), and thermographic analysis (TGA) using the procedures described below.

### General Procedures

### Primary Polymorphism Screen

A primary polymorphism screen was carried out in different solvent systems and utilized temperature cycling, crash cooling, evaporation and anti-solvent addition. The solvent systems were selected based on the results of the approximate solubility screen to include solvent system where new PXRD patterns were observed, solubility was observed to include process relevant solvents. Where new PXRD patterns were identified, the solids were analyzed by TG/DTA, DSC, FT-IR and ¹H NMR (where material amounts allowed).

### Saturated Solution Experiments

### (i) Crash Cooling

Saturated mother liquor solutions were placed in a refrigerator at 4°C for *ca.* 5 days. Any solids recovered were analyzed by PXRD. Where no solids or an insufficient amount of solid for analysis was evident, the solutions were placed in a freezer at -18°C for 2 days. Any solids were isolated and analyzed by PXRD. PXRD analysis was repeated on drying at 40°C for *ca.* 16 h.

### (ii) Evaporation

Saturated solutions were allowed to evaporate at ambient temperature and pressure. The solids obtained were analyzed by PXRD. The solids were then dried at 40°C for *ca.* 16 h and re-analyzed by PXRD.

### (iii) Anti-Solvent Addition

Anti-solvent was added to the saturated mother liquor solutions at 55°C. Anti-solvent was added dropwise. The solids obtained were isolated via centrifuge filtration (0.22 µm nylon filter) immediately after the addition of anti-solvent was complete. The isolated solids were analyzed by PXRD then re-analyzed after drying at 40°C for *ca.* 16 h.

### Competitive Slurry Experiments

Competitive slurry experiments are a solution-mediated process that provides a pathway for the less soluble (more stable) crystal to grow at the expense of the more soluble crystal form (Bernstein, J. Polymorphism in Molecular Crystals. Clarendon Press, Oxford, 2006; Brittain, H. G., Polymorphism in Pharmaceutical Solids. Marcel Dekker, Inc., New York, 1999). The choice of solvent affects the kinetics of polymorph conversion and not the thermodynamic relationship between polymorphic forms (Gu, C. H., Young, V. Jr., Grant, D. J., J. Pharm. Sci. 2001, 90 (11), 1878-1890).

Competitive slurry experiments can be carried out in order to locate the most stable form at the conditions investigated. Exemplary solvent systems that can be used for competitive slurry experiments are listed in Table 1.

**Table 1. Solvent Systems for Competitive Slurry Experiments**

| Solvent | ICH Class | Low Temperature (°C) | Upper Temperature (°C) |
|---|---|---|---|
| water | N/A | | 60 °C |
| heptane | 3 | room temperature | |
| cyclohexane | 2 | | |
| t-BME | 3 | | 50 °C |

### Powder X-Ray Diffraction (PXRD)

PXRD data were collected using a Bruker C2 (GADDS) General Area Detector Diffraction System (Bruker - Madison, WI). The radiation was Cu Kα (40 KV, 40mA). The sample-detector distance was 15 cm. Samples were placed in sealed glass capillaries with diameters of ≤ 1mm. The capillary was rotated during data collection. Transmission data were collected for approximately in the range of 2 to 32 degrees 2θ with a sample exposure time of at least 1000 s. The resulting two-dimensional diffraction arcs were integrated to create a traditional 1-dimensional PXRD pattern with a step size of 0.05 degrees. Alternatively, PXRD analysis was carried out on a PANalytical X'pert pro with PIXcel detector (128 channels), scanning the samples between 3 and 35° 2θ. The material was gently compressed and loaded onto a multi-well plate with Mylar polymer film to support the sample. The multi-well plate was then placed into the diffractometer and analyzed using Cu K radiation (α1 λ = 1.54060 Å; α2 = 1.54443 Å; β = 1.39225 Å; α1 : α2 ratio = 0.5) running in transmission mode (step size 0.0130° 2θ, step time 18.87s) using 40 kV / 40 mA generator settings.

### Differential Scanning Calorimetry (DSC)

TA INSTRUMENT^{®} models Q2000, Q1000, or 2920 were used to generate DSC data. The measurement was made using standard TA Instruments open pans. The measurement was made at a heating rate of 10 °C/min, in a nitrogen environment from room temperature to 300 °C, with a sample size of about 2-10 mg. The DSC plot was made with the endothermic peaks pointing down.

Alternatively, about 5 mg of material was weighed into an aluminum DSC pan and sealed non-hermetically with a pierced aluminum lid. The sample pan was then loaded into a Seiko DSC6200 (equipped with a cooler), cooled, and held at 20 °C. Once a stable heat-flow response was obtained, the sample and reference were heated to 260, 270, 285, 290, 295, 300, 310 or 330 °C at scan rate of 10 °C/min and the resulting heat flow response monitored. Nitrogen was used as the purge gas, at a flow rate of 50 cm³/min.

### Thermogravimetric Analysis (TGA)

TA INSTRUMENT^{®} models Q5000, Q500, or 2950 were used to generate TGA data. The measurement was made using standard TA Instruments Platinum pans. The measurement was made at a heating rate of 10 °C/min, in a nitrogen environment from room temperature to 300 °C, with a sample size about 10-30 mg.

### Dynamic Vapor Sorption (DVS)

About 20 mg of the crystalline form was transferred into a dynamic vapor sorption instrument from Surface Measurement Systems Ltd. (SMS). The weight change was recorded relative to the relative humidity (Target RH%) at 25 °C.

### Solid-State Nuclear Magnetic Resonance (SSNMR)

Carbon-13 cross polarization magic angle spinning (CPMAS) solid-state NMR experiments were conducted on a Bruker AV III instrument operating at a proton frequency of 400.13 MHz. Solid samples were spun at 13 KHz in a 4 mm ZrO₂ rotor. The contact time was 1.5 to 3 milliseconds and was ramped on the proton channel from 50 to 100% (A.E. Bennett et al, J. Chem. Phys., 1995, 103, 6951), (G. Metz, X. Wu and S.O. Smith, J. Magn. Reson. A., 1994, 110, 219-227). The relaxation delay was maintained at 5x ¹H T₁ of API (typically 30 seconds). Proton decoupling was applied using a TPPM sequence with a 2.8 microsecond pulse (90KHz nominal band width). The spectral sweep width was 300 ppm centered at 100 ppm. 2972 data points were acquired (giving a digital resolution of 20 Hz) and zero filled to 8192 prior to apodization with 20 Hz line broadening. Typically 1024 to 4096 free induction decays were co-added. The spectra were referenced indirectly to TMS using 3-methylglutaric acid (D. Barich, E. Gorman, M. Zell, and E. Munson, Solid State Nuc. Mag. Res., 2006, 30, 125-129). Approximately 70 mg of sample was used for each experiment. The temperature was set to 280K.

### Infrared (IR) Analysis

Micro Attenuated Total Reflectance (microATR) was used and the sample was analyzed using a suitable microATR accessory and the following measurement conditions:
apparatus: Thermo Nexus 670 FTIR spectrometer
number of scans: 32
resolution: 1 cm⁻¹
wavelength range: 4000 to 400 cm⁻¹
detector: DTGS with KBr windows
beamsplitter: Ge on KBr
micro ATR accessory: Harrick Split Pea with Si crystal.

### Example 1: Preparation of (9R,13S)-13-{4-[5-chloro-2-(4-chloro-1H-1,2,3-triazol-1-yl)phenyl]-6-oxo-1,6-dihydropyrimidin-1-yl}-3-(difluoromethyl)-9-methyl-3,4,7,15-tetraazatricyclo[12.3.1.0^{2,6}]octadeca-1(18),2(6),4,14,16-pentaen-8-one

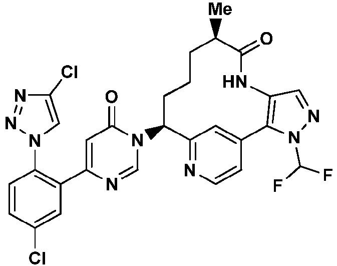

### 1A. Preparation of 1-(difluoromethyl)-4-nitro-1H-pyrazole

Cs₂CO₃ (14.41 g, 44.2 mmol) was suspended in a solution of 4-nitro-1*H*-pyrazole (5.00 g, 44.2 mmol) and DMF (40 mL). After heating to 120 °C for 5 min, solid sodium 2-chloro-2,2-difluoroacetate (13.48 g, 88 mmol) was added in 10 equal portions over 20 min. The reaction was complete after 10 min of additional heating. The mixture was added to a separatory funnel containing 100 mL water and extracted with Et₂O (2 x 50 mL). The combined organic layers were concentrated. Purification by normal-phase chromatography eluting with a gradient of hexanes/EtOAc yielded 1-(difluoromethyl)-4-nitro-1*H*-pyrazole (6.99 g, 42.9 mmol, 97% yield) as a clear, colorless oil. ¹H NMR (500MHz, CDCl₃) δ 8.58 (s, 1H), 8.22 (s, 1H), 7.39 - 7.05 (t, *J =* 60 Hz, 1H).

### 1B. Preparation of (S)-tert-butyl (1-(4-(1-(difluoromethyl)-4-nitro-1H-pyrazol-5-yl)pyridin-2-yl)but-3-en-1-yl)carbamate

To a N₂ flushed, 500 mL RBF was added (*S*)-*tert*-butyl (1-(4-chloropyridin-2-yl)but-3-en-1-yl)carbamate (10 g, 35.4 mmol), 1-(difluoromethyl)-4-nitro-1*H*-pyrazol (6.34 g, 38.9 mmol) and dioxane (100 mL). The solution was bubbled with N₂ for 5 min. Then Pd(OAc)₂ (0.40 g, 1.7 mmol), di(adamantan-1-yl)(butyl)phosphine (1.27 g, 3.5 mmol), K₂CO₃ (14.7 g, 106 mmol) and PvOH (1.08 g, 10.61 mmol) were added. The reaction mixture was bubbled with N₂ for 5 min then the reaction mixture was heated to 100 °C for 3 h. After this time, the solution was cooled to rt and water (200 mL) was added. The reaction mixture was then extracted with EtOAc (2 x 200 mL). The combined organic extracts were washed with water (200 mL), brine (200 mL), dried over Na₂SO₄, filtered and concentrated *in vacuo.* Purification by normal phase chromatography eluting with a gradient of hexanes/EtOAc afforded (*S*)-*tert*-butyl (1-(4-(1-(difluoromethyl)-4-nitro-1*H*-pyrazol-5-yl)pyridin-2-yl)but-3-en-1-yl)carbamate (12.91 g, 31.5 mmol, 89% yield) as a slightly yellow oil. MS(ESI) *m*/*z:* 410.4 [M+H]⁺. ¹H NMR (400MHz, CDCl₃) δ 8.80 (dd, *J*=5.1, 0.7 Hz, 1H), 8.36 (s, 1H), 7.34 (s, 1H), 7.31 (dd, *J*=5.1, 1.5 Hz, 1H), 7.27 - 6.91 (t, *J*=58 Hz, 1H), 5.79 - 5.63 (m, 1H), 5.16 - 5.03 (m, 2H), 4.92 (d, *J*=5.9 Hz, 1H), 2.67 (t, *J*=6.4 Hz, 2H), 1.46 (br. s., 9H).

### 1C. Preparation of (S)-tert-butyl (1-(4-(4-amino-1-(difluoromethyl)-1H-pyrazol-5-yl)pyridin-2-yl)but-3-en-1-yl)carbamate

To a 100 mL, 3-necked RBF was added a solution of (*S*)-*tert*-butyl (1-(4-(1-(difluoromethyl)-4-nitro-1*H*-pyrazol-5-yl)pyridin-2-yl)but-3-en-1-yl)carbamate (0.78 g, 1.90 mmol) in MeOH (12 mL) and a solution of NH₄Cl (1.02 g, 19 mmol) in water (3 mL). To the solution was added Fe (0.53 g, 9.49 mmol). The reaction mixture was heated to 65 °C for 3 h. Water (50 mL) was added. After cooling to rt, the mixture was filtered through a CELITE^{®} pad and rinsed with MeOH (200 mL). The filtrate was concentrated *in vacuo.* The residue was partitioned between EtOAC (100 mL) and water (100 mL). The organic phase was separated, washed with water (100 mL), brine (100 mL), dried over Na₂SO₄, filtered and concentrated *in vacuo.* Purification by normal phase chromatography eluting with a gradient of DCM/MeOH yielded (*S*)-*tert*-butyl (1-(4-(4-amino-1-(difluoromethyl)-1*H*-pyrazol-5-yl)pyridin-2-yl)but-3-en-1-yl)carbamate (0.585 g, 1.54 mmol, 81% yield) as an oil. MS(ESI) *m*/*z:* 380.1 [M+H]⁺. ¹H NMR (400MHz, CDCl₃) δ 8.70 (dd, *J*=5.0, 0.7 Hz, 1H), 7.43 (s, 1H), 7.36 (s, 1H), 7.32 (dd, *J*=5.1, 1.5 Hz, 1H), 7.28 - 6.97 (t, *J*=58 Hz, 1H), 5.80 - 5.66 (m, 1H), 5.65 - 5.53 (m, 1H), 5.13 - 5.03 (m, 2H), 4.87 (br. s., 1H), 3.22 (br. s., 2H), 2.65 (t, *J*=6.5 Hz, 2H), 1.52 - 1.37 (m, 9H).

### 1D. Preparation of tert-butyl ((S)-1-(4-(1-(difluoromethyl)-4-((R)-2-methylbut-3-enamido)-1H-pyrazol-5-yl)pyridin-2-yl)but-3-en-1-yl)carbamate

To a N₂ flushed, 3-necked, 250 mL RBF was added a solution of (*S*)-*tert*-butyl (1-(4-(4-amino-1-(difluoromethyl)-1*H*-pyrazol-5-yl)pyridin-2-yl)but-3-en-1-yl)carbamate (5 g, 13.18 mmol) and EtOAc (50 ml). The solution was cooled to -10 °C and (R)-2-methylbut-3-enoic acid (1.72 g, 17.13 mmol), pyridine (4.26 ml, 52.7 mmol). and T3P^{®} (23.54 ml, 39.5 mmol) were added. The cooling bath was removed and the solution was allowed to warm to rt and then stir over a period of 20 h. Water (30 mL) and EtOAc (30 mL) were added and the mixture was stirred for 30 min. The organic phase was separated and the aqueous layer was extracted with EtOAc (30 mL). The combined organic extracts were washed with brine (50 mL), dried over Na₂SO₄, filtered and concentrated *in vacuo.* Purification by normal phase chromatography eluting with a gradient of hexanes/EtOAc gave *tert*-butyl ((*S*)-1-(4-(1-(difluoromethyl)-4-((R)-2-methylbut-3-enamido)-1*H*-pyrazol-5-yl)pyridin-2-yl)but-3-en-1-yl)carbamate (5.69 g, 12.33 mmol, 94% yield). MS(ESI) *m*/*z:* 462.2 [M+H]⁺. ¹H NMR (400MHz, CDCl₃) δ 8.75 (dd, *J*=5.0, 0.6 Hz, 1H), 8.37 (s, 1H), 7.32 (t, *J*=59 Hz, 1H), 7.28 (br. s., 1H), 7.20 (s, 1H), 5.97 - 5.85 (m, 1H), 5.78 - 5.65 (m, 1H), 5.56 - 5.44 (m, 1H), 5.28 - 5.19 (m, 2H), 5.12 (d, *J*=2.0 Hz, 2H), 4.91 - 4.82 (m, 1H), 3.20 - 3.11 (m, 1H), 2.72 - 2.62 (m, 2H), 1.48 - 1.43 (s, 9H), 1.33 (d, *J*=6.8 Hz, 3H).

### 1E. Preparation of tert-butyl N-[(9R,10E,13S)-3-(difluoromethyl)-9-methyl-8-oxo-3,4,7,15-tetraazatricyclo[12.3.1.0^{2,6}]octadeca-1(18),2(6),4,10,14,16-hexaen-13-yl]carbamate

To a N₂ flushed, 2 L, 3-necked, RBF was added a solution of *tert*-butyl ((*S*)-1-(4-(1-(difluoromethyl)-4-((*R*)-2-methylbut-3-enamido)-1*H*-pyrazol-5-yl)pyridin-2-yl)but-3-en-1-yl)carbamate (3 g, 6.50 mmol) in EtOAc (1300 ml). The solution was sparged with argon for 15 min. Grubbs II (1.38 g, 1.63 mmol) was added in one portion. The reaction mixture was heated to reflux for 24 h. After cooling to rt, the solvent was removed and the residue was purified by normal phase chromatography eluting with a gradient of DCM/MeOH to yield *tert*-butyl *N*-[(9*R,*10*E,*13*S*)-3-(difluoromethyl)-9-methyl-8-oxo-3,4,7,15-tetraazatricyclo[12.3.1.0^{2,6}]octadeca-1(18),2(6),4,10,14,16-hexaen-13-yl]carbamate (2.13 g, 4.91 mmol, 76% yield) as a tan solid. MS(ESI) *m*/*z:* 434.4 [M+H]⁺. ¹H NMR (400MHz, CDCl₃) δ 8.71 (d, *J*=5.1 Hz, 1H), 7.78 (s, 1H), 7.44 - 7.40 (m, 1H), 7.36 (br. s., 1H), 7.27 (t, *J*=58 Hz, 1H), 6.87 (s, 1H), 6.49 - 6.39 (m, 1H), 5.78 (s, 1H), 4.80 (br. s., 2H), 3.18 - 3.08 (m, 1H), 3.08 - 2.98 (m, 1H), 2.06 - 1.93 (m, 1H), 1.51 (s, 9H), 1.19 (d, *J*=6.6 Hz, 3H).

### 1F. Preparation of tert-butyl N-[(9R,13S)-3-(difluoromethyl)-9-methyl-8-oxo-3,4,7,15-tetraazatricyclo[12.3.1.0^{2,6}]octadeca-1(18),2(6),4,14,16-pentaen-13-yl]carbamate

Pd/C (0.60 g, 0.570 mmol) was added to a 250 mL Parr hydrogenation flask containing a solution of *tert*-butyl *N-*[(9*R,*10*E,*13*S*)-3-(difluoromethyl)-9-methyl-8-oxo-3,4,7,15-tetraazatricyclo[12.3.1.0^{2,6}]octadeca-1(18),2(6),4,10,14,16-hexaen-13-yl]carbamate (2.46 g, 5.68 mmol) in EtOH (100 mL). The flask was purged with N₂ and pressurized to 55 psi of H₂ allowed to stir for 18 h. The reaction was filtered through CELITE^{®} and concentrated to yield *tert*-butyl *N*-[(9*R*,13*S*)-3-(difluoromethyl)-9-methyl-8-oxo-3,4,7,15-tetraazatricyclo[12.3.1.0^{2 6}]octadeca-1(18),2(6),4,14,16-pentaen-13-yl]carbamate (2.17 g, 88% yield) as a tan solid. MS(ESI) *m*/*z:* 436.3 [M+H]⁺. ¹H NMR (400MHz, DMSO-d₆) δ 9.32 (s, 1H), 8.71 (d, *J*=5.0 Hz, 1H), 7.96 (t, *J*=58 Hz, 1H), 7.43 (s, 1H), 7.32 (d, *J*=4.8 Hz, 1H), 7.22 (d, *J*=7.3 Hz, 1H), 4.66 (d, *J*=8.3 Hz, 1H), 2.62 (br. s., 1H), 1.88 (d, *J*=12.8 Hz, 1H), 1.77 - 1.59 (m, 2H), 1.42 - 1.28 (m, 9H), 1.15 (d, *J*=18.2 Hz, 2H), 0.83 (d, *J*=7.0 Hz, 3H).

### 1G. Preparation of (9R,13S)-13-amino-3-(difluoromethyl)-9-methyl-3,4,7,15-tetraazatricyclo[12.3.1.0^{2,6}]octadeca-1(18),2(6),4,14,16-pentaen-8-one

4 N HCl in dioxane (3.88 mL, 15.5 mmol) was added to a solution of *tert*-butyl *N-*[(9*R*,13*S*)-3-(difluoromethyl)-9-methyl-8-oxo-3,4,7,15-tetraazatricyclo[12.3.1.0^{2,6}] octadeca-1(18),2(6),4,14,16-pentaen-13-yl]carbamate (2.25 g, 5.2 mmol) in MeOH (10 mL). The reaction was allowed to stir at rt for 2 h. The reaction was cooled in an ice bath, and 7 N NH₃ in MeOH (13.3 mL, 93.0 mmol) was added. After 5 min, the reaction was diluted with CH₂Cl₂ (80 mL) and the solid that formed was filtered. The filtrate was concentrated to yield (9*R*,13*S*)-13-amino-3-(difluoromethyl)-9-methyl-3,4,7,15-tetraazatricyclo[12.3.1.0^{2,6}]octadeca-1(18),2(6),4,14,16-pentaen-8-one (1.3 g, 3.88 mmol, 75% yield). MS(ESI) *m*/*z:* 336.3 [M+H]⁺. ¹H NMR (400MHz, DMSO-d₆) δ 9.33 (s, 1H), 8.71 (d, *J*=5.0 Hz, 1H), 7.94 (t, *J*=58 Hz, 1H), 7.85 (s, 1H), 7.40 (s, 1H), 7.32 (d, *J*=5.0 Hz, 1H), 4.01 (dd, *J*=10.2, 5.1 Hz, 1H), 2.63 - 2.53 (m, 1H), 1.90 - 1.69 (m, 2H), 1.53 - 1.36 (m, 2H), 1.16 - 1.00 (m, 1H), 0.85 (d, *J*=7.0 Hz, 3H).

### 1H. Preparation of (9R,13S)-13-{4-[5-chloro-2-(4-chloro-1H-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one.

To a 30L glass reactor containing 6-(5-chloro-2-(4-chloro-1H-1,2,3-triazol-1-yl)phenyl)pyrimidin-4-ol (230 g, 0.73 mol), as prepared in Example 2, and powdered molecular sieves 4A (345 g) in DMAc (4.6 L) was added sequentially DBU (162 g, 1.06 mol), HATU (350 g, 0.92 mol). After 5 min of stirring at ambient temperature, (9R,13S)-13-amino-3-(difluoromethyl)-9-methyl-3,4,7,15-tetraazatricyclo[12.3.1.0^{2,6}]octadeca-1(18),2(6),4,14,16-pentaen-8-one (275 g, 0.82 mol) was added and the reaction mass was stirred for 2 h, after which it was filtered through a celite bed and diluted with 13.8 L of ethyl acetate. Water 6.9 L) was then added. The aqueous phase was back-extracted with 4.6 L ethyl acetate. The combined organic phases were further washed with water (6.9 L), with a saturated aqueous solution of NaCl (6.9L) and evaporated to dryness. The resulting mass was dissolved in DMAc (2.3 L). The DMAC rich phase was slowly added over 20 min to a reactor containing water (4.6 L, 0-10 °C), the reaction mass was stir for 30 min, before being filtered. The resulting wet solid was further slurried with water (2.3 L) and centrifuged. The wet solid was then dissolved in THF (9.2 L) and the organic layer washed twice with saturated NaCl solution (4.6 L x 2). The organic phase was treated with activated charcoal (69g) and filtered through a celite bed. The solvent was completely removed by evaporation. The resulting mass was finally re-dissolved in THF (1.85 L) at 55 °C and n-Heptane (6.9L) was added over 15 min. The reaction mass was cooled to 0-10 °C and stirred for 30 min, after which it was filtered. The wet cake was washed with n-Heptane (2.3L) and dried in an oven at 65 °C to afford 405 g (72% yield) of a crude (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one.

### Preparation of Form A

The crude above product was further processed to obtain Form A. Multiple batches of crude were combined totaling 1.5 Kg. The crude product (1.5 kg) was dissolved in a mixture of 17.17 L of ACN and 5.25 L of water. The solution was filtered and 2.2 L of ACN was added. The reaction mass was heated to 65 °C and water (13.5 L) was added over 30 min. Form A seed (15 g) was added and the reaction mass was stirred for an extra 30 min, and then water (9 L) was added over a period of 30 min. After two additional hours of stirring the reaction mass was cooled over 2h and further stirred at ambient temperature for 20h. The slurry was then filtered and the resulting solid was re-slurried in water (7.5 L) and filtered. The wet cake was dried in oven (65°C, under mild vacuum (450 mmHg) to afford 990 g, (84%) of (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one as crystalline Form A. MS(ESI) *m*/*z:* 626.2 [M+H]⁺. ¹H NMR (600MHz, DMSO-*d*6) δ 9.41 (s, N-H), 8.85 (s, 1H), 8.74 (d, *J*=5.0, 1H), 8.74 (s, 1H), 7.96 (t, *J*=57.7 Hz, 1 H), 7.92 (d, *J*=2.4Hz, 1H), 7.90 (s, 1H), 7.82 (dd, *J*=8.5, 2.4Hz, 1H), 7.75 (d, *J*=8.5Hz, 1H), 7.71 (br s, 1H), 7.43 (dd, *J*=5.0, 1.5Hz, 1H), 6.37 (s, 1H), 5.91 (bd, *J*=10.8Hz, 1H), 2.66 (m, 1H), 2.32 (m, 1H), 2.06 (m, 1H), 1.85 (m, 1H), 1.48 (m, 1H), 1.33 (m, 1H), 0.87 (d, *J*=6.8Hz, 3H), 0.35 (m, 1H).

### Preparation of Form B

Crystalline Form A was converted to crystalline Form B by slurrying 240 mg of Form A in about 10 mL MeOH for about 19 hours at room temperature. The slurry was then filtered by Buchner filtration and the solids dried at 40 °C under vacuum for about 19 h to give Form B.

### Preparation of Form C

Amorphous (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one was temperature cycled from room temperature to 4 °C or -18 °C starting from a solution of the compound in diethyl ether and with addition of anti-solvent dimethylformamide to provide crystalline Form C.

Temperature Cycling: Approximately 572 mg of amorphous (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one (prepared by freeze drying in 1,4-dioxane) was weighed into a 20 mL vial. Diethyl ether (20 mL) was added. The experiment was temperature cycled between 5°C and 40°C with 3 h holds at each temperature and 0.2°C/min heating/cooling rates with stirring. After *ca.* 24 h the experiment was sub-sampled. *Ca. 1* mL was isolated via 0.22 µm nylon centrifuge filter at ambient temperature and analyzed by PXRD. The remaining slurry was then isolated by centrifuge filtration and the solids were dried for *ca.* 24 h at ambient temperature under vacuum.

Form C was anhydrous and non-hygroscopic, with no change in form on VT-PXRD analysis.

### Preparation of Form D

Amorphous (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one was temperature cycled from room temperature to 4 °C or -18 °C starting from a solution of the compound in 1,4-dioxane to provide crystalline Form D. Approximately 591 mg of (9R,13S)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one (prepared by freeze drying in 1,4-dioxane) was weighed into a 20 mL vial. 1.6 mL of 1,4-dioxane was added. The experiment was temperature cycled between 5°C and 40°C with 3 h holds at each temperature, and 0.2°C/min heating/cooling rates with stirring. After *ca.* 24 h, an additional 1 mL of 1,4-dioxane was added, as the experiment was observed to have become a thick slurry. After *ca.* 4 days temperature cycling in total, the experiment was sub-sampled. *Ca.* 1 mL was isolated via 0.22 µm nylon centrifuge filter at ambient temperature and analyzed by PXRD. The remaining slurry was isolated via centrifuge filtration and transferred to a 20 mL vial and dried for *ca.* 17 h at ambient temperature under vacuum.

Form D was identified as an anhydrous form (containing less than 0.1 eq. 1,4-dioxane).

### Preparation of Form E

Amorphous (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one (approximately 500 mg) was dissolved in acetonitrile (8 mL). Some precipitation was observed; therefore, a further 10 mL of acetonitrile was added. The resulting suspension was syringe filtered using 0.45 µm PTFE filter and divided between 2 x 20 mL vials and allowed to evaporate at ambient temperature. The evaporated material was analyzed by PXRD and TG/DTA as crystalline Form E.

Form E was identified as anhydrous (containing less than 0.1 eq. 1,4-dioxane), non-hygroscopic form.

### Preparation of Form F

500 mg of amorphous (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one (prepared by freeze drying in 1,4-dioxane) was weighed into a 20 mL vial. Pyridine (1.7 mL) was added. The experiment was temperature cycled between 5°C and 40°C with 3 h holds at each temperature and 0.2°C/min heating/cooling rates with stirring. After *ca.* 3 days, the experiment was sub-sampled. *Ca.* 1 mL was isolated via 0.22 µm nylon centrifuge filter. The entire slurry was then isolated via centrifuge filtration. The solids were then transferred into a 20 mL vial. The solids were dried for *ca.* 5 h at ambient temperature. To the solids, 10 mL of ethanol was added and the mixture was slurried at 60°C with subsampling via centrifuge filtration and PXRD analysis after 5, 6 and 7 days to give Form F.

Alternatively, amorphous compound was temperature cycled from room temperature to 4 °C or -18 °C starting from a solution of the compound in methyl isobutyl ketone to afford solids, and then slurrying the resulting solids with heptane for 5 days led to formation of crystalline Form F. 574 mg of amorphous (9*R*,13*S*)-13-{4-[5-chloro-2-(4-chloro-1H-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one (prepared by freeze drying in 1,4-dioxane) was weighed into a 20 mL vial. 1.5 mL of MIBK was added. The preparation was temperature cycled between 5°C and 40°C with 3 h holds at each temperature and 0.2°C/min heating/cooling rates with stirring. After *ca. 3* days the experiment was sub-sampled. *Ca.* 1 mL was isolated via 0.22 µm nylon centrifuge filter at ambient temperature. The entire slurry was then isolated via centrifuge filtration. The solids were dried for *ca.* 17 h at ambient temperature. To the solids, 12.3 mL of heptane was added. The experiment was slurried at 60°C for *ca.* 3 days. The preparation was then sub-sampled. *Ca.* 1 mL was isolated via 0.22 µm nylon centrifuge filtration. The entire slurry was then isolated via centrifuge filtration and transferred to a 20 mL vial and dried at ambient under vacuum for *ca.* 4 days, to give Form F.

Form F was identified as an anhydrous (containing less than 0.1 eq. 1,4-dioxane), non-hygroscopic form.

### Preparation of Form G

Crystalline Form G was prepared by crystallization of (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one solution in acetonitrile and water (70/30 w/w) cosolvent system.

### Preparation of Form H

Crystalline Form H was prepared by crystallization of (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one solution in methyl acetate (10 volume parts) and stored at 5 °C for 3 days.

### Preparation of Form I

Crystalline Form I was prepared by storing Form H at 60 °C in vacuum for 3 weeks.

### Preparation of Form J

To a 400 mL reactor, 9.59 g acetone solvate of (9*R*,13*S*)-13-{4-[5-chloro-2-(4-chloro-1*H*-1,2,3-triazol-1-yl)phenyl]-6-oxo-1,6-dihydropyrimidin-1-yl}-3-(difluoromethyl)-9-methyl-3,4,7,15-tetraazatricyclo [12.3.1.0^{2,6}]octadeca-1(18),2(6),4,14,16-pentaen-8-one, 60.35 g acetonitrile and 9.13 g water were added to form a mixture. The mixture was stirred at 300 rpm and heated to 60 °C in 10 minutes. The mixture was stirred at 60 °C for 6 hrs. Then the mixture was allowed to cool to room temperature and the solid material was isolated by vacuum filtration.

### Example 2: Preparation of Intermediate 6-[5-chloro-2-(4-chloro-1H-1,2,3-triazol-1-yl)phenyl] pyrimidin-4-ol

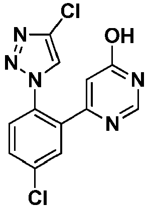

### 2A. Preparation of 4-chloro-2-(tetramethyl-1,3,2-dioxaborolan-2-yl)aniline

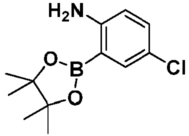

In a 20 mL microwave vial was added 2-bromo-4-chloroaniline (3 g, 14.53 mmol), 4,4,5,5-tetramethyl-2-(tetramethyl-1,3,2-dioxaborolan-2-yl)-1,3,2-dioxaborolane (5.53 g, 21.80 mmol), KOAc (3.66 g, 37.3 mmol), Pd(dppf)Cl₂-CH₂Cl₂ adduct (0.32 g, 0.44 mmol) and DMSO (9 mL). The resulting suspension was purged with N₂, capped and heated at 80 °C for 22 h. The reaction was cooled to rt. Water was added to dissolve the salts, then the reaction was filtered. The remaining solid was suspended in DCM and the insoluble solid was filtered. The filtrate was concentrated and then purified by normal phase chromatography to give 4-chloro-2-(tetramethyl-1,3,2-dioxaborolan-2-yl)aniline (3.15 g, 86% yield) as a white solid. MS(ESI) *m*/*z:* 172.3 (M-C₆H₁₀+H)⁺. ¹H NMR (400MHz, CDCl₃) δ 7.54 (d, *J*=2.6 Hz, 1H), 7.13 (dd, *J*=8.8, 2.6 Hz, 1H), 6.52 (d, *J*=8.6 Hz, 1H), 4.72 (br. s., 2H), 1.34 (s, 12H).

### 2B. Preparation of 4-chloro-2-(6-methoxypyrimidin-4-yl)aniline

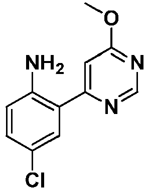

An RBF containing 4-chloro-6-methoxypyrimidine (3.13 g, 21.62 mmol), 4-chloro-2-(tetramethyl-1,3,2-dioxaborolan-2-yl)aniline (7.31 g, 21.62 mmol), Na₂CO₃ (2.29 g, 21.62 mmol), DME (86 ml), EtOH (10.81 ml) and water (10.81 ml) was equipped with a condenser. The mixture was purged with argon for several min, then Pd(dppf)Cl₂-CH₂Cl₂ adduct (1.77 g, 2.16 mmol) was added. The reaction was heated at 90 °C for 5 h. The reaction was cooled to rt, diluted with water and extracted with EtOAc. The organic layer was washed with brine, concentrated and purified by normal phase chromatography to give 4-chloro-2-(6-methoxypyrimidin-4-yl)aniline (2.86 g, 56.1% yield) as yellow solid. MS(ESI) *m*/*z:* 236.0 (M+H)⁺. ¹H NMR (500MHz, CDCl₃) δ 8.78 (d, *J*=1.1 Hz, 1H), 7.49 (d, *J*=2.5 Hz, 1H), 7.15 (dd, *J*=8.8, 2.5 Hz, 1H), 6.99 (d, *J*=1.1 Hz, 1H), 6.67 (d, *J*=8.8 Hz, 1H), 5.89 (br. s., 2H), 4.03 (s, 3H).

### 2C. Preparation of 4-{5-chloro-2-[4-(trimethylsilyl)-1H-1,2,3-triazol-1-yl]phenyl}-6-methoxypyrimidine

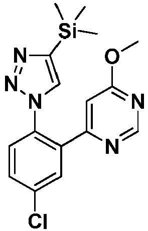

To a solution of 4-chloro-2-(6-methoxypyrimidin-4-yl)aniline (1.5 g, 6.36 mmol) in ACN (90 ml) at 0 °C was added 3-methylbutyl nitrite (1.28 ml, 9.55 mmol), followed by the dropwise addition of azidotrimethylsilane (1.26 ml, 9.55 mmol). Gas evolution was observed. After 10 min, the ice bath was removed, and the reaction was allowed to warm to rt. (Caution, aryl azides are potentially explosive.) After 1 h, ethynyltrimethylsilane (2.72 mL, 19.09 mmol) and Cu₂O (0.09 g, 0.64 mmol) were added and the reaction was stirred for an additional 1 h. The reaction was partitioned in EtOAc and sat NH₄Cl, and the layers were separated. The organic layer was washed with brine, dried over MgSO₄, filtered and concentrated. Purification by normal phase chromatography gave 4-{5-chloro-2-[4-(trimethylsilyl)-1H-1,2,3-triazol-1-yl]phenyl}-6-methoxypyrimidine (2.13 g, 5.92 mmol, 93% yield) as a yellow solid. MS(ESI) *m*/*z:* 360.3 (M+H)⁺. ¹H NMR (400MHz, CDCl₃) δ 8.71 (d, *J*=1.1 Hz, 1H), 7.82 (d, *J*=2.2 Hz, 1H), 7.61 - 7.56 (m, 1H), 7.54 - 7.48 (m, 2H), 6.20 (d, *J*=1.1 Hz, 1H), 3.92 (s, 3H), 0.32 - 0.28 (m, 9H).

### 2D. Preparation of 4-[5-chloro-2-(4-chloro-1H-1,2,3-triazol-1-yl)phenyl]-6-methoxypyrimidine

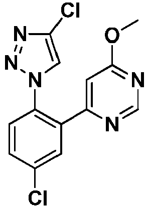

To a solution of 4-{5-chloro-2-[4-(trimethylsilyl)-1H-1,2,3-triazol-1-yl]phenyl}-6-methoxypyrimidine (1.56 g, 4.33 mmol) in ACN (28.9 ml) was added NCS (2.03 g, 15.17 mmol) and silica gel (6.51 g, 108 mmol). The reaction was stirred at 80 °C for 1 h. Then, the reaction was filtered to remove the silica gel and the collected silica gel was washed with EtOAc. The filtrate was washed with water (2x), brine and concentrated. Purification by normal phase chromatography gave 4-[5-chloro-2-(4-chloro-1H-1,2,3-triazol-1-yl)phenyl]-6-methoxy pyrimidine (0.90 g, 64.5% yield) as a yellow foam. MS(ESI) *m*/*z:* 322.3 (M+H)⁺. ¹H NMR (400MHz, CDCl₃) δ 8.70 (d, *J*=1.1 Hz, 1H), 7.75 (d, *J*=2.4 Hz, 1H), 7.66 - 7.55 (m, 2H), 7.50 (d, *J*=8.6 Hz, 1H), 6.52 (d, *J*=0.9 Hz, 1H), 3.98 (s, 3H).

### 2E. Preparation of 6-[5-chloro-2-(4-chloro-1H-1,2,3-triazol-1-yl)phenyl]pyrimidin-4-ol

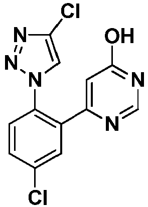

To a solution of 4-[5-chloro-2-(4-chloro-1H-1,2,3-triazol-1-yl)phenyl]-6-methoxypyrimidine (900 mg, 2.79 mmol) in AcOH (6 ml) was added 48% HBr in water (3 ml, 26.5 mmol). The mixture was stirred at 85 °C for 1 h. The reaction was concentrated to dryness and then partitioned between EtOAc and sat aqueous NaHCO₃.

The mixture was separated and the aqueous layer was extracted with EtOAc (2x). The organic layers were combined, concentrated, and then the residue was purified by normal phase chromatography to give a white solid. The solid was suspended in Et₂O, filtered and washed with Et₂O to give 6-[5-chloro-2-(4-chloro-1H-1,2,3-triazol-1-yl)phenyl] pyrimidin-4-ol (610 mg, 70.9% yield) as a white solid. MS(ESI) *m*/*z:* 308.3 (M+H)⁺. ¹H NMR (400^{M}Hz, CDCl₃) δ 7.96 (s, 1H), 7.74 - 7.67 (m, 2H), 7.62 (dd, *J*=8.5, 2.3 Hz, 1H), 7.47 (d, *J*=8.4 Hz, 1H), 6.44 (d, *J*=0.9 Hz, 1H).

### Example 3. Competitive Slurry Experiments

To a solvent (any one single solvent selected from water, heptane, cyclohexane and t-BME, 1 mL) is added 10 mg each of five polymorphic forms to form a slurry. The slurry is stirred at the appropriate temperature as set forth in Table 1 above for 48 hrs. 300 µL of saturated solution is isolated via centrifuge filter (0.22 µm nylon filter) after 48 hrs and 1 week. The resulting polymorphic solids are analyzed by PXRD.

### Example 4: PXRD, DSC, TGA, and DVS Analysis of Compound (I) in Crystalline Forms A through J

### Powder X-ray Diffraction (PXRD)

PXRD analysis of the crystalline Forms A through J of Compound (I) was undertaken using the method described above. The resulting diffraction patterns are shown in FIGS. 1, 5, 6A, 7A, 8A, 9A, 10A, 11A, 12, and 13A. Characteristic peaks of the crystalline forms are given below in Tables 2 through 11:

**Table 2**

| Form A Degrees 2θ ± 0.2 |
|---|
| 5.0 |
| 5.3 |
| 8.2 |
| 10.0 |
| 10.7 |
| 10.9 |
| 13.0 |
| 14.6 |
| 15.1 |
| 16.1 |
| 17.2 |
| 19.0 |
| 19.5 |
| 20.5 |
| 21.5 |
| 22.9 |
| 24.5 |

**Table 3**

| Form B Degrees 2θ ± 0.2 |
|---|
| 5.3 |
| 9.0 |
| 9.5 |
| 10.6 |
| 11.2 |
| 13.9 |
| 14.4 |
| 14.7 |
| 16.0 |
| 16.8 |
| 17.4 |
| 18.3 |
| 19.3 |
| 20.2 |
| 20.9 |
| 21.3 |
| 23.0 |
| 23.8 |
| 25.7 |
| 26.1 |

**Table 4**

| Form C Degrees 2θ ± 0.2 |
|---|
| 8.9 |
| 10.8 |
| 13.5 |
| 13.8 |
| 19.5 |
| 19.9 |
| 20.8 |
| 21.6 |
| 21.9 |
| 22.3 |
| 24.0 |
| 25.8 |
| 26.6 |

**Table 5**

| Form D Degrees 2θ ± 0.2 |
|---|
| 4.9 |
| 5.5 |
| 6.0 |
| 10.3 |
| 10.9 |
| 13.5 |
| 15.7 |
| 18.3 |
| 18.7 |
| 19.5 |
| 20.7 |
| 21.8 |
| 22.5 |
| 23.2 |
| 24.3 |
| 25.0 |
| 25.7 |
| 26.6 |
| 28.0 |
| 29.0 |

**Table 6**

| Form E Degrees 2θ ± 0.2 |
|---|
| 4.0 |
| 6.7 |
| 7.0 |
| 7.4 |
| 9.4 |
| 11.0 |
| 12.5 |
| 14.4 |
| 14.9 |
| 15.2 |
| 18.8 |
| 19.7 |
| 20.2 |
| 21.5 |
| 22.4 |
| 23.2 |
| 23.7 |

**Table 7**

| Form F Degrees 2θ ± 0.2 |
|---|
| 4.8 |
| 6.1 |
| 9.6 |
| 17.2 |
| 17.6 |
| 18.0 |
| 18.3 |
| 19.4 |
| 20.7 |
| 21.2 |
| 22.3 |
| 22.8 |
| 23.4 |
| 23.8 |
| 24.4 |
| 25.1 |
| 26.4 |
| 28.8 |

**Table 8**

| Form G Degrees 2θ ± 0.2 |
|---|
| 4.4 |
| 6.2 |
| 10.7 |
| 12.5 |
| 13.2 |
| 16.7 |
| 17.6 |
| 18.1 |
| 18.7 |
| 19.0 |
| 19.6 |
| 20.9 |
| 21.6 |
| 22.0 |
| 23.0 |
| 23.5 |
| 24.1 |
| 24.8 |
| 25.1 |
| 25.4 |

**Table 9**

| Form H Degrees 2θ ± 0.2 |
|---|
| 7.1 |
| 9.6 |
| 10.9 |
| 17.5 |
| 18.2 |
| 18.7 |
| 20.0 |
| 22.0 |
| 22.4 |
| 23.2 |
| 24.3 |
| 24.9 |
| 26.4 |
| 27.7 |
| 29.6 |

**Table 10**

| Form I Degrees 2θ ± 0.2 |
|---|
| 7.2 |
| 9.9 |
| 11.2 |
| 15.5 |
| 16.0 |
| 18.5 |
| 19.4 |
| 20.1 |
| 20.6 |
| 22.2 |
| 22.6 |
| 24.0 |
| 24.6 |
| 25.5 |
| 26.4 |

**Table 11**

| Form J Degrees 2θ ± 0.2 |
|---|
| 7.4 |
| 11.7 |
| 16.1 |
| 18.4 |
| 18.8 |
| 19.3 |
| 19.5 |
| 20.3 |
| 20.7 |
| 22.3 |
| 22.6 |
| 22.7 |
| 23.3 |
| 24.1 |
| 24.4 |
| 26.2 |
| 26.6 |
| 27.7 |
| 28.2 |
| 28.8 |

### Differential Scanning Calorimetry

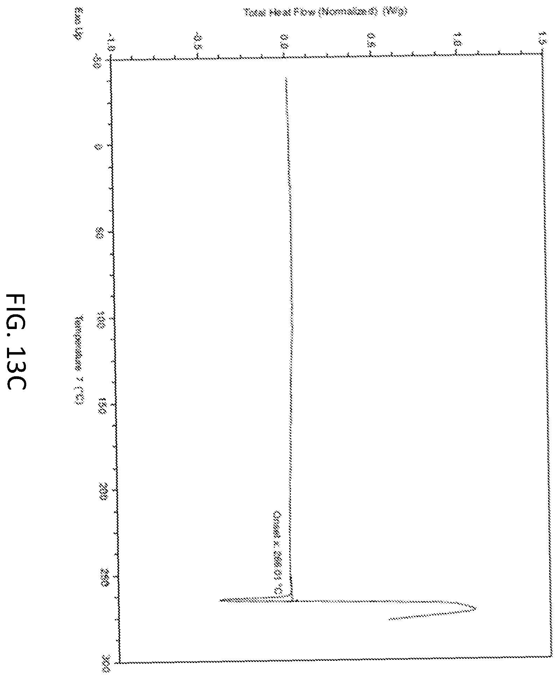

Crystalline Forms A, B, C, D, F, and J were analyzed by differential scanning calorimetry (DSC) according to the conditions described in the General Procedures. Illustrative DSC thermograms for Forms A, B, C, D, F, and J are shown in FIGS. 3, 5C, 6C, 7C, 9C, and 13C, respectively. For instance, the crystalline Form A in FIG. 3 showed an endothermic peak at about 261 °C. In another example, the crystalline Form J in FIG. 13C showed an onset of an endothermic peak at about 266 °C.

### Thermogravimetric Analysis

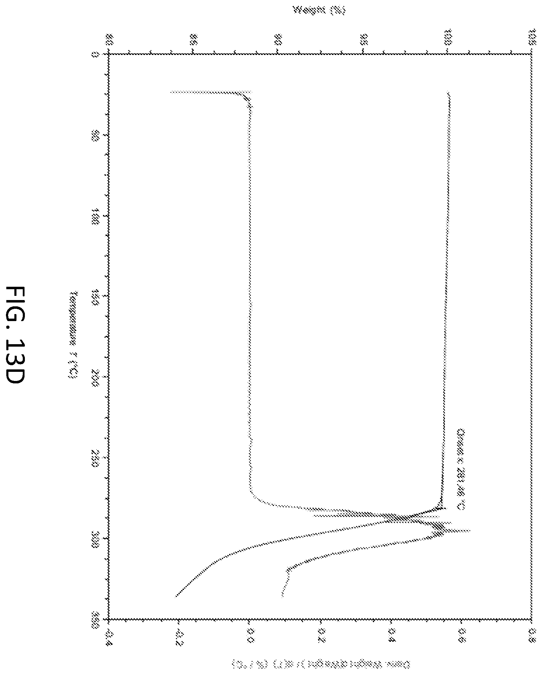

Crystalline Forms A and J were analyzed by thermogravimetric analysis (TGA) according to the conditions described in the General Procedures. Exemplary TGA thermograms for Forms A and J are shown in FIGS. 4 and 13D, respectively. For example, no significant loss of weight was observed for Form J in FIG. 13D below about 250 °C, with an onset of decomposition occurring at about 281 °C.

### Dynamic Vapor Sorption

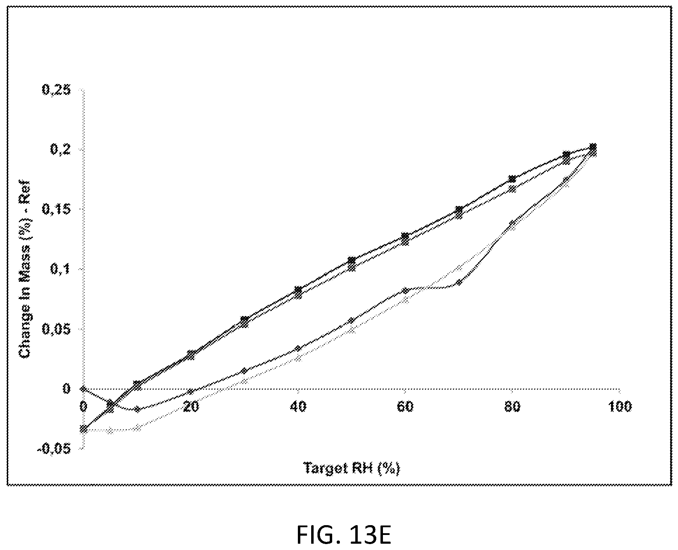

Crystalline Forms A and J were analyzed by dynamic vapor sorption (DVS) according to the conditions described in the General Procedures. The DVS isotherms for Forms A and J are shown in FIGS. 1C, 1D, and 13E. For each of Form A and Form J, the DVS analysis indicated the crystalline form was not hygroscopic. Each of Form A and Form J did not change after being subjected to DVS conditions, with each form exhibiting substantially the same PXRD and IR post-DVS analysis.

### Example 5: Characterization of the crystalline Form A of Compound (I) by solid-state Nuclear Magnetic Resonance (ssNMR) spectroscopy

The ¹³C solid-state CPMAS spectrum, as shown in FIG. 2 for Form A was collected in accordance with the procedures described in General Procedure. The ¹³C CPMAS chemical shift values of Form A are given below in Table 12:

**Table 12**

| **No.** | **ppm** |
|---|---|
| 1 | 181.4 |
| 2 | 160.2 |
| 3 | 158.7 |
| 4 | 121.0 |
| 5 | 114.3 |
| 6 | 58.2 |
| 7 | 26.8 |
| 8 | 25.7 |

### Example 6: Characterization of the crystalline forms of Compound (I) by infrared (IR) spectroscopy

IR spectra were obtained in accordance with the procedures described in the General Procedure.

Crystalline Form A gave an IR spectrum as shown in FIG. 1B. The main IR resonances for Form A are shown below:

| Major IR bands (cm⁻¹) | | |
|---|---|---|
| 1677vs | 1391m | 1029s |
| 1647s | 1325m | 1000m |
| 1607m | 1298m | 983m |
| 1593m | 1286m | 941m |
| 1586m | 1234m | 850m |
| 1532m | 1223m | 832s |
| 1481m | 1178m | 803m |
| 1445m | 1120m | 749m |
| 1429m | 1063m | 692m |

| | | |
|---|---|---|
| (vs = very strong, s = strong, m = medium intensity) | | |

Crystalline Form B gave an IR spectrum as shown in FIG. 5B. The main IR resonances for Form B are shown below:

| Major IR bands (cm⁻¹) | | |
|---|---|---|
| 1682vs | 1287m | 994m |
| 1649m | 1235m | 983m |
| 1607m | 1223m | 942m |
| 1592m | 1176m | 867m |
| 1532m | 1136m | 850m |
| 1481m | 1127m | 829s |
| 1444m | 1112m | 804m |
| 1428m | 1054m | 749m |
| 1389m | 1027s | 690m |

| | | |
|---|---|---|
| (vs = very strong, s = strong, m = medium intensity) | | |

Crystalline Form C gave an IR spectrum as shown in FIG. 6B. The main IR resonances for Form C are shown below:

| Major IR bands (cm⁻¹) | | |
|---|---|---|
| 1683vs | 1363m | 867m |
| 1536m | 1193m | 842m |
| 1489m | 1119m | 832s |
| 1457m | 1098m | 813m |
| 1446m | 1065m | 802m |
| 1388m | 1045m | 738m |
| 1373m | 1032m | |

| | | |
|---|---|---|
| (vs = very strong, s = strong, m = medium intensity) | | |

Crystalline Form D gave an IR spectrum as shown in FIG. 7B. The main IR resonances for Form D are shown below:

| Major IR bands (cm⁻¹) | | |
|---|---|---|
| 1678vs | 1389m | 990m |
| 1645s | 1303m | 983m |
| 1605m | 1294m | 869m |
| 1593m | 1285m | 850m |
| 1582m | 1234m | 832vs |
| 1533m | 1222m | 804m |
| 1516m | 1177m | 748m |
| 1480m | 1120m | 708m |
| 1451m | 1072s | 692m |
| 1444m | 1028s | |
| 1404m | 1000m | |

| | | |
|---|---|---|
| (vs = very strong, s = strong, m = medium intensity) | | |

Crystalline Form E gave an IR spectrum as shown in FIG. 8B. The main IR resonances for Form E are shown below:

| Major IR bands (cm⁻¹) | | |
|---|---|---|
| 1678vs | 1296m | 994m |
| 1610m | 1218m | 941m |
| 1590m | 1187m | 865m |
| 1533m | 1161m | 832s |
| 1486m | 1116m | 803m |
| 1461m | 1097m | 770m |
| 1442m | 1066s | 738m |
| 1373m | 1031s | 694m |
| 1366m | 1002m | 651m |

| | | |
|---|---|---|
| (vs = very strong, s = strong, m = medium intensity) | | |

Crystalline Form F gave an IR spectrum as shown in FIG. 9B. The main IR resonances for Form F are shown below:

| Major IR bands (cm⁻¹) | | |
|---|---|---|
| 1662vs | 1087m | 839m |
| 1604m | 1052m | 825m |
| 1591m | 1037m | 807m |
| 1542m | 1028m | 697m |
| 1487m | 876m | 688m |
| 1400m | 863m | |
| 1364m | 843m | |

| | | |
|---|---|---|
| (vs = very strong, s = strong, m = medium intensity) | | |

Crystalline Form G gave an IR spectrum as shown in FIG. 10B. The main IR resonances for Form G are as follows (vs = very strong, s = strong, m = medium intensity): 1680vs, 1672vs, 1609m, 1607m, 1535m, 1533m, 1488m, 1461m, 1441m, 1367m, 1118m, 1096m, 1066m, 1029m, 993m, 866m, 836s, 804m, 738m, 694m.

Crystalline Form H gave an IR spectrum as shown in FIG. 11B. The main IR resonances for Form H are as follows (vs = very strong, s = strong, m = medium intensity): 1732s, 1694vs, 1673s, 1534m, 1483m, 1461m, 1448m, 1433m, 1416m, 1377m, 1297m, 1283m, 1254m, 1223m, 1184m, 1113m, 1074m, 1041m, 1029s, 1001m, 873m, 850m, 844m, 831vs, 814m, 803m, 738m.

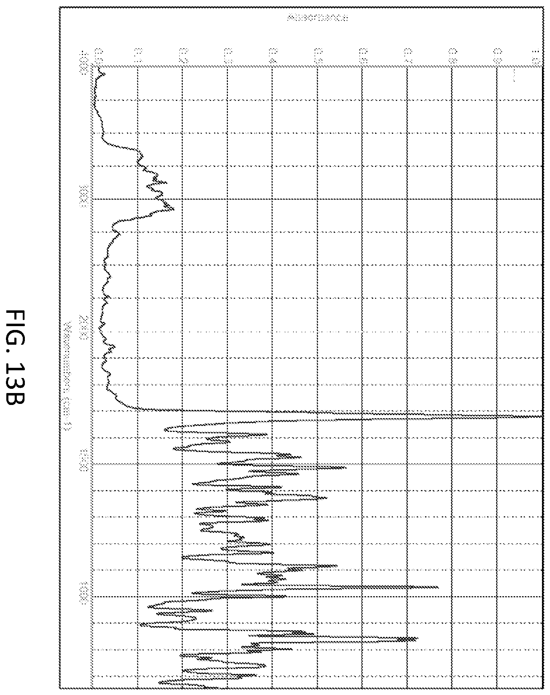

Crystalline Form J gave an IR spectrum as shown in FIG. 13B. The main IR resonances for Form J are as follows (vs = very strong, s = strong, m = medium intensity): 1678vs, 1610m, 1584m, 1536m, 1525m, 1486m, 1462m, 1412m, 1392m, 1371m, 1346m, 1296m, 1286m, 1234m, 1223m, 1196m, 1165m, 1114m, 1099m, 1078m, 1065m, 1052m, 1034s, 999m, 867m, 858m, 841s, 833s, 814m, 800m, 789m, 736m, 703m, 694m.

While the invention has been described in detail and with reference to specific embodiments thereof, it will be apparent to one skilled in the art that various changes and modifications can be made therein without departing from the spirit and scope thereof. The following clauses form part of the present disclosure.
1. A crystalline form of Compound (I): 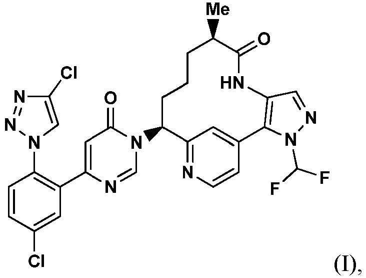 selected from Form A, Form B, Form C, Form D, Form E, Form F, Form G, Form H, Form I, and Form J.
2. The crystalline Form A according to clause 1 characterized by at least one of the following:
   a) a powder x-ray diffraction pattern comprising one or more 2θ values selected from: 5.0±0.2, 5.3±0.2, 8.2±0.2, 10.0±0.2, 10.7±0.2, 10.9±0.2, 13.0±0.2, 14.6±0.2, 15.1±0.2, 16.1±0.2, 17.2±0.2, 19.0±0.2, 19.5±0.2, 20.5±0.2, 21.5±0.2, 22.9±0.2, and 24.5±0.2;
   b) an observed powder x-ray diffraction pattern substantially as shown in FIG. 1A;
   c) an infrared (IR) spectrum having one or more peaks selected from: 1677±2, 1647±2, 1607±2, 1593±2, 1586±2, 1532±2, 1481±2, 1445±2, 1429±2, 1391±2, 1325±2, 1298±2, 1286±2, 1234±2, 1223±2, 1178±2, 1120±2, 1063±2, 1029±2, 1000±2, 983±2, 941±2, 850±2, 832±2, 803±2, 749±2, and 692±2 cm⁻¹;
   d) an infrared (IR) spectrum substantially as shown in FIG. 1B;
   e) a differential scanning calorimetry (DSC) thermogram having an endothermic peak at about 261 °C;
   f) a differential scanning calorimetry (DSC) thermogram substantially as shown in FIG. 3;
   g) a thermogravimetric analysis (TGA) thermogram substantially as shown in FIG. 4; and/or
   h) a dynamic vapor sorption (DVS) isotherm substantially as shown in FIG. 1C.
3. The crystalline Form B according to clause 1 characterized by at least one of the following:
   a) a powder x-ray diffraction pattern comprising one or more 2θ values selected from: 5.3±0.2, 9.0±0.2, 9.5±0.2, 10.6±0.2, 11.2±0.2, 13.9±0.2, 14.4±0.2, 14.7±0.2, 16.0±0.2, 16.8±0.2, 17.4±0.2, 18.3±0.2, 19.3±0.2, 20.2±0.2, 20.9±0.2, 21.3±0.2, 23.0±0.2, 23.8±0.2, 25.8±0.2, and 26.1±0.2;
   b) an observed powder x-ray diffraction pattern substantially as shown in FIG. 5A;
   c) an infrared (IR) spectrum having one or more peaks selected from: 1682±2, 1649±2, 1607±2, 1592±2, 1532±2, 1481±2, 1444±2, 1428±2, 1389±2, 1287±2, 1235±2, 1223±2, 1176±2, 1136±2, 1127±2, 1112±2, 1054±2, 1027±2, 994±2, 983±2, 942±2, 867±2, 850±2, 829±2, 804±2, 749±2, and 690±2 cm⁻¹;
   d) an infrared (IR) spectrum substantially as shown in FIG. 5B;
   e) a differential scanning calorimetry (DSC) thermogram having an endothermic peak at about 235 °C; and/or
   f) a differential scanning calorimetry (DSC) thermogram substantially as shown in FIG. 5C.
4. The crystalline Form C according to clause 1 characterized by at least one of the following:
   a) a powder x-ray diffraction pattern comprising one or more 2θ values selected from: 8.9±0.2, 10.8±0.2, 13.5±0.2, 13.8±0.2, 19.5±0.2, 19.9±0.2, 20.8±0.2, 21.6±0.2, 21.9±0.2, 22.3±0.2, 24.0±0.2, 25.8±0.2, and 26.6±0.2;
   b) an observed powder x-ray diffraction pattern substantially as shown in FIG. 6A;
   c) an infrared (IR) spectrum having one or more peaks selected from: 1683±2, 1536±2, 1489±2, 1457±2, 1446±2, 1388±2, 1373±2, 1363±2, 1193±2, 1119±2, 1098±2, 1065±2, 1045±2, 1032±2, 867±2, 842±2, 832±2, 813±2, 802±2, and 738±2 cm⁻¹;
   d) an infrared (IR) spectrum substantially as shown in FIG. 6B;
   e) a differential scanning calorimetry (DSC) thermogram having an endothermic peak at about 277 °C; and/or
   f) a differential scanning calorimetry (DSC) thermogram substantially as shown in FIG. 6C.
5. The crystalline Form D according to clause 1 characterized by at least one of the following:
   a) a powder x-ray diffraction pattern comprising one or more 2θ values selected from: 4.9±0.2, 5.5±0.2, 6.0±0.2, 10.3±0.2, 10.9±0.2, 13.5±0.2, 15.7±0.2, 18.3±0.2, 18.7±0.2, 19.5±0.2, 20.7±0.2, 21.8±0.2, 22.5±0.2, 23.2±0.2, 24.3±0.2, 25.0±0.2, 25.7±0.2, 26.6±0.2, 28.0±0.2, and 29.0±0.2;
   b) an observed powder x-ray diffraction pattern substantially as shown in FIG. 7A;
   c) an infrared (IR) spectrum having one or more peaks selected from: 1678±2, 1645±2, 1605±2, 1593±2, 1582±2, 1533±2, 1516±2, 1480±2, 1451±2, 1444±2, 1404±2, 1389±2, 1303±2, 1294±2, 1285±2, 1234±2, 1222±2, 1177±2, 1120±2, 1072±2, 1028±2, 1000±2, 990±2, 983±2, 869±2, 850±2, 832±2, 804±2, 748±2, 708±2, and 692±2 cm⁻¹;
   d) an infrared (IR) spectrum substantially as shown in FIG. 7B.
   e) a differential scanning calorimetry (DSC) thermogram having an endothermic peak at about 250 °C; and/or
   f) a differential scanning calorimetry (DSC) thermogram substantially as shown in FIG. 7C.
6. The crystalline Form E according to clause 1 characterized by at least one of the following:
   a) a powder x-ray diffraction pattern comprising one or more 2θ values selected from: 4.0±0.2, 6.7±0.2, 7.0±0.2, 7.4±0.2, 9.4±0.2, 11.0±0.2, 12.5±0.2, 14.4±0.2, 14.9±0.2, 15.2±0.2, 18.8±0.2, 19.7±0.2, 20.2±0.2, 21.5±0.2, 22.4±0.2, 23.2±0.2, and 23.7±0.2;
   b) an observed powder x-ray diffraction pattern substantially as shown in FIG. 8A;
   c) an infrared (IR) spectrum having one or more peaks selected from: 1678±2, 1610±2, 1590±2, 1533±2, 1486±2, 1461±2, 1442±2, 1373±2, 1366±2, 1296±2, 1218±2, 1187±2, 1161±2, 1116±2, 1097±2, 1066±2, 1031±2, 1002±2, 994±2, 941±2, 865±2, 832±2, 803±2, 770±2, 738±2, 694±2, and 651±2 cm⁻¹; and/or
   d) an infrared (IR) spectrum substantially as shown in FIG. 8B.
7. The crystalline Form F according to clause 1 characterized by at least one of the following:
   a) a powder x-ray diffraction pattern comprising one or more 2θ values selected from: 4.8±0.2, 6.1±0.2, 9.6±0.2, 12.2±0.2, 17.2±0.2, 17.6±0.2, 18.0±0.2, 18.3±0.2, 19.4±0.2, 20.7±0.2, 21.2±0.2, 22.3±0.2, 22.8±0.2, 23.4±0.2, 23.8±0.2, 24.4±0.2, 25.1±0.2, 26.4±0.2, and 28.8±0.2;
   b) an observed powder x-ray diffraction pattern substantially as shown in FIG. 9A;
   c) an infrared (IR) spectrum having one or more peaks selected from: 1662±2, 1604±2, 1591±2, 1542±2, 1487±2, 1400±2, 1364±2, 1087±2, 1052±2, 1037±2, 1028±2, 876±2, 863±2, 843±2, 839±2, 825±2, 807±2, 697±2, and 688±2 cm⁻¹;
   d) an infrared (IR) spectrum substantially as shown in FIG. 9B;
   e) a differential scanning calorimetry (DSC) thermogram having an endothermic peak at about 242 °C; and/or
   f) a differential scanning calorimetry (DSC) thermogram substantially as shown in FIG. 9C.
8. The crystalline Form G according to clause 1 characterized by at least one of the following:
   a) a powder x-ray diffraction pattern comprising one or more 2θ values selected from: 4.4±0.2, 6.2±0.2, 10.7±0.2, 12.5±0.2, 13.2±0.2, 16.7±0.2, 17.6±0.2, 18.1±0.2, 18.7±0.2, 19.0±0.2, 19.6±0.2, 20.9±0.2, 21.6±0.2, 22.0±0.2, 23.0±0.2, 23.5±0.2, 24.1±0.2, 24.8±0.2, 25.1±0.2, and 25.4±0.2;
   b) an observed powder x-ray diffraction pattern substantially as shown in FIG. 10A;
   c) an infrared (IR) spectrum having one or more peaks selected from: 1680±2, 1672±2, 1609±2, 1607±2, 1535±2, 1533±2, 1488±2, 1461±2, 1441±2, 1367±2, 1118±2, 1096±2, 1066±2, 1029±2, 993±2, 866±2, 836±2, 804±2, 738±2, and 694±2 cm⁻¹; and/or
   d) an infrared (IR) spectrum substantially as shown in FIG. 10B.
9. The crystalline Form H according to clause 1 characterized by at least one of the following:
   a) a powder x-ray diffraction pattern comprising one or more 2θ values selected from: 7.1±0.2, 9.6±0.2, 10.9±0.2, 17.5±0.2, 18.2±0.2, 18.7±0.2, 20.0±0.2, 22.0±0.2, 22.4±0.2, 23.2±0.2, 24.3±0.2, 24.9±0.2, 26.4±0.2, 27.7±0.2, and 29.6±0.2;
   b) an observed powder x-ray diffraction pattern substantially as shown in FIG. 11A;
   c) an infrared (IR) spectrum having one or more peaks selected from: 1732±2, 1694±2, 1673±2, 1534±2, 1483±2, 1461±2, 1448±2, 1433±2, 1416±2, 1377±2, 1297±2, 1283±2, 1254±2, 1223±2, 1184±2, 1113±2, 1074±2, 1041±2, 1029±2, 1001±2, 873±2, 850±2, 844±2, 831±2, 814±2, 803±2, and 738±2 cm⁻¹; and/or
   d) an infrared (IR) spectrum substantially as shown in FIG. 12B.
10. The crystalline Form I according to clause 1 characterized by at least one of the following:
   a) a powder x-ray diffraction pattern comprising one or more 2θ values selected from: 7.2±0.2, 9.9±0.2, 11.2±0.2, 15.5±0.2, 16.0±0.2, 18.5±0.2, 19.4±0.2, 20.1±0.2, 20.6±0.2, 22.2±0.2, 22.6±0.2, 24.0±0.2, 24.6±0.2, 25.5±0.2, and 26.4±0.2; and/or
   b) an observed powder x-ray diffraction pattern substantially as shown in FIG. 12;
11. The crystalline Form J according to clause 1 characterized by at least one of the following:
   a) a powder x-ray diffraction pattern comprising one or more 2θ values selected from: 7.4±0.2, 9.0±0.2, 11.3±0.2, 11.7±0.2, 12.5±0.2, 14.8±0.2, 15.1±0.2, 15.5±0.2, 16.1±0.2, 17.6±0.2, 18.4±0.2, 18.8±0.2, 19.3±0.2, 23.3±0.2, 24.4±0.2, 26.2±0.2, 26.6±0.2, 27.7±0.2, 28.2±0.2, and 28.8±0.2;
   b) an observed powder x-ray diffraction pattern substantially as shown in FIG. 13A;
   c) an infrared (IR) spectrum having one or more peaks selected from: 1678±2, 1610±2, 1584±2, 1536±2, 1525±2, 1486±2, 1462±2, 1412±2, 1392±2, 1371±2, 1346±2, 1296±2, 1286±2, 1234±2, 1223±2, 1196±2, 1165±2, 1114±2, 1099±2, 1078±2, 1065±2, 1052±2, 1034±2, 999±2, 867±2, 858±2, 841±2, 833±2, 814±2, 800±2, 789±2, 736±2, 703±2, and 694±2 cm⁻¹; and/or
   d) an infrared (IR) spectrum substantially as shown in FIG. 13B;
   e) a differential scanning calorimetry (DSC) thermogram having an onset temperature at about 266 °C;
   f) a differential scanning calorimetry (DSC) thermogram substantially as shown in FIG. 13C;
   g) a thermogravimetric analysis (TGA) thermogram substantially as shown in FIG. 13D; and/or
   h) a dynamic vapor sorption (DVS) isotherm substantially as shown in FIG. 13E.
12. A crystalline form of Compound (I): 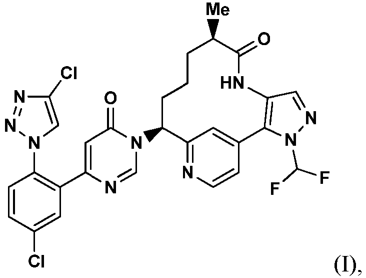 characterized by at least one of the following:
   a) a powder x-ray diffraction pattern comprising two or more 2θ values selected from: 4.8±0.2, 6.1±0.2, 9.6±0.2, 12.2±0.2, 17.2±0.2, 17.6±0.2, 18.0±0.2, 18.3±0.2, 19.4±0.2, 20.7±0.2, 21.2±0.2, 22.3±0.2, 22.8±0.2, 23.4±0.2, 23.8±0.2, 24.4±0.2, 25.1±0.2, 26.4±0.2, and 28.8±0.2;
   b) an observed powder x-ray diffraction pattern substantially as shown in FIG. 9A;
   c) an infrared (IR) spectrum having one or more peaks selected from: 1662±2, 1604±2, 1591±2, 1542±2, 1487±2, 1400±2, 1364±2, 1087±2, 1052±2, 1037±2, 1028±2, 876±2, 863±2, 843±2, 839±2, 825±2, 807±2, 697±2, and 688±2 cm⁻¹;
   d) an infrared (IR) spectrum substantially as shown in FIG. 9B;
   e) a differential scanning calorimetry (DSC) thermogram having an endothermic peak at about 242 °C; and/or
   f) a differential scanning calorimetry (DSC) thermogram substantially as shown in FIG. 9C.
13. A crystalline form of Compound (I): 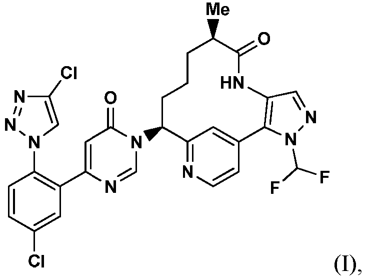 characterized by at least one of the following:
   a) a powder x-ray diffraction pattern comprising two or more 2θ values selected from: 7.4±0.2, 9.0±0.2, 11.3±0.2, 11.7±0.2, 12.5±0.2, 14.8±0.2, 15.1±0.2, 15.5±0.2, 16.1±0.2, 17.6±0.2, 18.4±0.2, 18.8±0.2, 19.3±0.2, 23.3±0.2, 24.4±0.2, 26.2±0.2, 26.6±0.2, 27.7±0.2, 28.2±0.2, and 28.8±0.2;
   b) an observed powder x-ray diffraction pattern substantially as shown in FIG. 13A;
   c) an infrared (IR) spectrum having one or more peaks selected from: 1678±2, 1610±2, 1584±2, 1536±2, 1525±2, 1486±2, 1462±2, 1412±2, 1392±2, 1371±2, 1346±2, 1296±2, 1286±2, 1234±2, 1223±2, 1196±2, 1165±2, 1114±2, 1099±2, 1078±2, 1065±2, 1052±2, 1034±2, 999±2, 867±2, 858±2, 841±2, 833±2, 814±2, 800±2, 789±2, 736±2, 703±2, and 694±2 cm⁻¹; and/or
   d) an infrared (IR) spectrum substantially as shown in FIG. 13B;
   e) a differential scanning calorimetry (DSC) thermogram having an onset temperature at about 266 °C;
   f) a differential scanning calorimetry (DSC) thermogram substantially as shown in FIG. 13C;
   g) a thermogravimetric analysis (TGA) thermogram substantially as shown in FIG. 13D; and/or
   h) a dynamic vapor sorption (DVS) isotherm substantially as shown in FIG. 13E.
14. A pharmaceutical composition comprising a crystalline form of Compound (I) of any one of clauses 1 to 13, and a pharmaceutically acceptable carrier or diluent.
15. A method of treating thrombosis in a mammal in need thereof, comprising administering to the mammal a therapeutically effective amount of a crystalline form of Compound (I) of any one of clauses 1 to 13.

## Claims

1. A crystalline Form A of Compound (I): 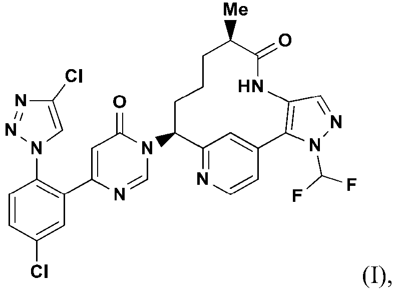 **characterized by** at least one of the following:
a) a powder x-ray diffraction pattern comprising one or more 2θ values selected from: 5.0±0.2, 5.3±0.2, 8.2±0.2, 10.0±0.2, 10.7±0.2, 10.9±0.2, 13.0±0.2, 14.6+0.2, 15.1+0.2, 16.1+0.2, 17.2+0.2, 19.0±0.2, 19.5±0.2, 20.5±0.2, 21.5±0.2, 22.9±0.2, and 24.5±0.2;
b) an observed powder x-ray diffraction pattern as shown in FIG. 1A;
c) an infrared (IR) spectrum having one or more peaks selected from: 1677±2, 1647±2, 1607±2, 1593±2, 1586±2, 1532±2, 1481±2, 1445±2, 1429±2, 1391±2, 132542, 1298±2, 1286±2, 1234±2, 1223±2, 1178±2, 1120±2, 1063±2, 1029±2, 1000±2, 983±2, 941±2, 850±2, 832±2, 803±2, 749±2, and 692±2 cm⁻¹;
d) an infrared (IR) spectrum as shown in FIG. 1B;
e) a differential scanning calorimetry (DSC) thermogram having an endothermic peak at about 261 °C;
f) a differential scanning calorimetry (DSC) thermogram as shown in FIG. 3;
g) a thermogravimetric analysis (TGA) thermogram as shown in FIG. 4; and/or
h) a dynamic vapor sorption (DVS) isotherm as shown in FIG. 1C.

2. The crystalline form of Compound (I) of claim 1 **characterized by** a powder x-ray diffraction pattern comprising two or more 2θ values selected from: 5.0±0.2, 5.3±0.2, 8.2±0.2, 10.0±0.2, 10.7±0.2, 10.9±0.2, 13.0±0.2, 14.6±0.2, 15.1±0.2, 16.1±0.2, 17.2±0.2, 19.0±0.2, 19.5±0.2, 20.5±0.2, 21.5±0.2, 22.9±0.2, and 24.5±0.2.

3. The crystalline form of Compound (I) of claim 1 **characterized by** a powder x-ray diffraction pattern comprising three or more, four or more, five or more, six or more, seven or more, eight or more, nine or more, or ten or more 20 values selected from: 5.0±0.2, 5.3±0.2, 8.2±0.2, 10.0±0.2, 10.7±0.2, 10.9±0.2, 13.0±0.2, 14.6±0.2, 15.1±0.2, 16.1±0.2, 17.2±0.2, 19.0±0.2, 19.5±0.2, 20.5±0.2, 21.5±0.2, 22.9±0.2, and 24.5±0.2.

4. The crystalline form of Compound (I) of claim 1 **characterized by** a powder x-ray diffraction pattern comprising 2θ values: 10.0±0.2, 13.0±0.2, 19.0±0.2, 19.5±0.2, 21.5±0.2, and 24.5±0.2.

5. The crystalline form of Compound (I) of claim 1 **characterized by** a powder x-ray diffraction pattern comprising 2θ values: 5.0±0.2, 10.0±0.2, and 10.7±0.2.

6. A pharmaceutical composition comprising the crystalline form of Compound (I) of any one of claims 1 to 5, and a pharmaceutically acceptable carrier or diluent.

7. The crystalline form of Compound (I) of any one of claims 1 to 5 or the pharmaceutical composition of claim 6 for use in a method of treating thrombosis.

8. The crystalline form of Compound (I) of any one of claims 1 to 5 or the pharmaceutical composition of claim 6 for use in a method of preventing thrombotic events, optionally in patients with renal and/or hepatic diseases.

9. The crystalline form of Compound (I) of any one of claims 1 to 5 or the pharmaceutical composition of claim 6 for use in a method of treating acute coronary syndrome, chronic coronary syndrome, deep vein thrombosis, unstable angina, myocardial infarction, atrial fibrillation, ischemic stroke, peripheral occlusive arterial disease, transient ischemic attack, secondary stroke prevention, or pulmonary embolism.

10. The crystalline form of Compound (I) of any one of claims 1 to 5 or the pharmaceutical composition of claim 6 for use in a method of treatment or prophylaxis of a disease selected from unstable angina, an acute coronary syndrome, atrial fibrillation, first myocardial infarction, recurrent myocardial infarction, ischemic sudden death, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from medical implants, devices, or procedures in which blood is exposed to an artificial surface that promotes thrombosis.

11. The crystalline form of Compound (I) of any one of claims 1 to 5 or the pharmaceutical composition of claim 6 for use in a method of treatment or prophylaxis of a disease selected from acute coronary syndrome, atrial fibrillation and stroke.

12. The crystalline form of Compound (I) or pharmaceutical composition for use according to claim 10 wherein the disease is acute coronary syndrome.

13. The crystalline form of Compound (I) or pharmaceutical composition for use according to claim 10 wherein the disease is atrial fibrillation.

14. The crystalline form of Compound (I) or pharmaceutical composition for use according to claim 10 wherein the disease is stroke.

15. The crystalline form of Compound (I) of any one of claims 1 to 5 or the pharmaceutical composition of claim 6 for use in a method of treatment or prophylaxis of acute coronary syndrome, deep vein thrombosis, chronic coronary syndrome, or secondary stroke prevention.

16. The crystalline form of Compound (I) of any one of claims 1 to 5 or the pharmaceutical composition of claim 6 for use in a method of prophylaxis of a patient of venous thromboembolism in acutely ill medical patients at risk for thromboembolic complication not at high risk of bleeding, or deep vein thrombosis which may lead to pulmonary embolism in patients undergoing knee or hip replacement surgery.

17. The crystalline form of Compound (I) of any one of claims 1 to 5 or the pharmaceutical composition of claim 6 for use in a method of reducing the risk of major cardiovascular events, cardiovascular death, myocardial infarction and stroke in patients with chronic coronary artery disease or peripheral artery disease.

18. The crystalline form of Compound (I) of any one of claims 1 to 5 or the pharmaceutical composition of claim 6 for use in a method of reducing the risk of recurrence of deep vein thrombosis and/or pulmonary embolism or stroke in patients with nonvalvular atrial fibrillation.

19. The crystalline form of Compound (I) of any one of claims 1 to 5 for use in manufacture of a pharmaceutical composition for the treatment or prevention of thromboembolic disorders.
